# EUROPEAN PATENT APPLICATION

(11) **EP 4 434 534 A1**
(43) Date of publication of application: **25.09.2024**
(21) Application number: 23163488.2
(22) Date of filing: 22.03.2023
(51) Int. Cl.: A61K 38/00, A61K 48/00, A61K 38/17, A61Q 19/08, A61P 43/00, C12N 9/14, C12N 9/24

(54) **KLOTHO MRNA**

(71) Applicant: ADvantage Therapeutics, Inc., Miami, FL 33127 (US)
(72) Inventor: The designation of the inventor has not yet been filed
(74) Representative: SONN Patentanwälte GmbH & Co KG

(57) **Abstract**

The present invention relates to a Klotho messenger-RNA (mRNA), wherein the mRNA has a 5' CAP region, a 5' un-translated region (5'-UTR), a coding region encoding a Klotho polypeptide, a 3' untranslated region (3'-UTR) and a poly-adenosine Tail (poly-A tail), wherein the Klotho polypeptide comprises the KL1 domain of human Klotho, preferably wherein the coding region encoding the Klotho polypeptide comprises an RNA sequence having at least 80 % sequence identity to SEQ ID NO: 1, and wherein the coding region encoding the Klotho polypeptide preferably has a GC content of at least 54 %.

## Description

The present invention relates to the field of Klotho, especially Klotho mRNA and formulations, kits and uses related thereto.

Klotho is an enzyme that in humans is encoded by the KL gene. There are three subfamilies of Klotho: α-Klotho, β-Klotho, and γ-Klotho. When the subfamily is not specified, the word "Klotho" typically means the α-Klotho subfamily, because α-Klotho was discovered before the other subfamily members. Therefore, "Klotho" and "a-Klotho" are used interchangeably herein.

Klotho is described in more detail, e.g., in WO 2019/113373 A2: Two transcripts that arise from a single Klotho gene through alternative RNA splicing have been identified. The first transcript encodes Klotho isoform 1 - a full-length, 1,012 amino acid, single-pass transmembrane-membrane protein, with a short cytoplasmic tail (human residues 1003-1012), a transmembrane (TM) domain (human residues 982-1002), and an extracellular region or domain (human residues 1-981) comprising two (largely) homologous (internal repeat) domains (termed KL1 (human residues 56-506, which is 450 residues long) and KL2 (human residues 515-953, which is 438 residues long), which each share 20%-40% amino acid sequence homology to b-glucosidases, but may lack similar levels of glucosidase catalytic activity), and a signal peptide (SP) domain (human residues 1-33; also referred to as "signal sequence", "SS"). The SP, KL1, and KL2 domain-containing extracellular region (human residues 1-981) may be enzymatically cleaved by a/b-secretases, and released into the circulatory stream as a 130 kDa circulating protein, termed soluble Klotho (or sKlotho, s-Klotho, alpha soluble Klotho, etc.). The extracellular region can also be cleaved into separate 68 kDa protein (KL1 + SS) and 64 kDa protein (KL2).

The second transcript, a splicing variant of alpha-Klotho mRNA, encodes a second isoform of Klotho protein corresponding mainly to the KL1 domain. The internal splice donor site is thought to be located in exon 3 of the Klotho gene. The resultant alternatively spliced transcript contains a 50 bp insertion after exon 3, with an in-frame translation stop codon at the end thereof. The expressed protein product is secreted into the circulation and is termed secreted Klotho (or Klotho isoform 2), which differs from the canonical sequence of isoform 1 at amino acid residues 535-549, and with amino acid residues 550-1012 missing.

Klotho was first described in 1997 as a repressor of ageing. Klotho knock out mice develop severe ageing phenotypes 4-5 weeks after birth and die within 9 weeks. Mice overexpressing Klotho from birth on live up to 30% longer than their wildtype littermates.

The Klotho gene is highly expressed in the liver, kidney and the brain. The anti-aging benefits are mostly related to its inhibition of the cellular uptake of glucose (i.e., inhibiting the interaction of insulin with the IGF-1 receptor). Furthermore, Klotho positively impacts neuroprotection and neurogenesis, stimulation of autophagy, regulation of oxidative stress, growth factor signaling, ion homeostasis and organ protection.

Several studies show that Klotho serum levels are high in children and young adults and decrease by 30-40% with age. Children also have better insulin uptake. Klotho blood levels below a certain threshold seem to correlate with AD/dementia, heart disease, and decreased lifespan. The slow decline of serum Klotho levels offers an opportunity for supplementation. In addition, the difference of Klotho levels between health and disease is very small. Therefore, a slight increase in Klotho levels may be sufficient to get above the threshold. Klotho can therefore be considered a promising anti-aging compound.

In addition to the anti-aging effects of Klotho, Klotho deficiencies can be found in many different diseases. Among others: kidney diseases, cardiovascular diseases, brain and lung diseases have been shown to benefit from the elevation of Klotho as reviewed in Prud'homme et al., 2022, table 1 and 3 (Prud'homme et al. "Pathobiology of the Klotho Antiaging Protein and Therapeutic Considerations." Frontiers in Aging 3 (2022)). Hence, Klotho also acts as a tumor suppressor in various kinds of tumors (Sachdeva, et al. "Klotho and the treatment of human malignancies." Cancers 12.6 (2020): 1665).

In view of the potential benefits of Klotho, strategies are being pursued to increase the expression level of Klotho in individuals and/or to administer Klotho, e.g., recombinant s-Klotho. Such strategies and uses of Klotho are described, e.g., in WO 2019/113373 A2, WO2022211420A1, WO2022008971A2 , WO2020163017 A1, WO2020106997A1, WO2020106351A1, WO2020039425A1, WO2019140250A1, WO2019113373A2, WO2019113061A1, WO2018098375A1, WO2017210607A1, WO2016127097A1, WO2016088059A1, WO2014152993A1, WO2011084452A1, and WO2008135993A1.

However, despite the promising mechanisms in which Klotho protein has been shown to be relevant, there has yet to be an approved treatment brought to market in the approximately 25 years that Klotho has been discussed as a promising target by major or minor pharmaceutical companies.

It is an object of the present invention to address this need.

Therefore, the present invention provides a Klotho messenger-RNA (mRNA), wherein the mRNA has a 5' CAP region, a 5' untranslated region (5'-UTR), a coding region encoding a Klotho polypeptide, a 3' untranslated region (3'-UTR) and a poly-adenosine tail (poly-A tail), wherein the Klotho polypeptide comprises the KL1 domain of human Klotho, preferably wherein the coding region encoding the Klotho polypeptide comprises an RNA sequence having at least 80 % sequence identity to SEQ ID NO: 1, and wherein the coding region encoding the Klotho polypeptide preferably has a GC content of at least 54 %.

One of the main reasons why pharmaceutical companies have so far failed to realize the potential of Klotho protein is most likely that the focus of the players in field so far has been on the application of exogeneous manufactured Klotho protein derived e.g. from E. coli or cell culture. These types of proteins miss at least two features of how the Klotho protein is endogenously expressed by the body's own cells, which are:
(1) fully humanized, fully individualized and fully personalized;
(2) the allocation and delivery strategy of the body for Klotho.

Klotho mRNA is not (only) expressed by direct transcription from the DNA within the parental e.g. kidney cell. It is also, most likely even mainly, packed into exosomes (cf Sahu "Regulation of aged skeletal muscle regeneration by circulating extracellular vesicles." Nature Aging 1.12 (2021): 1148-1161). These are then budded of the cell to spread into the blood to reach other target tissues and cells including brain cells. Also further shortcomings/challenges on the protein level might be taken into account, such as short half life of the protein itself, intracellular availability, targeting, lack of specificity with intravenous administration, and inability for oral administration. Therefore, despite currently available methods for administering recombinant Klotho and for increasing the expression level of endogenous Klotho, new and improved options for harnessing the benefits of this protein are needed. In particular, new and improved methods and may be even more important new formats including exosomes for providing the Klotho benefit portfolio to individuals in order to positively impact on aging and/or to harness the therapeutic outcome are desired.

mRNA has emerged as a revolutionary tool in the field of medicine due to its ability to carry genetic instructions to cells and direct the production of specific proteins. While the use of mRNA-based vaccines has garnered much attention in recent times, mRNA-based therapeutics have also shown tremendous potential in treating a wide range of conditions and diseases. The basic principle behind exogenous mRNA-based therapeutics is to introduce such mRNA molecules into cells, which can then instruct the cells to produce therapeutic proteins that are otherwise missing or dysfunctional in the body or are just expressed at insufficient levels.

The success of such mRNA, in particular mRNA-based therapeutics, heavily relies on the level of expression of the mRNA in the body, as it determines the amount of protein produced. There are several general approaches to improve the expression levels of mRNA, including modifying the 5' and 3' untranslated regions (UTRs) of the mRNA, and/or optimizing the formulation and delivery method. Taking together, it is often the individual mRNA sequence itself that determines the level of expression achieved.

General methods for the preparation and use of mRNA molecules for such methods are described, e.g., in WO 2013/151736 A2. WO 2013/151736 A2 describes a large number of different examples for such mRNAs. Among them, Example 125 in paragraph [0001618] refers to the intravenous administration of a Klotho mRNA to CD1 mice. The RNA sequence is included in the sequence listing of WO 2013/151736 A2 as SEQ ID NO: 196. Analysis of this sequence shows that it apparently codes for Klotho isoform 2. It is stated in Example 125 of WO 2013/151736 A2 that said mRNA was chemically modified in various ways and that it was administered complexed with Lipofectamine 2000. However, WO 2013/151736 A2 does not state that the RNA sequence itself was optimized in any way.

While the use of mRNA as a therapeutic agent has shown great potential for treating various diseases, its application to specific cases can be challenging. A primary reason that may have hampered the development of effective Klotho mRNA in the past may be that inadequate expression levels are achieved. Low expression levels are a significant challenge in the development of mRNA-based therapeutics. mRNA molecules carry instructions for protein synthesis, and their expression level determines the amount of protein that will be produced in cells. Inadequate expression levels can result in insufficient therapeutic effects, thus limiting the clinical utility. If the expression level is too low, the therapeutic effect may not be sufficient, and the treatment may fail. Therefore, improving the expression levels of mRNA is necessary to enable the development of more effective therapeutic and other applications.

The RNA sequence is a critical determinant of protein expression levels, and modifying it can significantly impact the efficacy of mRNA-based therapeutics. With the help of codon optimization algorithms, it is possible to modify the RNA sequence without altering the protein sequence. These algorithms typically take into account various factors such as codon usage bias, amino acid frequency, and secondary structure of the mRNA. Many of these codon optimization algorithms are available through software or online, and researchers can easily access them to optimize the sequence of their mRNA of interest.

The present inventors set out to obtain mRNA for the expression of Klotho with the aim of achieving high expression levels. For initial experiments, they selected soluble Klotho, i.e., corresponding to amino acids M1 to S981 of Klotho isoform 1. They started from the sequence of native human Klotho isoform 1 according to NCBI Reference Sequence: NM_004795.4. As described in more detail in the Examples, the sequence coding for soluble Klotho, i.e., amino acids M1 to S981 was extracted (SEQ ID NO: 17). This sequence is 2946 nt long and can be divided into the signal peptide (amino acids 1-33; nt 1-99) and KL1 and KL2 domains including adjacent linking regions (amino acids E34 to S981; nt 100-2946). The part coding for the signal peptide was kept constant and the latter part of the sequence corresponding to nt 100-2946 was optimized using four different codon optimization algorithms, as provided through websites of different gene synthesis providers:
- "GENEius algorithm" (https://eurofinsgenomics.eu/en/gene-synthesis-molecular-biology/gene-synthesis/geneius/) provided by Eurofins Genomics Germany GmbH: resulting in SEQ ID NO: 25.
- "IDT Codon Optimization Tool"
   (https://eu.idtdna.com/pages/tools/codon-optimization-tool) provided by Integrated DNA Technologies, Inc.: resulting in SEQ ID NO: 26
- "ExpOptimizer" (https://www.novoprolabs.com/tools/codon-optimization) provided by NovoPro Bioscience, Inc.: resulting in SEQ ID NO: 27
- "Twist Codon Optimization Tool" (https://www.twistbioscience.com/twist-ordering-platform) provided by Twist Bioscience: resulting in SEQ ID NO: 28

The expression levels obtained with the four commercially optimized sequence variants varied quite substantially (see Examples and Figures). Therefore, the inventors set out to test if it would be possible to achieve even higher expression levels. When the inventors compared the optimized sequences to the sequence of native human Klotho (SEQ ID NO: 17), they eventually made the surprising realization that the optimized sequences all had significantly lower GC contents (see Table 1). Investigating this further, they found that the expression level appeared to follow the GC content: The sequence obtained from the "GENEius algorithm" (SEQ ID NO: 25) had the highest GC content (52.6 %) and also gave the highest expression levels (see Examples and Figures). The second best result was achieved with the sequence obtained from "ExpOptimizer" (SEQ ID NO: 27; GC content 51.1 %), followed by the sequence from the "IDT Codon Optimization Tool" (SEQ ID NO: 26; GC content: 49.9 %) and finally the "Twist Codon Optimization Tool" (SEQ ID NO: 28; GC content: 48.7%).

The inventors therefore came up with the hypothesis that for the expression of Klotho, a high GC content may be beneficial. They tested this hypothesis by creating a number of mRNA variants having different GC contents, namely:
- Variant "GC62" (SEQ ID NO: 18); GC content: 61,6 %
- Variant "GC60" (SEQ ID NO: 19); GC content: 60,1 %
- Variant "GC57" (SEQ ID NO: 22); GC content: 57,4 %
- Variant "GC55" (SEQ ID NO: 23); GC content: 54,9 %
- Variant "GC53" (SEQ ID NO: 24); GC content: 52,8 %

Surprisingly, a direct relationship between the GC content and the expression level was indeed found. The expression level continued to increase going from variant "GC53" to variant "GC62", i.e., even above the GC content of native Klotho.

A summary of the tested sequences and their GC content is given in the following Table.

**Table 1: Tested mRNA variants coding for soluble Klotho (M1 to S981 of human Klotho isoform 1) and GC content:**

| **Codon usage** | **SEQ ID NO:** | **GC content (%)** |
|---|---|---|
| Native human Klotho | 17 | 54,5 |
| Variant "GC62" | 18 | 61,6 |
| Variant "GC60" | 19 | 60,1 |
| Variant "GC57" | 22 | 57,4 |
| Variant "GC55" | 23 | 54,9 |
| Variant "GC53" | 24 | 52,8 |
| GENEius algorithm | 25 | 52,6 |
| IDT Codon Optimization Tool | 26 | 49,9 |
| ExpOptimizer | 27 | 51,1 |
| Twist Codon Optimization Tool | 28 | 48,1 |

In addition, the inventors examined the sequence identity of the various sequences. It was found that the closer the variants were in sequence identity to the best performing variant "GC62", the higher the expression level. Interestingly, this trend was also true for four sequences obtained from the commercial sequence optimization tools. The best performing sequence obtained from the "GENEius algorithm" (SEQ ID NO: 25) had the highest sequence identity to "GC62" (80.6 %); followed by the second best sequence which was obtained from "ExpOptimizer" (SEQ ID NO: 27; 79.6 % sequence identity to "GC62"); followed by the third best sequence, which was from the "IDT Codon Optimization Tool" (SEQ ID NO: 26; 77.3 % sequence identity to "GC62") and finally the "Twist Codon Optimization Tool" that gave the lowest results (SEQ ID NO: 28; 76.4 % sequence identity to "GC62"). All the custom variants had higher sequence identities to "GC62" than the ones obtained from the commercial sequence optimization tools. Also in this case, expression levels increased continuously with increasing sequence identity to "GC62", starting from the variant "GC53" (SEQ ID NO: 24; 81.6 % sequence identity to "GC62") and going up all the way to "GC60" (SEQ ID NO: 19; 92.0% sequence identity to "GC62"). Thus, a direct relationship between sequence identity to "GC62" and expression level was found. The sequence identities between all the variants tested are summarized in the following Table.

**Table 2: Sequence identities between different mRNA variants tested. Values are given in (%).**

| **SEQ ID NO:** | **17** | **18** | **19** | **22** | **23** | **24** | **25** | **26** | **27** | **28** |
|---|---|---|---|---|---|---|---|---|---|---|
| **17** | 100 | | | | | | | | | |
| **18** | 80,3 | 100 | | | | | | | | |
| **19** | 80,1 | 92 | 100 | | | | | | | |
| **22** | 79,2 | 88,6 | 89,5 | 100 | | | | | | |
| **23** | 79, 2 | 85,2 | 86 | 90,3 | 100 | | | | | |
| **24** | 78,1 | 81,6 | 83,3 | 85,4 | 85 | 100 | | | | |
| **25** | 77 | 80,4 | 79,9 | 79,8 | 79,7 | 79,4 | 100 | | | |
| **26** | 76, 6 | 77,3 | 77,5 | 77,2 | 77,8 | 77,6 | 77,8 | 100 | | |
| **27** | 77,7 | 79,6 | 79,3 | 79,2 | 78,1 | 78,4 | 78,7 | 76,9 | 100 | |
| **28** | 66,2 | 76,4 | 77,1 | 77,3 | 77 | 77,1 | 78 | 78,3 | 77,9 | 100 |

It is interesting to note that the four sequences obtained from the commercial sequence optimization tools do not share a high degree of sequence identity between each other. As follows from the above table, each pair among the four sequences has a sequence identity below 79 %. Thus, even though the four sequences were apparently optimized in different ways, none of the sequence comes close to the preferred variant "GC 62", the best being the one obtained from the "GENEius algorithm" (SEQ ID NO: 25) having a sequence identity to "GC62" of 80.6 %.

In addition to the sequence variants coding for soluble Klotho (amino acids M1 to S981) described above, the present inventors also designed corresponding sequence variants coding for human Klotho isoform 2 (amino acids M1 to H549) . As laid out above, human Klotho isoform 2 shares the amino acids M1 to V534 with Klotho isoform 1. Thus, the inventors designed a "GC62" variant of isoform 2 (SEQ ID NO: 41), wherein nucleotides 1 to 1602 (coding for M1 to V534) are identical to nucleotides 1 to 1602 of the "GC62" variant of isoform 1 (SEQ ID NO: 18). Only the nucleotides 1603 to 1650 (coding for D535 to H549) are different in the isoform 2 variant SEQ ID NO: 41 compared to the isoform 1 variant SEQ ID NO: 18. The GC content of the isoform 2 variant SEQ ID NO: 41 is similarly to that of the isoform 1 variant SEQ ID NO: 18, namely 62.8 %.

It is instructive to compare the preferred isoform 2 variants created in the context of the invention to the Klotho mRNA sequence disclosed in WO 2013/151736 A2, mentioned above. SEQ ID NO: 192 of WO 2013/151736 A2 has 1793 nucleotides and contains an open reading frame of 1650 nucleotides that appears to encode for human Klotho isoform 2. The present inventors therefore compared this sequence to the best performing "GC62" variant SEQ ID NO: 41 created in the context of the invention. Strikingly, the sequence used in WO 2013/151736 A2 also has a lower GC content (53.8 % for SEQ ID NO: 192 of WO 2013/151736 A2; compared to 62.8 % for SEQ ID NO: 41 of the present application). In addition, SEQ ID NO: 192 of WO 2013/151736 A2 only has 75.7 % sequence identity to SEQ ID NO: 41 of the present application. Thus, the sequence variants created in the context of the present invention are advantageous over the mRNA sequence disclosed in WO 2013/151736 A2 for similar reasons as laid out above for the commercially optimized variants of mRNA encoding Klotho isoform 1 - namely, higher GC content and higher sequence identity to the best performing "GC62" variants created in the context of the present invention.

All embodiments of the invention are described together in the following detailed description and all preferred embodiments relate to all embodiments and aspects alike. All detailed descriptions, e.g. of mRNAs and compositions, relate to preferred embodiments of all aspects of the invention, e.g. compositions for use, uses and methods. All embodiments can be combined with each other, except where stated otherwise.

Human Klotho isoform 1 (UniProt Q9UEF7-1) has the following sequence; SEQ ID NO: 49:

MPASAPPRRPRPPPPSLSLLLVLLGLGGRRLRAEPGDGAQTWARFSRPPAPEAAGLFQGT
FPDGFLWAVGSAAYQTEGGWQQHGKGASIWDTFTHHPLAPPGDSRNASLPLGAPSPLQPA
TGDVASDSYNNVFRDTEALRELGVTHYRFSISWARVLPNGSAGVPNREGLRYYRRLLERL
RELGVQPVVTLYHWDLPQRLQDAYGGWANRALADHFRDYAELCFRHFGGQVKYWITIDNP
YVVAWHGYATGRLAPGIRGSPRLGYLVAHNLLLAHAKVWHLYNTSFRPTQGGQVSIALSS
HWINPRRMTDHSIKECQKSLDFVLGWFAKPVFIDGDYPESMKNNLSSILPDFTESEKKFI
KGTADFFALCFGPTLSFQLLDPHMKFRQLESPNLRQLLSWIDLEFNHPQIFIVENGWFVS
GTTKRDDAKYMYYLKKFIMETLKAIKLDGVDVIGYTAWSLMDGFEWHRGYSIRRGLFYVD
FLSQDKMLLPKSSALFYQKLIEKNGFPPLPENQPLEGTFPCDFAWGVVDNYIQVDTTLSQ
FTDLNVYLWDVHHSKRLIKVDGVVTKKRKSYCVDFAAIQPQIALLQEMHVTHFRFSLDWA
LILPLGNQSQVNHTILQYYRCMASELVRVNITPVVALWQPMAPNQGLPRLLARQGAWENP
YTALAFAEYARLCFQELGHHVKLWITMNEPYTRNMTYSAGHNLLKAHALAWHVYNEKFRH

AQNGKISIALQADWIEPACPFSQKDKEVAERVLEFDIGWLAEPIFGSGDYPWVMRDWLNQ
RNNFLLPYFTEDEKKLIQGTFDFLALSHYTTILVDSEKEDPIKYNDYLEVQEMTDITWLN
SPSQVAVVPWGLRKVLNWLKFKYGDLPMYIISNGIDDGLHAEDDQLRVYYMQNYINEALK
AHILDGINLCGYFAYSFNDRTAPRFGLYRYAADQFEPKASMKHYRKIIDSNGFPGPETLE
RFCPEEFTVCTECSFFHTRKSLLAFIAFLFFASIIISLSLIFYYSKKGRRSYK

The different domains of the sequence are highlighted above as follows:
- *Italics underline: Signal peptide ("SP") (M1 to A33)*
- **Bold underline: KL1 domain (L56 to F506)**
- ***Bold italics: KL2 domain (L515 to G952)***
- Underline: transmembrane ("TM") domain (L982 to Y1002)
- *Italics:* cytoplasmic tail ("CT") (Y1003 to K1012)
- No emphasis: linking regions ("lr") between domains
Human Klotho isoform 2 (UniProt Q9UEF7-2) has the following sequence; SEQ ID NO: 50:

MPASAPPRRPRPPPPSLSLLLVLLGLGGRRLRAEPGDGAQTWARFSRPPAPEAAGLFQGT
FPDGFLWAVGSAAYQTEGGWQQHGKGASIWDTFTHHPLAPPGDSRNASLPLGAPSPLQPA
TGDVASDSYNNVFRDTEALRELGVTHYRFSISWARVLPNGSAGVPNREGLRYYRRLLERL
RELGVQPVVTLYHWDLPQRLQDAYGGWANRALADHFRDYAELCFRHFGGQVKYWITIDNP
YVVAWHGYATGRLAPGIRGSPRLGYLVAHNLLLAHAKVWHLYNTSFRPTQGGQVSIALSS
HWINPRRMTDHSIKECQKSLDFVLGWFAKPVFIDGDYPESMKNNLSSILPDFTESEKKFI
KGTADFFALCFGPTLSFQLLDPHMKFRQLESPNLRQLLSWIDLEFNHPQIFIVENGWFVS
GTTKRDDAKYMYYLKKFIMETLKAIKLDGVDVIGYTAWSLMDGFEWHRGYSIRRGLFYVD
FLSQDKMLLPKSSALFYQKLIEKNGFPPLPENQPLEGTFPCDFAWGVVDNYIQVSQLTKP
ISSLTKPYH

The different domains of the sequence are highlighted above as follows:
- *Italics underline: Signal peptide ("SP") (Ml to A33)*
- **Bold underline: KL1 domain (L56 to F506)**
- ***Bold italics: sequence corresponding to KL2 domain of isoform 1 (L515 to V534)***
- Underline: alternative sequence (S535 to H549)
- No emphasis: linking regions ("lr") between domains

As indicated in the sequences above, herein all the sequences between the different domains are referred to as "linking regions" (lr). Which sequence a given "lr" refers to, follows from the adjacent domains. For instance, when the present application refers to the part "KL1-lr-KL2" of Klotho isoform 1, this corresponds to amino acids L56 to G952, and "lr" in this instance refers to P507 to P514. Similarly, "lr-KL1-lr-KL2-lr" refers to E34 to S981, wherein the first "lr" is E34 to G55, the second "lr" is P507 to P514, and the third "lr" is to F953 to S981.

In the sequence of Klotho isoform 2, the portion from P507 to H549 (i.e., the C-terminal part of the protein following the KL1 domain) is referred to herein as the "end region" ("end"). Thus, as used herein and as shown above, "end" corresponds to P507 to H549 and includes an lr (P507 to P514), the "sequence corresponding to KL2 domain of isoform 1" (L515 to V534) and the "alternative sequence" (S535 to H549).

All domains and regions of the sequences referred to herein, such as the KL1, KL2, SP, TM, CT, lr, end domains and regions, may refer to the sequences of SEQ ID NO: 49 and 50 shown above or to any variants thereof, in particular variants with modified or improved properties, as laid out herein below. It is preferred, that these domains and regions refer to the sequences of SEQ ID NO: 49 and 50 shown above.

In an aspect, the present invention relates to a Klotho messenger-RNA (mRNA), wherein the mRNA has a 5' CAP region, a 5' untranslated region (5'-UTR), a coding region encoding a Klotho polypeptide, a 3' untranslated region (3'-UTR) and a poly-adenosine Tail (poly-A tail), wherein the Klotho polypeptide comprises the KL1 domain of human Klotho.

It is known in the art that the KL1 domain of human Klotho provides beneficial effects independently from the other parts of the Klotho protein. This is reported, for instance, Gupta et al. ("KL1 domain of longevity factor Klotho mimics the metabolome of cognitive stimulation and enhances cognition in young and aging mice." Journal of Neuroscience 42.19 (2022): 4016-4025). Thus, when the Klotho polypeptide comprises at least the KL1 domain of human Klotho, it can provide important beneficial effects.

In a preferred embodiment, the Klotho polypeptide further comprises the signal peptide and/or the KL2 domain of human Klotho. It is preferred, if the Klotho polypeptide comprises at least the KL1 and the KL2 domain of human Klotho. It is particularly preferred, if the Klotho polypeptide comprises the signal peptide, the KL1 domain and the KL2 domain of human Klotho.

In another preferred embodiment, the Klotho polypeptide further comprises the transmembrane (TM) domain of human Klotho. Preferably, the Klotho polypeptide further comprises the cytoplasmic tail (CT) of human Klotho. When the TM and preferably also the CT domains are present, this has the advantage that a membrane anchored version of Klotho protein can be delivered to an individual. This constitutes a significant advantage over strategies used for Klotho so far, in which recombinant protein is administered. When recombinant protein is administered, only soluble versions of Klotho can be used, which means that only the soluble side of the Klotho signalling is accessible. In contrast, by using mRNA, a membrane-anchored version of Klotho can be delivered, which allows exploiting the full breadth of Klotho signalling.

As used herein, the phrases "KL1 domain", "KL2 domain" and "signal peptide" in relation to human Klotho preferably refer to the KL1, KL2 and SP sequences described above with reference to human Klotho isoform 1 (UniProt Q9UEF7-1; SEQ ID NO: 49) and to human Klotho isoform 2 (UniProt Q9UEF7-2; SEQ ID NO: 50) or to any variants thereof, in particular any of the variants described below. Preferably, the signal peptide is SEQ ID NO: 51, the KL1 domain is SEQ ID NO: 52, and the KL2 domain is SEQ ID NO: 54.

A functional variant of Klotho called "KL-VS" is found in a certain part of the human population (see e.g. Arking et al. "Association between a functional variant of the KLOTHO gene and high-density lipoprotein cholesterol, blood pressure, stroke, and longevity" Circulation Research 96.4 (2005): 412-418; Arking et al. "Association of human aging with a functional variant of Klotho" PNAS 99.2 (2002): 856-861.). This variant contains two mutations in the KL1 domain, namely F325V and C370S. This variant is associated with overall decreased mortality and other positive effects. Thus, in a preferred embodiment, the KL1 domain of human Klotho is the VS variant. Preferably, the KL1 domain is SEQ ID NO: 53.

Any other suitable variant of the SP, KL1 domain and/or the KL2 domain of human Klotho can advantageously be used in the context of the present invention. It is preferred, when the Klotho polypeptide is a native Klotho protein, especially a native human Klotho protein, in particular a native human Klotho isoform 1 or a native human Klotho isoform 2, especially SEQ ID NO: 49 OR 50.

It is further preferred when the Klotho polypeptide is a Klotho protein, protein fragment, and/or protein variant as described in WO 2019/113373 A2, which is incorporated herein by reference. The Klotho polypeptide can comprise all or a subset of amino acid residues 1-1012, 1-981, 29- 981, 34-981, 36-981, 131-981, 1-549, 29-549, 34-549, 36-549, or 131-549 of human Klotho isoform 1 or 2. Some embodiments can include a protein having one or more amino acid variations as compared to human Klotho isoform 1. Illustratively, the protein can comprise a human C370 variant. For instance, the protein can include a C370S alteration, thereby comprising S370. The protein can include human F352 or other than F352V in some embodiments. In at least one embodiment, the protein can include the C370S alteration, thereby comprising S370, without a F352V variant, preferably with F352. The protein can include H193 or other than the H193R variant. All other standard amino acid substitutions at amino acid residue (or position) 193, 352, and/or 370 of human Klotho isoform 1 are contemplated and explicitly disclosed herein.

Some embodiments can include a variation at amino acid residue 45 of human Klotho isoform 1. At position 45, the residue can be a valine (Val; V), a phenylalanine (Phe; F), or another amino acid.

In some embodiments, the Klotho polypeptide can include a signal peptide or signal sequence. For example, the protein can include a native Klotho signal sequence. The protein can include a non-native signal sequence. In some embodiments, the signal sequence can be an N-terminal signal sequence and/or upstream of (or N- terminal to) a Klotho protein sequence. In other embodiments, the signal sequence can be C-terminal or otherwise disposed. Preferably, the signal sequence is the SP of human Klotho as set forth in SEQ ID NO: 51.

Substituting a Phenylalanine in the place of the Valine at (native) position 45 of human Klotho (V45F) has been reported to have benefits. For example, it has been reported in WO 2019/113373 A2 that V45F soluble Klotho, and fragments or fusion proteins thereof, express at higher levels in CHO and HEK-293 cells than does the native, wild type V45 protein. Accordingly, some embodiments of the present disclosure include a Klotho polypeptide having the V45F substitution.

The mRNA used in the present invention contains (at least) five essential elements which are all known and available to a person skilled in the art (in this order from 5' to 3'): a 5' CAP region, a 5' untranslated region (5'-UTR), a coding region, a 3' untranslated region (3'-UTR) and a poly-adenosine tail (poly-A tail) .

The coding region should, of course encode a (human) Klotho polypeptide, the other components may be the (native) Klotho UTRs or, preferably, other UTRs. Specifically preferred UTRs according to the present invention are UTRs which improve the properties of the mRNA molecule according to the present invention, i.e. by effecting better and/or longer and/or more effective translation of the mRNA into the Klotho polypeptide at the site of administration.

A "CAP region" ("5'CAP") refers to a structure found on the 5' end of an mRNA molecule and generally consists of a guanosine nucleotide connected to the mRNA via an unusual 5' to 5' triphosphate linkage. This guanosine nucleotide is methylated on the 7-position directly after capping in vivo by a methyl transferase ("7-methylguanylate cap" ("m7G"), "cap-0"). Further modifications include the possible methylation of the 2' hydroxy-groups of the first two ribose sugars of the 5' end of the mRNA (i.e. "CAP1" and "CAP2"): "CAP1" has a methylated 2'-hydroxy group on the first ribose sugar, while "CAP2" has methylated 2'-hydroxy groups on the first two ribose sugars. The 5' cap is chemically similar to the 3' end of an RNA molecule (the 5' carbon of the cap ribose is bonded, and the 3' unbonded). This provides significant resistance to 5' exonucleases and is therefore also providing stability in vivo. For generation of mRNAs according to the present invention also CAP analogues may be used including: monomethylated CAP analogue (mCAP), Anti-Reverse Cap Analog (ARCA CAP), m7G(5')ppp(5')A RNA CAP structure analog, G(5')ppp(5')A RNA CAP structure analog, and G(5')ppp(5')G RNA CAP structure analog.

The term "(5'- or 3'-) UTR" refers to the well-established concept of untranslated region of a mRNA in molecular genetics. There is one UTR on each side of a coding sequence on a strand of mRNA. The UTR on the 5' side, is the 5'-UTR (or leader sequence), the UTR on the 3' side, is the 3'-UTR (or trailer sequence). The 5'-UTR is upstream from the coding sequence. Within the 5'-UTR is a sequence that is recognized by the ribosome which allows the ribosome to bind and initiate translation. The mechanism of translation initiation differs in prokaryotes and eukaryotes. The 3'-UTR is found immediately following the translation stop codon. The 3'-UTR plays a critical role in translation termination as well as post-transcriptional gene expression. The UTRs as used in the present invention are usually delivering beneficial stability and expression (translation) properties to the mRNA molecules according to the present invention.

The "poly-A tail" consists of multiple adenosine monophosphates; it is a part of naturally occurring mRNA that has only adenine bases. This process called "polyadenylation" is part of the process that produces mature messenger RNA (mRNA) for translation in the course of gene expression. The natural process of polyadenylation begins as the transcription of a gene terminates. The 3'-most segment of the newly made pre-mRNA is first cleaved off by a set of proteins; these proteins then synthesize the poly(A) tail at the RNA's 3' end. In some genes, these proteins add a poly(A) tail at one of several possible sites. Therefore, polyadenylation can produce more than one transcript from a single gene (alternative polyadenylation), similar to alternative splicing. The poly(A) tail is important for the nuclear export, translation, and stability of mRNA. For the present invention, it is therefore mainly the translation and stability properties that are important for a sufficient polyadenylation of the mRNA molecules according to the present invention. During the protein generation, the tail is shortened over time, and, when it is short enough, the mRNA is enzymatically degraded. The poly-A tail according to the present invention is provided in the manner currently used and applied in the art of administering mRNA molecules in human therapy. For example, the poly-A tail may be at least 60 adenosine monophosphates long. According to a preferred embodiment, the poly-A tail is at least 100 adenosine monophosphates long, especially at least 120 adenosine monophosphates. This allows excellent stability and protein generation; however, as for the other features, the action and activity of the mRNA molecule according to the present invention can also be regulated by the poly-A tail feature.

As laid out in more detail above and as shown in the Examples herein, it has been surprisingly found in the context of the present invention that there is a direct relationship between the GC content of the Klotho mRNA and the expression level of the Klotho polypeptide. As used herein, the "GC content" of a certain mRNA or a certain part of a certain mRNA refers to the sum of G and C bases in relation to the total number of bases in said mRNA or in said part of the mRNA, expressed as a percentage. Thus, the GC content can be calculated as 100%* (C+G)/(A+C+G+U), wherein "C", "G", "A" and "U" refer to the number of C-, G-, A- and U-bases, respectively. In connection with the nucleoside variants disclosed herein below, it is important to note that the specific preferred variants described above do not influence the GC content, i.e. the variant is conservative in this respect (e.g. a cytidine variant still counts as a cytidine for the calculation of the GC content).

Therefore, in a preferred embodiment of the inventive Klotho mRNA, the coding region encoding the Klotho polypeptide has a GC content of at least 50 %, preferably at least 51 %, more preferred at least 52 %, more preferred at least 53 %, more preferred at least 54 %, more preferred at least 55 %, more preferred at least 56 %, more preferred at least 57 %, more preferred at least 58 %, more preferred at least 59 %, more preferred at least 60 %, more preferred at least 61 %, more preferred at least 62 %, more preferred at least 63. It is further preferred if the coding region encoding the Klotho polypeptide has a GC content of between 50 % and 74 %, preferably between 51 % and 73 %, more preferred between 52 % and 72 %, more preferred between 53 % and 71 %, more preferred between 54 % and 70 %, more preferred between 55 % and 69 %, more preferred between 56 % and 68 %, more preferred between 57 % and 67 %, more preferred between 58 % and 66 %, more preferred between 59 % and 65 %, more preferred between 60 % and 64 %, more preferred between 61 % and 63 %.

In a preferred embodiment, the Klotho mRNA has a GC content of at least 50 %, preferably at least 51 %, more preferred at least 52 %, more preferred at least 53 %, more preferred at least 54 %, more preferred at least 55 %, more preferred at least 56 %, more preferred at least 57 %, more preferred at least 58 %, more preferred at least 59 %, more preferred at least 60 %, more preferred at least 61 %, more preferred at least 62 %, more preferred at least 63. It is further preferred if the Klotho mRNA has a GC content of between 50 % and 74 %, preferably between 51 % and 73 %, more preferred between 52 % and 72 %, more preferred between 53 % and 71 %, more preferred between 54 % and 70 %, more preferred between 55 % and 69 %, more preferred between 56 % and 68 %, more preferred between 57 % and 67 %, more preferred between 58 % and 66 %, more preferred between 59 % and 65 %, more preferred between 60 % and 64 %, more preferred between 61 % and 63 %.

As laid out in more detail above, it found in the course of the present invention that the closer the Klotho mRNA is in sequence identity to the "GC62" variant created in the course of the present invention, the higher the expression level was. The sequence of the GC62 variant of the KL1 domain of native human Klotho according to SEQ ID NO: 52 is set forth in SEQ ID NO: 1. Therefore, in a preferred embodiment, the coding region encoding the Klotho polypeptide comprises an RNA sequence having at least 80 % sequence identity to SEQ ID NO: 1. Preferably, the sequence identity is at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %. Preferably the RNA sequence is SEQ ID NO: 1 or 3, especially SEQ ID NO: 1.

In preferred embodiments, the coding region encoding the Klotho polypeptide comprises SEQ ID NO: 1, 2, 3 or 4, preferably SEQ ID NO: 1.

SEQ ID NOs: 1 and 2 code for the KL1 domain of native human Klotho according to SEQ ID NO: 52, whereas SEQ ID NOs: 3 and 4 code for the KL1 domain of the VS variant described above according to SEQ ID NO: 53.

The sequence of the GC62 variant of the domains KL1-lr-KL2 of native human Klotho isoform 1 (amino acids L56 to G952 of SEQ ID NO: 49) is set forth in SEQ ID NO: 9. Therefore, in a further preferred embodiment, the coding region encoding the Klotho polypeptide comprises an RNA sequence having at least 80 % sequence identity to SEQ ID NO: 9, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 9 or 11, especially SEQ ID NO: 9.

In a preferred embodiment, the coding region encoding the Klotho polypeptide comprises SEQ ID NO: 9, 10, 11 or 12, preferably SEQ ID NO: 9.

The sequence of the GC62 variant of the domains lr-KL1-lr-KL2-lr of native human Klotho isoform 1 (amino acids E34 to S981 of SEQ ID NO: 49) is set forth in SEQ ID NO: 13. This sequence codes for the full sequence of soluble Klotho isoform 1 except for the N-Terminal SP. It should be noted that in the experiments referred to herein above and described in more detail in the Examples and Figures below, beneficial effects of increasing GC content and sequence identity to the "GC62" variant (SEQ ID NO: 18) were observed independently from the sequence of the signal peptide used. In fact, in the comparative tests comparing the four variants obtained from commercial codon optimization algorithms (GENEius algorithm, IDT Codon Optimization Tool, ExpOptimizer, Twist Codon Optimization Tool) as well as the variants "GC 53", "GC55", "GC57", "GC60" and "GC62", the same SP sequence was used and only the remaining part of the mRNA construct (corresponding to the domains lr-KL1-lr-KL2-lr; nucleotides 100-2946) were optimized. Thus, the advantageous effects of the high sequence similarity to the "GC62" variant was independent from the specific SP sequence used. Therefore, in a further preferred embodiment the coding region encoding the Klotho polypeptide comprises an RNA sequence having at least 80 % sequence identity to SEQ ID NO: 13, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 13 or 15, especially SEQ ID NO: 13.

In preferred embodiments, the coding region encoding the Klotho polypeptide comprises SEQ ID NO: 13, 14, 15 or 16, preferably SEQ ID NO: 13.

The sequence of the GC62 variant of the domains lr-KL1-lr-KL2-lr-TM-CT of native human Klotho isoform 1 (amino acids E34 to K1012 of SEQ ID NO: 49) is set forth in SEQ ID NO: 64. This sequence codes for the full sequence of Klotho isoform 1 except for the N-terminal SP but including the transmembrane domain and the cytoplasmic tail. This is particularly advantageous since, by combining it with any suitable signal peptide (native or otherwise), it allows delivering a membrane-anchored version of the Klotho protein, which makes it possible to exploit the full breadth of Klotho signalling rather than just the soluble part of it. Therefore, in a further preferred embodiment the coding region encoding the Klotho polypeptide comprises an RNA sequence having at least 80 % sequence identity to SEQ ID NO: 64, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 64 or 66, especially SEQ ID NO: 64.

In preferred embodiments, the coding region encoding the Klotho polypeptide comprises SEQ ID NO: 64, 65, 66 or 67, preferably SEQ ID NO: 64.

The sequence of the GC62 variant of the full soluble native human Klotho isoform 1 (amino acids M1 to S981 of SEQ ID NO: 49; corresponding to SEQ ID NO: 57) is set forth in SEQ ID NO: 18. This sequence was also used in the context of the comparative experiments referred to above and described in more detail below in the Examples and Figures. Therefore, in a further preferred embodiment, the coding region encoding the Klotho polypeptide comprises an RNA sequence having at least 80 % sequence identity to SEQ ID NO: 18, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 18 or 20, especially SEQ ID NO: 18.

In further preferred embodiments, the coding region encoding the Klotho polypeptide comprises SEQ ID NO: 18, 19, 20 or 21, preferably SEQ ID NO: 18.

The sequence of the GC62 variant of the full native human Klotho isoform 1 (coding for the full amino acid sequence of SEQ ID NO: 49) is set forth in SEQ ID NO: 68. As explained above with respect to SEQ ID NO: 64, this sequence also has the advantage that it allows delivering a membrane-anchored version of the Klotho protein, which makes it possible to exploit the full breadth of Klotho signalling rather than just the soluble part of it. Therefore, in a further preferred embodiment the coding region encoding the Klotho polypeptide comprises an RNA sequence having at least 80 % sequence identity to SEQ ID NO: 68, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 68 or 70, especially SEQ ID NO: 68.

In preferred embodiments, the coding region encoding the Klotho polypeptide comprises SEQ ID NO: 68, 69, 70 or 71, preferably SEQ ID NO: 64.

The sequence of the GC62 variant of KL1-end domains of the native human Klotho isoform 2 (amino acids L56 to H549 of SEQ ID NO: 50) is set forth in SEQ ID NO: 33. Therefore, in a preferred embodiment, the coding region encoding the Klotho polypeptide comprises an RNA sequence having at least 80 % sequence identity to SEQ ID NO: 33, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 33 or 35, especially SEQ ID NO: 33.

In further preferred embodiments, the coding region encoding the Klotho polypeptide comprises SEQ ID NO: 33, 34, 35 or 36, preferably SEQ ID NO: 33.

The sequence of the GC62 variant of the lr-KL1-end domains of the native human Klotho isoform 2 (amino acids E34 to H549 of SEQ ID NO: 50) is set forth in SEQ ID NO: 33. This sequence codes for the full sequence of soluble Klotho isoform 2 except for the N-Terminal SP. Therefore, in a further preferred embodiment, the coding region encoding the Klotho polypeptide comprises an RNA sequence having at least 80 % sequence identity to SEQ ID NO: 37, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 37 or 39, especially SEQ ID NO: 37.

In further preferred embodiments, the coding region encoding the Klotho polypeptide comprises SEQ ID NO: 37, 38, 39 or 40, preferably SEQ ID NO: 37.

The sequence of the GC62 variant of the full soluble native human Klotho isoform 2 (amino acid sequence SEQ ID NO: 50) is set forth in SEQ ID NO: 41. Therefore, in a further preferred embodiment the coding region encoding the Klotho polypeptide comprises an RNA sequence having at least 80 % sequence identity to SEQ ID NO: 41, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 41 or 43, especially SEQ ID NO: 41.

In preferred embodiments, the coding region encoding the Klotho polypeptide comprises SEQ ID NO: 41, 42, 43 or 44, preferably SEQ ID NO: 41.

In a particularly preferred embodiment, the coding region encoding the Klotho polypeptide has at least 80 % sequence identity to SEQ ID NO: 5, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 5, 6, 7, or 8.

In a further particularly preferred embodiment, the coding region encoding the Klotho polypeptide has at least 80 % sequence identity to SEQ ID NO: 18, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 18, 19, 20, or 21. In further embodiments, the Klotho polypeptide is SEQ ID NO: 22, 23, or 24.

In a further particularly preferred embodiment, the coding region encoding the Klotho polypeptide has at least 80 % sequence identity to SEQ ID NO: 68, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 68, 69, 70, or 71.

In a further particularly preferred embodiment, the coding region encoding the Klotho polypeptide has at least 80 % sequence identity to SEQ ID NO: 41, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the coding region encoding the Klotho polypeptide is SEQ ID NO: 41, 42, 43, or 44.

As laid out herein above, it is preferred in the context of the invention if the Klotho polypeptide comprises the signal peptide, the KL1 domain and/or the KL2 domain of human Klotho.

In a preferred embodiment, the KL1 domain of the Klotho polypeptide has at least 80 % sequence identity to SEQ ID NO: 52, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, preferably wherein the KL1 domain of the Klotho polypeptide is SEQ ID NO: 52 or 53, especially SEQ ID NO: 52.

In a further preferred embodiment, the KL2 domain of the Klotho polypeptide has at least 80 % sequence identity to SEQ ID NO: 54, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, especially wherein the KL2 domain of the Klotho polypeptide is SEQ ID NO: 54.

In yet a further preferred embodiment, the signal peptide of the Klotho polypeptide has at least 80 % sequence identity to SEQ ID NO: 51, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, especially wherein the signal peptide of the Klotho polypeptide is SEQ ID NO: 51.

It is particularly preferred when the above-described embodiments relating to the SP, the KL1 domain and the KL2 domain are combined. For instance, it is preferred when the Klotho polypeptide comprises a KL1 domain of the Klotho polypeptide having at least 80 % sequence identity to SEQ ID NO: 52, a KL2 domain of the Klotho polypeptide having at least 80 % sequence identity to SEQ ID NO: 54, and/or a signal peptide of the Klotho polypeptide having at least 80 % sequence identity to SEQ ID NO: 51.

In a further preferred embodiment, the Klotho polypeptide has at least 90 % sequence identity to SEQ ID NO: 55, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 55 or 56, preferably 55.

In a further preferred embodiment, the Klotho polypeptide has at least 90 % sequence identity to SEQ ID NO: 57, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 57 or 58, preferably 57.

In a further preferred embodiment, the Klotho polypeptide has at least 90 % sequence identity to SEQ ID NO: 49, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 49 or 76, preferably 49.

In yet a further preferred embodiment, the Klotho polypeptide has at least 90 % sequence identity to SEQ ID NO: 59, preferably at least 95 %, more preferred at least 96 %, more preferred at least 97 %, more preferred at least 98 %, more preferred at least 99 %, more preferred at least 99.5 %, more preferred 100 %. It is especially preferred if the Klotho polypeptide is SEQ ID NO: 59 or 60, preferably 59.

In the context of the present invention, several specific sequences for the 5'-UTR and the 3'-UTR were found to be particularly advantageous, especially for achieving high expression levels.

Specifically, for the 5'-UTR, the sequence of SEQ ID NO: 61 was found to be advantageous. Therefore, in a preferred embodiment of the Klotho mRNA, the 5'-UTR has at least 70 % sequence identity to SEQ ID NO: 61, preferably at least 75 %, more preferred at least 80 %, more preferred at least 85 %, more preferred at least 90 %, more preferred at least 95 %, more preferred at least 96 %, more preferred at least 97 %, more preferred at least 98 %, more preferred at least 99 %, more preferred at least 99.5 %, more preferred 100 %. It is especially preferred if the 5'-UTR is SEQ ID NO: 61.

Similarly, for the 3'-UTR it was found that the sequence of SEQ ID NO: 62 is particularly advantageous. Therefore, in a preferred embodiment, the 3'-UTR has at least 70 % sequence identity to SEQ ID NO: 62, preferably at least 75 %, more preferred at least 80 %, more preferred at least 85 %, more preferred at least 90 %, more preferred at least 95 %, more preferred at least 96 %, more preferred at least 97 %, more preferred at least 98 %, more preferred at least 99 %, more preferred at least 99.5 %, more preferred 100 %. It is especially preferred if the 3'-UTR is SEQ ID NO: 62.

According to a preferred embodiment of the present invention, the present mRNA comprises in the 5'-UTR and/or 3'-UTR (preferably in the 3'UTR) one or more stabilization sequences that are capable of increasing the half-life of the mRNA intracellularly. These stabilization sequences may exhibit a 100% sequence homology with naturally occurring sequences that are present in viruses, bacteria and eukaryotic cells, but may however also be partly or completely synthetic. Examples for such stabilizing sequences are described in: Nucleic Acids Res. 2010; 38 (Database issue): D75-D80. UTRdb and UTRsite (RELEASE 2010): a collection of sequences and regulatory motifs of the untranslated regions of eukaryotic mRNAs and under http://utrdb.ba.itb.cnr.it/.

In an embodiment of the Klotho mRNA the 5'-UTR or 3'-UTR or the 5'-UTR and the 3'-UTR correspond to native human Klotho mRNA, preferably native human Klotho mRNA as set forth in NCBI Reference Sequence: NM_004795.4. Alternative, in another preferred embodiment, the 5'-UTR or 3'-UTR or the 5'-UTR and the 3'-UTR are different from native human Klotho mRNA.

As a further example of stabilizing sequences that may be used in the present invention, the non-translated sequences (UTR) of the β-globin gene, for example of Homo sapiens or Xenopus laevis, may be mentioned.

Another example of a stabilization sequence has been described in Holcik et al. (Proc. Natl. Acad. Sci. USA 1997, 94: 2410 to 2414) and has the general formula:
(C/U)CCANₓCCC(U/A)PyₓUC(C/U)CC (SEQ ID NO: 63), which is contained in the 3'UTR of the very stable mRNAs that code for example for alpha(1)-collagen or for alpha globin, and ALOX15 or for tyrosine hydroxylase (and wherein "x" is (independently in Nₓ and Pyₓ) an integer of 0 to 10, preferably of 0 to 5 (Holcik et al., 1997), especially 0, 1, 2, 4 and/or 5).

Such stabilization sequences may be used individually or in combination with one another for stabilizing the inventive mRNA as well as in combination with other stabilization sequences known to the person skilled in the art. E.g.: The stabilizing effect of human β-globin 3'-UTR sequences is further augmented by using two human β-globin 3'-UTRs arranged in a head-to-tail orientation.

Accordingly, a preferred embodiment of the Klotho mRNA according to the present invention is an mRNA molecule, wherein the 5'-UTR or 3'-UTR or the 5'-UTR and the 3'-UTR are different from the native Klotho mRNA, preferably wherein the 5'-UTR or 3'-UTR or the 5'-UTR and the 3'-UTR contain at least one stabilization sequence, preferably a stabilization sequence with the general formula (C/U)CCANₓCCC(U/A)PyₓUC(C/U)CC (SEQ ID NO:38).

Preferably, the 5'-UTR and/or 3'-UTR are the 5'-UTR and/or 3'-UTR of a different human mRNA than Klotho, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.

Accordingly, the present invention preferably relates to an mRNA which comprises in the 3'-UTR one or more stabilization sequences that are capable of increasing the half-life of the mRNA in the cytosol. These stabilization sequences may exhibit a 100% sequence homology with naturally occurring sequences that are present in viruses, bacteria and eukaryotic cells, but may, however, also be partly or completely synthetic. As an example of stabilizing sequences that may be used in the present invention, the non-translated sequences (UTR) of the β-globin gene, for example of homo sapiens or xenopus laevis, may be mentioned. As already stated, another example of a stabilization sequence has the general formula (C/U)CCANₓCCC(U/A)PyₓUC(C/U)CC, which is contained in the 3'-UTR of the very stable mRNA that codes for alpha-globin, alpha-(1)-collagen, 15-lipoxygenase or for tyrosine hydroxylase (c.f. Holcik et al., Proc. Natl. Acad. Sci. USA 1997, 94: 2410 to 2414). Such stabilization sequences may be used individually or in combination with one another for stabilizing the inventive modified mRNA as well as in combination with other stabilization sequences known to the person skilled in the art.

Another preferred embodiment of the present invention is the 5'-TOP-UTR derived from the ribosomal protein 32L, followed by a stabilizing sequence derived from the albumin-3'-UTR.

Accordingly, a preferred embodiment of the Klotho mRNA according to the present invention is an mRNA molecule containing a tract of multiple adenosine monophosphates at the 3' end of the 3'-UTR. This so-called poly-adenosine (poly-A) tail consists of at least 60 adenosine monophosphates, preferably at least 100 and most preferably at least 120 adenosine monophosphates.

In certain cases, destabilizing the mRNA might be desirable as well to limit the duration of protein production. This effect can be achieved by incorporating destabilizing sequence elements (DSE) like AU-rich elements into 3'-UTRs, thus ensuring rapid mRNA degradation and a short duration of protein expression.

Although it may be desired for certain embodiments to provide an mRNA which is devoid of destabilizing sequence elements (DSE) in the 3' and/or 5' UTR, there may be other embodiments, wherein the presence or introduction of such DSEs is advantageous. In general, a "DSE" refers to a sequence, which reduces the half-life of a transcript, e.g. the half-life of the mRNA according to the present invention inside a cell and/or organism, e.g. a human patient. Accordingly, a DSE comprises a sequence of nucleotides, which reduces the intracellular half-life of an RNA transcript.

DSE sequences are found in short-lived mRNAs such as, for example: c-fos, c-jun, c-myc, GM-CSF, IL-3, TNF-alpha, IL-2, IL-6, IL-8, IL-10, Urokinase, bcl-2, SGL T1 (Na(+)-coupled glucose transporter), Cox-2 (cyclooxygenase 2), PAI-2 (plasminogen activator inhibitor type 2), beta(1)-adrenergic receptor or GAP43 (5'-UTR and 3'-UTR).

Further DSEs are AU-rich elements (AREs) and/or U-rich elements (UREs), including single, tandem or multiple or overlapping copies of the nonamer UUAUUUA(U/A)(U/A) (where U/A is either an A or a U) and/or the pentamer AUUUA and/or the tetramer AUUU. Further DSEs are described in Nucleic Acids Res. 2010; 38 (Database issue) : D75-D80. UTRdb and UTRsite (RELEASE 2010): a collection of sequences and regulatory motifs of the untranslated regions of eukaryotic mRNAs and under http://utrdb.ba.itb.cnr.it/.

Accordingly, it may also be preferred if the 5'-UTR or 3'-UTR or the 5'-UTR and the 3'-UTR contain at least one destabilization sequence element (DSE), preferably AU-rich elements (AREs) and/or U-rich elements (UREs), especially a single, tandem or multiple or overlapping copies of the nonamer UUAUUUA(U/A) (U/A), such as the pentamer AUUUA and/or the tetramer AUUU (the term "U/A" meaning either A or U).

These stabilizing and destabilizing elements can be used alone or in combination to aim at a given duration of protein production and to individualize the treatment of the present invention to the local skin hypotrophy conditions, especially atrophic skin condition, the severity of affected skin and/or the specific group of patients.

General concepts for improved mRNA-based therapeutics (see e.g. Sahin et al., Nat. Rev. Drug Disc. 2014. 13(10): 759-780) are also applicable for the present invention.

Although in most cases, the use of exclusively canonical nucleotides may be preferred, there may be certain occasions where the Klotho mRNA according to the present invention may contain other monophosphate nucleosides than those of cytidine (C), uridine (U), adenosine (A) or guanosine (G) residues (the canonical nucleotides). There are a significant number of naturally occurring analogs of these monophosphate nucleosides and also (even more) synthetic variants of these mRNA residues. Embodiments of such variants can be found e.g. in WO 2014/153052 A2.

According to an embodiment, in the present Klotho mRNA, at least 5%, preferably at least 10%, more preferably at least 30%, especially at least 50% of all
- cytidine residues are replaced by 5-methyl-cytidine residues, and/or
- cytidine residues are replaced by 2-amino-2-deoxy-cytidine residues, and/or
- cytidine residues are replaced by 2-fluoro-2-deoxy-cytidine residues, and/or
- cytidine residues are replaced by 2-thio-cytidine residues, and/or
- cytidine residues are replaced by 5-iodo-cytidine residues, and/or
- uridine residues are replaced by pseudo-uridine residues, and/or
- uridine residues are replaced by 1-methyl-pseudo-uridine residues, and/or
- uridine residues are replaced by 2-thio-uridine residues, and/or
- uridine residues are replaced by 5-methyl-uridine residues, and/or
- adenosine residues are replaced by N6-methyl-adenosine residues.

Specific embodiments are Klotho mRNAs, wherein in the Klotho mRNA, at least 5%, preferably at least 10%, more preferably at least 30%, especially at least 50% of all
- cytidine residues are replaced by 5-methyl-cytidine residues, and/or
- uridine residues are replaced by pseudo-uridine residues, and/or
- uridine residues are replaced by 2-thio-uridine residues.

In a further preferred embodiment of the Klotho mRNA, the coding region has a codon adaption index (CAI) of at least 0.70, preferably at least 0.75, more preferred at least 0.80, more preferred at least 0.85, more preferred at least 0.86, more preferred at least 0.87, more preferred at least 0.88, more preferred at least 0.89, more preferred at least 0.90, more preferred at least 0.91, more preferred at least 0.92, more preferred at least 0.93, more preferred at least 0.94, more preferred at least 0.95, more preferred at least 0.96, more preferred at least 0.97, more preferred at least 0.98, more preferred at least 0.99, more preferred 1.00. However, surprisingly, also a lower CAI can be beneficial to improve expression levels. Therefore, it is preferred if the coding region has a codon adaption index (CAI) of below 1.00, preferably below 0.99, more preferred below 0.98, more preferred below 0.97, more preferred below 0.96, more preferred below 0.95.

The CAI is a measurement of the relative adaptiveness of the codon usage of a gene towards the codon usage of highly expressed genes. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (Sharp et al., Nucleic Acids Res. 15 (1987): 1281-1295., Jansen et al., Nucleic Acids Res. 31 (2003): 2242-2251).

In a particularly preferred embodiment of the Klotho mRNA, the Klotho mRNA has at least 80 % sequence identity to SEQ ID NO: 29, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the Klotho mRNA is SEQ ID NO: 29, 30, 31 or 32, preferably SEQ ID NO: 29.

In a further particularly preferred embodiment of the Klotho mRNA, the Klotho mRNA has at least 80 % sequence identity to SEQ ID NO: 72, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the Klotho mRNA is SEQ ID NO: 72, 73, 74 or 75, preferably SEQ ID NO: 72.

In a further particularly preferred embodiment of the Klotho mRNA, the Klotho mRNA has at least 80 % sequence identity to SEQ ID NO: 45, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %. It is especially preferred if the Klotho mRNA is SEQ ID NO: 45, 46, 47 or 48, preferably SEQ ID NO: 45.

In another aspect, the present invention relates to a pharmaceutical formulation comprising the Klotho mRNA according to any one of the preceding Embodiments.

Suitable formulations for mRNA therapeutics are well available in the art (see e.g. Sahin et al., 2014; WO 2014/153052 A2 (paragraphs 122 to 136), etc.).

The present invention therefore also relates to a pharmaceutical formulation comprising a Klotho mRNA according to the present invention. The present formulation preferably comprises the mRNA in a pharmaceutically acceptable environment, e.g. with suitable components usually provided in mRNA therapeutics (excipients, carriers, buffers, auxiliary substances (e.g. stabilizers), etc.)

The mRNA formulations according to the present invention preferably contain a suitable carrier. Suitable carriers include polymer based carriers, such as cationic polymers including linear and branched PEI and viromers, lipid nanoparticles and liposomes, nanoliposomes, ceramide-containing nanoliposomes, proteoliposomes, cationic amphiphilic lipids e.g: SAINT^{®}-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, small-molecule targeted conjugates, poly-lactic-co-glycolic acid (PLGA) polymers and other vectorial tags. Also contemplated is the use of bionanocapsules and other viral capsid protein assemblies as a suitable carrier. (Hum. Gene Ther. 2008 Sep;19(9):887-95).

Preferred carriers are cationic polymers including linear and branched PEI and viromers, lipid nanoparticles and liposomes, transfersomes, and nanoparticulates including calcium phosphate nanoparticulates (i.e. naked RNA precipitated with CaCl₂ and then administered).

A preferred embodiment of the present invention relates to the use of non-complexed mRNA, i.e. non-complexed mRNA in a suitable aqueous buffer solution, preferably a physiological glucose buffered aqueous solution (physiological). For example, a 1xHEPES buffered solution; a 1xPhosphate buffered solution, Na-Citrate buffered solution; Na-Acetate buffered solution; Ringer's Lactate solution; preferred with Glucose (e.g.: 5% Glucose); physiologic solutions can be preferably applied.

Preferably the present invention applies liposomes, especially liposomes which are based on DOTAP, DOTMA, Dotap-DOPE, DOTAP-DSPE, Dotap-DSPE-PEG, Dotap-DOPE-PEG, Dotap-DSPE-PEG-Na-Cholate, Dotap-DOPE-PEG-Na-Cholate, DOTAP with cationic amphiphilic macromolecules (CAM) as complexes, and combinations thereof.

In a preferred embodiment of the inventive pharmaceutical formulation, the formulation comprises a further mRNA, wherein the further mRNA has a further 5' CAP region, a further 5'-UTR, a further coding region encoding a further polypeptide, a further 3'-UTR and a further poly-A tail.

The further polypeptide preferably causes further beneficial effects when expressed in the cells of an individual. It is particularly preferred if the further polypeptide has an additive, or, preferably, a synergistic effect with the Klotho polypeptide. Preferably, the further polypeptide is an anti-aging peptide or an anti-aging protein, i.e., any peptide or protein known to the skilled person to have an anti-aging effect. Alternatively, or in addition, it is preferred if the further polypeptide has a therapeutic effect, preferably, a therapeutic effect in relation to any of the therapeutic indications described herein.

In a preferred embodiment, the further polypeptide is Bactericidal/Permeability Increasing Protein Family B, member 4 (BPIFB4) protein or an isoform or variant thereof, preferably Longevity-Associated Variant of BPIFB4 (LAV-BPIFB4). Preferably, BPIFB4 is as set forth in UniProt Entry P59827-1 or P59827-2, especially P59827-1 (Sequence version 2). Preferably, the LAV-variant is as set forth in UniProt Entry P59827-1 or P59827-2, especially P59827-1 (Sequence version 2), wherein the following amino acids are substituted: Ile229Val, Asn281Thr, Leu488Phe and Ile494Thr.

In a preferred embodiment, the BPIFB4 has at least one, preferably at least two, preferably at least three mutations selected from Ile229Val, Asn281Thr, Leu488Phe and Ile494Thr; preferably the BPIFB4 has all of the named mutations.

In further preferred embodiments, the further polypeptide is a BPIFB4 protein or an isoform or variant thereof as set forth in WO 2014/102343 A1 or WO 2019/034723 A1, both of which are incorporated herein by reference.

In further preferred embodiments, the further polypeptide is a protein described in Bin-Jumah et al. "Genes and longevity of lifespan." International Journal of Molecular Sciences 23.3 (2022): 1499. In particular, it is preferred if the further polypeptide is a protein selected from Apolipoprotein E (preferably gene symbol: APOE), Tumor protein p53 (preferably gene symbol: P53), Sirtuin 1 protein (preferably gene symbol: SIRT1), FOXO1 transcription factor (preferably gene symbol: DAF-16), Cholinergic receptor nicotinic alpha 3 subunit (preferably gene symbol: CHRNA3), SH2B adaptor protein 3 (preferably gene symbol: SH2B3), Cyclin dependent kinase inhibitor 2A (preferably gene symbol: CDKN2A), Elongation of very-long-chain fatty acids-like 2 (preferably gene symbol: ELOVL2), Werner protein (preferably gene symbol: WRN), Paraoxonase 1 (preferably gene symbol: PON1), Superoxide dismutase 2 (preferably gene symbol: SOD2), Lamin A protein (preferably gene symbol: LMNA), Cholesteryl ester transfer protein (preferably gene symbol: CETP), Apolipoprotein C3 (preferably gene symbol: APOC3), Microsomal triglyceride transfer protein (preferably gene symbol: MTP), Phosphatidylinositol 3-kinase (PI3K) (preferably gene symbol: PIK3CA), Insulin-like growth factor 1 (IGF-1) receptor (preferably gene symbol: DAF-2), Protein-L-iso-aspartyl methyltransferase (preferably gene symbol: PIMT), Growth hormone (preferably gene symbol: GH1), cAMP-response element binding protein (preferably gene symbol: CREB), Mitogen-activated protein kinase (preferably gene symbol: MAPK), epidermal growth factor receptor (preferably gene symbol: EGFR), Nuclear factor kappa B (preferably gene symbol: NF-kB), Phospholipase C beta (preferably gene symbol: PLC-β), Methionine sulfoxide reductase A (preferably gene symbol: MSR-A), Mediator of cell motility 1 (preferably gene symbol: MEMO1), Nei like DNA Glycosylase 1 (preferably gene symbol: NEIL1), Peroxisome proliferator-activated receptor gamma 2 (preferably gene symbol: PPARγ2), Eukaryotic translation initiation factor 3 subunit K (preferably gene symbol: EIF3K), ATM serine/threonine kinase (preferably gene symbol: ATM), B-cell lymphoma 2 (preferably gene symbol: BCL2), Cell division cycle 42 (preferably gene symbol: CDC42), Diacylglycerol O-acyltransferase 1 (preferably gene symbol: DGAT1), Early growth response 1 (preferably gene symbol: EGR1), Fibroblast growth factor 23 (preferably gene symbol: FGF23), Fibroblast growth factor 21 (preferably gene symbol: FGF21), Fructosamine 3 kinase related protein (preferably gene symbol: FN3KRP), Phosphoglycolate phosphatase (preferably gene symbol: PGP), Insulin receptor substrate 1 (preferably gene symbol: IRS1), Polycomb complex protein BMI-1 (preferably gene symbol: BMI1), Neuregulin 1 (preferably gene symbol: NRG-1), Signal transducer and activator of transcription (preferably gene symbol: STAT), E2F Transcription Factor 1 (preferably gene symbol: E2F1), Vascular endothelial growth factor A (preferably gene symbol: VEGF-A), Xenobiotic metabolizing enzymes (preferably gene symbol: XME), Myc proto-oncogene protein (preferably gene symbol: MYC), C-X-C chemokine receptor type 4 (preferably gene symbol: CXCR4), Silent information regulator 2 (preferably gene symbol: SIR-2), Extracellular signal-regulated kinase (preferably gene symbol: ERK), SLC31 (preferably gene symbol: SLC31). For each protein, the native human version is preferred. All isoforms and/or variants of these proteins are included. When multiple isoforms or variants exist, the isoform or variant that is most common in the human population is preferred.

According to another aspect, the present invention relates to a kit for administering the Klotho mRNA according to any one of the preceding Embodiments to an individual comprising
- the Klotho mRNA according to any one of the preceding Embodiments, and
- a device for administering the Klotho mRNA.

Preferably the individual is an animal, preferably a human or a non-human animal. It is particularly preferred if the individual is a mammal, especially a human.

In a preferred embodiment of the inventive kit, the device is for intravenous, intramuscular, subcutaneous, intradermal, transdermal, epidermal, or topical administration.

Preferably, the device for administering the Klotho mRNA is a syringe, a needle, an autoinjector, and/or a needle-free injection system; preferably wherein the device is for intravenous and/or intramuscular injection. It is especially preferred if the device for administering the Klotho mRNA is a skin delivery device. In preferred embodiments, the skin delivery device is an intradermal delivery device, preferably selected from the group consisting of needle based injection systems. For instance, the skin delivery device can be a transdermal delivery device, preferably selected from the group consisting of transdermal patches, hollow and solid microneedle systems, microstructured transdermal systems, electrophoresis systems, and iontophoresis systems. The skin delivery device may also be an epidermal delivery device, preferably selected from the group consisting of needle free injection systems, laser based systems, especially Erbium YAG laser systems, and gene gun systems.

These administration devices are in principle available in the art; adaption to the administration of the mRNA according to the present invention is easily possible for a person skilled in the art.

A further aspect of the present invention relates to the inventive Klotho mRNA for use as a medicament.

Therapeutic uses of Klotho are known in the art. The present invention provides a particularly advantageous way of delivering these therapeutic effects to individuals in need thereof. Therapeutic uses of Klotho are e.g. described in Prud'homme et al. "Pathobiology of the Klotho Antiaging Protein and Therapeutic Considerations." Frontiers in Aging 3 (2022); as well as in Sachdeva, et al. "Klotho and the treatment of human malignancies." Cancers 12.6 (2020): 1665.

Therefore, in an aspect, the invention relates to the inventive Klotho mRNA or the inventive pharmaceutical composition for use in the prevention or treatment of a disease selected from the group consisting of kidney diseases, cardiovascular diseases, brain diseases, lung diseases, bone diseases, and metabolic diseases.

In another aspect, the invention relates to the inventive Klotho mRNA or the inventive pharmaceutical composition for use in the prevention or treatment of a kidney disease, preferably in the prevention or treatment of a disease selected from the group consisting of chronic kidney disease, fibrosis, hyperphosphatemia, ischemic injury, nephrectomy, toxic injury, diabetic nephropathy, and calciprotein deposition.

In another aspect, the invention relates to the inventive Klotho mRNA or the inventive pharmaceutical composition for use in the prevention or treatment of a cardiovascular disease, preferably in the prevention or treatment of a disease selected from the group consisting of arterial/aortic calcification, atherosclerosis, cardiomyopathy, cardiac hypertrophy, hypertension, and myocardial ischemic injury/infarct.

In another aspect, the invention relates to the inventive Klotho mRNA or the inventive pharmaceutical composition for use in the prevention or treatment of a brain disease, preferably in the prevention or treatment of a disease selected from the group consisting of Alzheimer's disease, hippocampal neuronal loss, cognitive deficits, and frailty.

In another aspect, the invention relates to the inventive Klotho mRNA or the inventive pharmaceutical composition for use in the prevention or treatment of cancer, preferably in the prevention or treatment of a cancer selected from the group consisting of colorectal cancer, esophageal cancer, gastric cancer, pancreatic cancer, breast cancer, lung cancer, ovarian cancer, thyroid cancer, melanoma, renal cancer, and cervical cancer.

In another aspect, the invention relates to the inventive Klotho mRNA or the inventive pharmaceutical composition for use in the prevention or treatment of a lung disease, preferably in the prevention or treatment of pulmonary fibrosis or chronic obstructive pulmonary disease.

In another aspect, the invention relates to the inventive Klotho mRNA or the inventive pharmaceutical composition for use in the prevention or treatment of a bone disease, preferably in the prevention or treatment of osteoporosis or osteomalacia, especially osteomalacia from chronic kidney disease.

In another aspect, the invention relates to the inventive Klotho mRNA or the inventive pharmaceutical composition for use in the prevention or treatment of a metabolic disease, preferably in the prevention or treatment of a disease selected from the group consisting of diabetes, in particular type 1 and/or type 2 diabetes, pancreatic β-cell apoptosis, autoimmune disorders, and inflammation, especially insulitis.

In relation to all aspects described herein (both the therapeutic and the non-therapeutic uses described herein), it is preferred if the inventive Klotho mRNA or the inventive composition is administered intravenously, intramuscularly, subcutaneously, intradermally, transdermally, epidermally, or topically, especially epidermally.

Administration of the Klotho mRNA or of the pharmaceutic composition comprising the Klotho mRNA can be performed according to optimised expression aims, depending on the amount of mRNA applied, the stability of the mRNA molecule and the status of disease. For example, the Klotho mRNA / the pharmaceutical composition may be administered at least once, at least twice, at least twice within one month, preferably weekly. For example, if the Klotho mRNA / the pharmaceutical composition may be administered at least twice, at least twice within one month, preferably weekly doses applied may vary.

The amount of mRNA delivered per dose may also be made dependent on the stability of the molecule, etc.. Preferably the Klotho mRNA / the pharmaceutical composition is administered in an amount of 0.01µg to 100 mg per dose, preferably of 0.1µg to 10mg per dose, especially of 1µg to 1mg per dose.

The present invention also relates to a method for preventing or treating a disease in an individual in need thereof, comprising administering an effective amount of the inventive Klotho mRNA or of the inventive pharmaceutical composition to the individual. All features and preferred embodiments described herein with respect to the Klotho mRNA for use or the pharmaceutical composition for use are also preferred embodiments of the method for treatment, e.g., as relates to the disease to be prevented or treated, the dosing regime, the method of administration, etc.

In another aspect, the invention relates to the inventive Klotho mRNA or the inventive pharmaceutical composition for use in the prevention or treatment of an aging-related disorder and/or a symptom of aging. Preferably, the aging-related disorder or symptom of aging is selected from one or more of actinic keratosis, age-related macular degeneration (AMD), Alzheimer's disease, arthritis, atherosclerosis and cardiovascular disease, benign prostatic hyperplasia (BPH), bone atrophy, cachexia, cancer, cardiomyopathy, cataracts, chronic obstructive pulmonary disease (COPD), constipation, decrease in overall energy, decrease in visual acuity, delirium, dementia, depression, dermal atrophy (thinning of the skin), diminished peripheral vision, greater risk of heat stroke or hypothermia, hearing loss, hypertension, increased susceptibility to infection (including influenza and pneumonia), lentigines (aging spots), liver conditions (e.g ., nonalcoholic fatty liver disease, nonalcoholic steatohepatitis, and cirrhosis), memory loss, metabolic syndrome, muscle atrophy (e.g., Sarcopenia and myopenia), frailty, muscle repair or rejuvenation deficiency, muscular dystrophy, osteoarthritis, osteoporosis, periodontitis, photoaging, reduced metabolism (including increased risk for obesity), reduced reflexes and coordination including difficulty with balance, respiratory disease (including acute respiratory distress syndrome (ARDS) with or without acute lung injury (ALI) and pulmonary fibrosis (e.g. idiopathic pulmonary fibrosis)), rheumatoid arthritis, sarcopenic obesity, sexual dysfunction, shingles, type 2 diabetes, urologic changes (including incontinence), vaginal atrophy, whitening or graying of hair, prolonged/inefficient wound healing, wrinkling/sagging skin (including loss of skin elasticity), and xerosis cutis (skin dryness); or the disease includes asthma, deafness, or a viral infections and/or a symptom of the disease comprises sepsis.

In another aspect, the present invention also relates to the use of the Klotho mRNA or the pharmaceutical composition according to the invention for preventing and/or reducing a symptom of aging.

In yet another aspect, the invention relates to the use of the Klotho mRNA or the pharmaceutical composition according to the invention for increasing the lifespan in an individual, preferably in a mammal, preferably a human.

It is preferred, if the above uses are non-therapeutic uses, especially the use for increasing the lifespan. Therefore, it is preferred if the individual is a healthy individual. It is preferred if the individual does not suffer from any of the diseases described herein.

In yet another aspect, the present invention relates to a Cosmetic formulation comprising the Klotho mRNA according to the invention. Preferably, the cosmetic formulation comprises a cosmetic carrier. Preferably, cosmetic carrier is an ointment, a gel, especially a hydrogel, a liposome, nano/microparticles, or an emulsion.

According to a further aspect, the present invention also relates to a cosmetic skin care method, comprising contacting a skin of an individual, preferably a human individual, with the Klotho mRNA or the cosmetic formulation according to the invention. Preferably, the skin is an ageing skin. It is especially preferred, if the cosmetic skin care method reduces any sign of aging, such as wrinkles.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The term "preventing" or "prevention" as used herein means to stop a disease state or condition from occurring in an individual completely or almost completely or at least to a (preferably significant) extent, especially when the individual is predisposed to such a risk of contracting a disease state or condition. However, these terms should not be interpreted as an absolute success in the sense that a patient can never develop an associated disease, reaction or condition but as the reduction of the chance of developing the disease, reaction or condition in a prophylactic treatment.

"Percent (%) sequence identity", "X% identical" (such as "70% identical") or similar terms with respect to a reference nucleotide sequence is defined as the percentage of nucleotides in a candidate sequence that are identical with the nucleotides in the reference sequence, after aligning the sequences and introducing gaps, if necessary, to achieve the maximum percent sequence identity, and not considering any conservative substitutions as part of the sequence identity. Gaps cause a lack of identity. Alignment for purposes of determining percent nucleotide sequence identity can be achieved in various ways that are within the skill in the art, for instance, using publicly available computer software such as BLAST, BLAST-2, ALIGN, ALIGN-2, Megalign (DNASTAR) or the "needle" pairwise sequence alignment application of the EMBOSS software package. Those skilled in the art can determine appropriate parameters for aligning sequences, including any algorithms needed to achieve maximal alignment over the full length of the sequences being compared. For purposes herein, however, % nucleotide sequence identity values are calculated using the sequence alignment of the computer programme "needle" of the EMBOSS software package (publicly available from European Molecular Biology Laboratory; Rice et al., EMBOSS: the European Molecular Biology Open Software Suite, Trends Genet. 2000 Jun;16(6):276-7, PMID: 10827456).
The needle programme can be accessed under the web site http://www.ebi.ac.uk/Tools/psa/emboss_needle or downloaded for local installation as part of the EMBOSS package from http://emboss.sourceforge.net/. It runs on many widely-used UNIX operating systems, such as Linux.
To align two nucleotide sequences, the needle programme is preferably run with the following parameters:
Commandline: needle -auto -stdout -asequence SEQUENCE_FILE_A - bsequence SEQUENCE_FILE_B -datafile EDNAFULL -gapopen 10.0 -gapextend 0.5 -endopen 10.0 -endextend 0.5 -aformat3 pair -snucleotide1 -snucleotide2 (Align_format: pair Report_file: stdout)
The % nucleotide sequence identity of a given nucleotide sequence A to, with, or against a given nucleotide sequence B (which can alternatively be phrased as a given nucleotide sequence A that has or comprises a certain % nucleotide sequence identity to, with, or against a given nucleotide sequence B) is calculated as follows: 100 times the fraction X/Y
where X is the number of nucleotides scored as identical matches by the sequence alignment program needle in that program's alignment of A and B, and where Y is the total number of nucleotides in B. It will be appreciated that where the length of nucleotide sequence A is not equal to the length of nucleotide sequence B, the % nucleotide sequence identity of A to B will not equal the % nucleotide sequence identity of B to A. In cases where "a sequence of A is at least N% identical to the entire sequence of B", Y is the entire length of B. Unless specifically stated otherwise, all % nucleotide sequence identity values used herein are obtained as described in the immediately preceding paragraph using the needle computer program.

"Percent (%) sequence identity", "Percent (%) amino acid sequence identity", "X% identical" (such as "70% identical") or similar terms with respect to a reference polypeptide or protein sequence is defined in the same way as laid out above for nucleotide sequence identity, i.e., as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Gaps cause a lack of identity. Alignment for purposes of determining percent amino acid sequence identity is preferably done by the same methods as laid out above for nucleotide sequences. To align two protein sequences, the needle programme is preferably run with the following parameters:
Commandline: needle -auto -stdout -asequence SEQUENCE_FILE_A-bsequence SEQUENCE_FILE_B -datafile EBLOSUM62 -gapopen 10.0 - gapextend 0.5 -endopen 10.0 -endextend 0.5 -aformat3 pair -sprotein1-sprotein2 (Align_format: pair Report_file: stdout)
The % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y
where X is the number of amino acid residues scored as identical matches by the sequence alignment program needle in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. In cases where "the sequence of A is more than N% identical to the entire sequence of B", Y is the entire sequence length of B (i.e. the entire number of amino acid residues in B). Unless specifically stated otherwise, all % amino acid sequence identity values used herein are obtained as described in the immediately preceding paragraph using the needle computer program.

Herein, "UniProt" refers to the Universal Protein Resource. UniProt is a comprehensive resource for protein sequence and annotation data. UniProt is a collaboration between the European Bioinformatics Institute (EMBL-EBI), the SIB Swiss Institute of Bioinformatics and the Protein Information Resource (PIR). Across the three institutes more than 100 people are involved through different tasks such as database curation, software development and support. Website: http://www.uniprot.org/

Entries in the UniProt databases are identified by their accession codes (referred to herein e.g. as "UniProt accession code" or briefly as "UniProt" followed by the accession code), usually a code of six alphanumeric letters (e.g. "Q1HVF7"). If not specified otherwise, the accession codes used herein refer to entries in the Protein Knowledgebase (UniProtKB) of UniProt. If not stated otherwise, the UniProt database state for all entries referenced herein is of 2 March 2023 (UniProt/UniProtKB Release 2023_01).

In the context of the present application, sequence variants (designated as "natural variant" in UniProt) are expressly included when referring to a UniProt database entry.

Unless specified otherwise, all parameters as used herein correspond to parameters at IUPAC SATP-conditions ("Standard Ambient Temperature and Pressure"), in particular a temperature of 25 °C and a pressure of 101.300 Pa.

Percentages (%) as used herein correspond to weight per volume (w/v) unless specified as weight per weight (w/w) or otherwise.

The present invention relates to the following preferred embodiments:
Embodiment 1. Klotho messenger-RNA (mRNA), wherein the mRNA has a 5' CAP region, a 5' untranslated region (5'-UTR), a coding region encoding a Klotho polypeptide, a 3' untranslated region (3'-UTR) and a poly-adenosine Tail (poly-A tail), wherein the Klotho polypeptide comprises the KL1 domain of human Klotho.
Embodiment 2. The Klotho mRNA according to any one of the preceding Embodiments, wherein the Klotho polypeptide comprises the KL1 domain and the KL2 domain of human Klotho.
Embodiment 3. The Klotho mRNA according to any one of the preceding Embodiments, wherein the Klotho polypeptide comprises the signal peptide, the KL1 domain and the KL2 domain of human Klotho.
Embodiment 4. The Klotho mRNA according to any one of the preceding Embodiments, wherein the Klotho polypeptide further comprises the transmembrane (TM) domain of human Klotho, preferably wherein the Klotho polypeptide further comprises the cytoplasmic tail (CT) of human Klotho.
Embodiment 5. The Klotho mRNA according to any one of the preceding Embodiments, wherein the coding region encoding the Klotho polypeptide has a GC content of at least 50 %, preferably at least 51 %, more preferred at least 52 %, more preferred at least 53 %, more preferred at least 54 %, more preferred at least 55 %, more preferred at least 56 %, more preferred at least 57 %, more preferred at least 58 %, more preferred at least 59 %, more preferred at least 60 %, more preferred at least 61 %, more preferred at least 62 %, more preferred at least 63 %.
Embodiment 6. The Klotho mRNA according to any one of the preceding Embodiments, wherein the coding region encoding the Klotho polypeptide has a GC content of between 50 % and 74 %, preferably between 51 % and 73 %, more preferred between 52 % and 72 %, more preferred between 53 % and 71 %, more preferred between 54 % and 70 %, more preferred between 55 % and 69 %, more preferred between 56 % and 68 %, more preferred between 57 % and 67 %, more preferred between 58 % and 66 %, more preferred between 59 % and 65 %, more preferred between 60 % and 64 %, more preferred between 61 % and 63 %.
Embodiment 7. The Klotho mRNA according to any one of the preceding Embodiments, wherein the Klotho mRNA has a GC content of at least 50 %, preferably at least 51 %, more preferred at least 52 %, more preferred at least 53 %, more preferred at least 54 %, more preferred at least 55 %, more preferred at least 56 %, more preferred at least 57 %, more preferred at least 58 %, more preferred at least 59 %, more preferred at least 60 %, more preferred at least 61 %, more preferred at least 62 %, more preferred at least 63 %.
Embodiment 8. The Klotho mRNA according to any one of the preceding Embodiments, wherein the Klotho mRNA has a GC content of between 50 % and 74 %, preferably between 51 % and 73 %, more preferred between 52 % and 72 %, more preferred between 53 % and 71 %, more preferred between 54 % and 70 %, more preferred between 55 % and 69 %, more preferred between 56 % and 68 %, more preferred between 57 % and 67 %, more preferred between 58 % and 66 %, more preferred between 59 % and 65 %, more preferred between 60 % and 64 %, more preferred between 61 % and 63 %.
Embodiment 9. The Klotho mRNA according to any one of the preceding Embodiments, wherein the coding region encoding the Klotho polypeptide comprises an RNA sequence having at least 80 % sequence identity to SEQ ID NO: 1, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 1 or 3, especially SEQ ID NO: 1.
Embodiment 10. The Klotho mRNA according to any one of the preceding Embodiments, wherein the coding region encoding the Klotho polypeptide comprises SEQ ID NO: 1, 2, 3 or 4, preferably SEQ ID NO: 1.
Embodiment 11. The Klotho mRNA according to any one of the preceding Embodiments, wherein the coding region encoding the Klotho polypeptide comprises an RNA sequence having at least 80 % sequence identity to SEQ ID NO: 9, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 9 or 11, especially SEQ ID NO: 9.
Embodiment 12. The Klotho mRNA according to any one of the preceding Embodiments, wherein the coding region encoding the Klotho polypeptide comprises SEQ ID NO: 9, 10, 11 or 12, preferably SEQ ID NO: 9.
Embodiment 13. The Klotho mRNA according to any one of the preceding Embodiments, wherein the coding region encoding the Klotho polypeptide comprises an RNA sequence having at least 80 % sequence identity to SEQ ID NO: 13, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 13 or 15, especially SEQ ID NO: 13.
Embodiment 14. The Klotho mRNA according to any one of the preceding Embodiments, wherein the coding region encoding the Klotho polypeptide comprises SEQ ID NO: 13, 14, 15 or 16, preferably SEQ ID NO: 13.
Embodiment 15. The Klotho mRNA according to any one of the preceding Embodiments, wherein the coding region encoding the Klotho polypeptide comprises an RNA sequence having at least 80 % sequence identity to SEQ ID NO: 64, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 64 or 66, especially SEQ ID NO: 64.
Embodiment 16. The Klotho mRNA according to any one of the preceding Embodiments, wherein the coding region encoding the Klotho polypeptide comprises SEQ ID NO: 64, 65, 66 or 67, preferably SEQ ID NO: 64.
Embodiment 17. The Klotho mRNA according to any one of the preceding Embodiments, wherein the coding region encoding the Klotho polypeptide comprises an RNA sequence having at least 80 % sequence identity to SEQ ID NO: 18, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 18 or 20, especially SEQ ID NO: 18.
Embodiment 18. The Klotho mRNA according to any one of the preceding Embodiments, wherein the coding region encoding the Klotho polypeptide comprises SEQ ID NO: 18, 19, 20 or 21, preferably SEQ ID NO: 18.
Embodiment 19. The Klotho mRNA according to any one of the preceding Embodiments, wherein the coding region encoding the Klotho polypeptide comprises an RNA sequence having at least 80 % sequence identity to SEQ ID NO: 68, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 68 or 70, especially SEQ ID NO: 68.
Embodiment 20. The Klotho mRNA according to any one of the preceding Embodiments, wherein the coding region encoding the Klotho polypeptide comprises SEQ ID NO: 68, 69, 70 or 71, preferably SEQ ID NO: 64.
Embodiment 21. The Klotho mRNA according to any one of the preceding Embodiments, wherein the coding region encoding the Klotho polypeptide comprises an RNA sequence having at least 80 % sequence identity to SEQ ID NO: 33, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 33 or 35, especially SEQ ID NO: 33.
Embodiment 22. The Klotho mRNA according to any one of the preceding Embodiments, wherein the coding region encoding the Klotho polypeptide comprises SEQ ID NO: 33, 34, 35 or 36, preferably SEQ ID NO: 33.
Embodiment 23. The Klotho mRNA according to any one of the preceding Embodiments, wherein the coding region encoding the Klotho polypeptide comprises an RNA sequence having at least 80 % sequence identity to SEQ ID NO: 37, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 37 or 39, especially SEQ ID NO: 37.
Embodiment 24. The Klotho mRNA according to any one of the preceding Embodiments, wherein the coding region encoding the Klotho polypeptide comprises SEQ ID NO: 37, 38, 39 or 40, preferably SEQ ID NO: 37.
Embodiment 25. The Klotho mRNA according to any one of the preceding Embodiments, wherein the coding region encoding the Klotho polypeptide comprises an RNA sequence having at least 80 % sequence identity to SEQ ID NO: 41, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, preferably wherein the RNA sequence is SEQ ID NO: 41 or 43, especially SEQ ID NO: 41.
Embodiment 26. The Klotho mRNA according to any one of the preceding Embodiments, wherein the coding region encoding the Klotho polypeptide comprises SEQ ID NO: 41, 42, 43 or 44, preferably SEQ ID NO: 41.
Embodiment 27. The Klotho mRNA according to any one of the preceding Embodiments, wherein the coding region encoding the Klotho polypeptide has at least 80 % sequence identity to SEQ ID NO: 5, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %.
Embodiment 28. The Klotho mRNA according to any one of the preceding Embodiments, wherein the coding region encoding the Klotho polypeptide is SEQ ID NO: 5.
Embodiment 29. The Klotho mRNA according to any one of the preceding Embodiments, wherein the coding region encoding the Klotho polypeptide is SEQ ID NO: 6.
Embodiment 30. The Klotho mRNA according to any one of the preceding Embodiments, wherein the coding region encoding the Klotho polypeptide is SEQ ID NO: 7.
Embodiment 31. The Klotho mRNA according to any one of the preceding Embodiments, wherein the coding region encoding the Klotho polypeptide is SEQ ID NO: 8.
Embodiment 32. The Klotho mRNA according to any one of the preceding Embodiments, wherein the coding region encoding the Klotho polypeptide has at least 80 % sequence identity to SEQ ID NO: 18, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %.
Embodiment 33. The Klotho mRNA according to any one of the preceding Embodiments, wherein the coding region encoding the Klotho polypeptide is SEQ ID NO: 18.
Embodiment 34. The Klotho mRNA according to any one of the preceding Embodiments, wherein the coding region encoding the Klotho polypeptide is SEQ ID NO: 19.
Embodiment 35. The Klotho mRNA according to any one of the preceding Embodiments, wherein the coding region encoding the Klotho polypeptide is SEQ ID NO: 20.
Embodiment 36. The Klotho mRNA according to any one of the preceding Embodiments, wherein the coding region encoding the Klotho polypeptide is SEQ ID NO: 21.
Embodiment 37. The Klotho mRNA according to any one of the preceding Embodiments, wherein the coding region encoding the Klotho polypeptide is SEQ ID NO: 22.
Embodiment 38. The Klotho mRNA according to any one of the preceding Embodiments, wherein the coding region encoding the Klotho polypeptide is SEQ ID NO: 23.
Embodiment 39. The Klotho mRNA according to any one of the preceding Embodiments, wherein the coding region encoding the Klotho polypeptide is SEQ ID NO: 24.
Embodiment 40. The Klotho mRNA according to any one of the preceding Embodiments, wherein the coding region encoding the Klotho polypeptide has at least 80 % sequence identity to SEQ ID NO: 68, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %.
Embodiment 41. The Klotho mRNA according to any one of the preceding Embodiments, wherein the coding region encoding the Klotho polypeptide is SEQ ID NO: 68.
Embodiment 42. The Klotho mRNA according to any one of the preceding Embodiments, wherein the coding region encoding the Klotho polypeptide is SEQ ID NO: 69.
Embodiment 43. The Klotho mRNA according to any one of the preceding Embodiments, wherein the coding region encoding the Klotho polypeptide is SEQ ID NO: 70.
Embodiment 44. The Klotho mRNA according to any one of the preceding Embodiments, wherein the coding region encoding the Klotho polypeptide is SEQ ID NO: 71.
Embodiment 45. The Klotho mRNA according to any one of the preceding Embodiments, wherein the coding region encoding the Klotho polypeptide has at least 80 % sequence identity to SEQ ID NO: 41, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %.
Embodiment 46. The Klotho mRNA according to any one of the preceding Embodiments, wherein the coding region encoding the Klotho polypeptide is SEQ ID NO: 41.
Embodiment 47. The Klotho mRNA according to any one of the preceding Embodiments, wherein the coding region encoding the Klotho polypeptide is SEQ ID NO: 42.
Embodiment 48. The Klotho mRNA according to any one of the preceding Embodiments, wherein the coding region encoding the Klotho polypeptide is SEQ ID NO: 43.
Embodiment 49. The Klotho mRNA according to any one of the preceding Embodiments, wherein the coding region encoding the Klotho polypeptide is SEQ ID NO: 44.
Embodiment 50. The Klotho mRNA according to any one of the preceding Embodiments, wherein the KL1 domain of the Klotho polypeptide has at least 80 % sequence identity to SEQ ID NO: 52, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, preferably wherein the KL1 domain of the Klotho polypeptide is SEQ ID NO: 52 or 53, especially SEQ ID NO: 52.
Embodiment 51. The Klotho mRNA according to any one of the preceding Embodiments, wherein the KL2 domain of the Klotho polypeptide has at least 80 % sequence identity to SEQ ID NO: 54, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, especially wherein the KL2 domain of the Klotho polypeptide is SEQ ID NO: 54.
Embodiment 52. The Klotho mRNA according to any one of the preceding Embodiments, wherein the signal peptide of the Klotho polypeptide has at least 80 % sequence identity to SEQ ID NO: 51, preferably at least 85 %, more preferred at least 90 %, more preferred at least 95 %, more preferred at least 96 %, more preferred at least 97 %, more preferred at least 98 %, more preferred at least 99 %, more preferred at least 99.5 %, especially wherein the signal peptide of the Klotho polypeptide is SEQ ID NO: 51.
Embodiment 53. The Klotho mRNA according to any one of the preceding Embodiments, wherein the Klotho polypeptide has at least 90 % sequence identity to SEQ ID NO: 55, preferably at least 95 %, more preferred at least 96 %, more preferred at least 97 %, more preferred at least 98 %, more preferred at least 99 %, more preferred at least 99.5 %, more preferred 100 %.
Embodiment 54. The Klotho mRNA according to any one of the preceding Embodiments, wherein the Klotho polypeptide is SEQ ID NO: 55 or 56, preferably 55.
Embodiment 55. The Klotho mRNA according to any one of the preceding Embodiments, wherein the Klotho polypeptide has at least 90 % sequence identity to SEQ ID NO: 57, preferably at least 95 %, more preferred at least 96 %, more preferred at least 97 %, more preferred at least 98 %, more preferred at least 99 %, more preferred at least 99.5 %, more preferred 100 %.
Embodiment 56. The Klotho mRNA according to any one of the preceding Embodiments, wherein the Klotho polypeptide is SEQ ID NO: 57 or 58, preferably 57.
Embodiment 57. The Klotho mRNA according to any one of the preceding Embodiments, wherein the Klotho polypeptide has at least 90 % sequence identity to SEQ ID NO: 49, preferably at least 95 %, more preferred at least 96 %, more preferred at least 97 %, more preferred at least 98 %, more preferred at least 99 %, more preferred at least 99.5 %, more preferred 100 %.
Embodiment 58. The Klotho mRNA according to any one of the preceding Embodiments, wherein the Klotho polypeptide is SEQ ID NO: 49 or 76, preferably 49.
Embodiment 59. The Klotho mRNA according to any one of the preceding Embodiments, wherein the Klotho polypeptide has at least 90 % sequence identity to SEQ ID NO: 59, preferably at least 95 %, more preferred at least 96 %, more preferred at least 97 %, more preferred at least 98 %, more preferred at least 99 %, more preferred at least 99.5 %, more preferred 100 %.
Embodiment 60. The Klotho mRNA according to any one of the preceding Embodiments, wherein the Klotho polypeptide is SEQ ID NO: 59 or 60, preferably 59.
Embodiment 61. The Klotho mRNA according to any one of the preceding Embodiments, wherein the poly-A tail comprises at least 60 adenosine monophosphates, preferably at least 100 adenosine monophosphates, especially at least 120 adenosine monophosphates.
Embodiment 62. The Klotho mRNA according to any one of the preceding Embodiments, wherein the 5'-UTR or 3'-UTR or the 5'-UTR and the 3'-UTR correspond to native human Klotho mRNA.
Embodiment 63. The Klotho mRNA according to any one of the preceding Embodiments, wherein the 5'-UTR or 3'-UTR or the 5'-UTR and the 3'-UTR are different from native human Klotho mRNA.
Embodiment 64. The Klotho mRNA according to any one of the preceding Embodiments, wherein the 5'-UTR or 3'-UTR or the 5'-UTR and the 3'-UTR contain at least one stabilisation sequence, preferably a stabilisation sequence with the general formula (C/U)CCANₓCCC(U/A)PyₓUC(C/U)CC (SEQ ID NO: 63), wherein "x" is, independently in Nₓ and Pyₓ, an integer of 0 to 10, preferably of 0 to 5, especially 0, 1, 2, 4 and/or 5).
Embodiment 65. The Klotho mRNA according to any one of the preceding Embodiments, wherein the 5'-UTR or 3'-UTR or the 5'-UTR and the 3'-UTR contain at least one destabilisation sequence element (DSE), preferably AU-rich elements (AREs) and/or U-rich elements (UREs), especially a single, tandem or multiple or overlapping copies of the nonamer UUAUUUA(U/A)(U/A).
Embodiment 66. The Klotho mRNA according to any one of the preceding Embodiments, wherein the 5'-UTR or 3'-UTR or the 5'-UTR and the 3'-UTR are the 5'-UTR and/or 3'-UTR of a different human mRNA than native human Klotho mRNA, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopox-virus, and mixtures thereof, especially selected from alpha Globin, beta Globin, alpha(1) Collagen, and mixtures thereof.
Embodiment 67. The Klotho mRNA according to any one of the preceding Embodiments, wherein in the Klotho mRNA, at least 5%, preferably at least 10%, preferably at least 30%, especially at least 50% of all
   - cytidine residues are replaced by 5-methyl-cytidine residues, and/or
   - cytidine residues are replaced by 2-amino-2-deoxy-cytidine residues, and/or
   - cytidine residues are replaced by 2-fluoro-2-deoxy-cytidine residues, and/or
   - cytidine residues are replaced by 2-thio-cytidine residues, and/or
   - cytidine residues are replaced by 5-iodo-cytidine residues, and/or
   - uridine residues are replaced by pseudo-uridine residues, and/or
   - uridine residues are replaced by 1-methyl-pseudo-uridine residues, and/or
   - uridine residues are replaced by 2-thio-uridine residues, and/or
   - uridine residues are replaced by 5-methyl-uridine residues, and/or
   - adenosine residues are replaced by N6-methyl-adenosine residues.
Embodiment 68. The Klotho mRNA according to any one of the preceding Embodiments, wherein in the Klotho mRNA, at least 5%, preferably at least 10%, preferably at least 30%, especially at least 50% of all
   - cytidine residues are replaced by 5-methyl-cytidine residues, and/or
   - uridine residues are replaced by pseudo-uridine residues, and/or
   - uridine residues are replaced by 2-thio-uridine residues.
Embodiment 69. The Klotho mRNA according to any one of the preceding Embodiments, wherein the coding region has a codon adaption index (CAI) of at least 0.70, preferably at least 0.75, more preferred at least 0.80, more preferred at least 0.85, more preferred at least 0.86, more preferred at least 0.87, more preferred at least 0.88, more preferred at least 0.89, more preferred at least 0.90, more preferred at least 0.91, more preferred at least 0.92, more preferred at least 0.93, more preferred at least 0.94, more preferred at least 0.95, more preferred at least 0.96, more preferred at least 0.97, more preferred at least 0.98, more preferred at least 0.99, more preferred 1.00.
Embodiment 70. The Klotho mRNA according to any one of the preceding Embodiments, wherein the coding region has a codon adaption index (CAI) of below 1.00, preferably below 0.99, more preferred below 0.98, more preferred below 0.97, more preferred below 0.96, more preferred below 0.95.
Embodiment 71. The Klotho mRNA according to any one of the preceding Embodiments, wherein the 5'-UTR has at least 70 % sequence identity to SEQ ID NO: 61, preferably at least 75 %, more preferred at least 80 %, more preferred at least 85 %, more preferred at least 90 %, more preferred at least 95 %, more preferred at least 96 %, more preferred at least 97 %, more preferred at least 98 %, more preferred at least 99 %, more preferred at least 99.5 %, more preferred 100 %.
Embodiment 72. The Klotho mRNA according to any one of the preceding Embodiments, wherein the 5'-UTR is SEQ ID NO: 61.
Embodiment 73. The Klotho mRNA according to any one of the preceding Embodiments, wherein the 3'-UTR has at least 70 % sequence identity to SEQ ID NO: 62, preferably at least 75 %, more preferred at least 80 %, more preferred at least 85 %, more preferred at least 90 %, more preferred at least 95 %, more preferred at least 96 %, more preferred at least 97 %, more preferred at least 98 %, more preferred at least 99 %, more preferred at least 99.5 %, more preferred 100 %.
Embodiment 74. The Klotho mRNA according to any one of the preceding Embodiments, wherein the 3'-UTR is SEQ ID NO: 62.
Embodiment 75. The Klotho mRNA according to any one of the previous Embodiments, wherein the Klotho mRNA has at least 80 % sequence identity to SEQ ID NO: 29, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %.
Embodiment 76. The Klotho mRNA according to any one of the preceding Embodiments, wherein the Klotho mRNA is SEQ ID NO: 29, 30, 31, or 32, preferably SEQ ID NO: 29.
Embodiment 77. The Klotho mRNA according to any one of the previous Embodiments, wherein the Klotho mRNA has at least 80 % sequence identity to SEQ ID NO: 72, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %.
Embodiment 78. The Klotho mRNA according to any one of the preceding Embodiments, wherein the Klotho mRNA is SEQ ID NO: 72, 73, 74, or 75, preferably SEQ ID NO: 72.
Embodiment 79. The Klotho mRNA according to any one of the previous Embodiments, wherein the Klotho mRNA has at least 80 % sequence identity to SEQ ID NO: 45, preferably at least 81 %, more preferred at least 82 %, more preferred at least 83 %, more preferred at least 84 %, more preferred at least 85 %, more preferred at least 86 %, more preferred at least 87 %, more preferred at least 88 %, more preferred at least 89 %, more preferred at least 90 %, more preferred at least 91 %, more preferred at least 92 %, more preferred at least 93 %, more preferred at least 94 %, more preferred at least 95 %, more preferred at least 96 %, more preferred at least 97 %, more preferred at least 98 %, more preferred at least 99 %, more preferred at least 99.5 %, more preferred at least 99.8 %, more preferred 100 %.
Embodiment 80. The Klotho mRNA according to any one of the preceding Embodiments, wherein the Klotho mRNA is SEQ ID NO: 45, 46, 47, or 48, preferably SEQ ID NO: 45.
Embodiment 81. Pharmaceutical formulation comprising the Klotho mRNA according to any one of the preceding Embodiments.
Embodiment 82. The pharmaceutical formulation according to any one of the preceding Embodiments, comprising a pharmaceutically acceptable carrier, preferably polymer based carriers, especially cationic polymers, including linear and branched PEI and viromers; lipid nanoparticles and liposomes, nanoliposomes, ceramide-containing nanoliposomes, proteoliposomes, cationic amphiphilic lipids e.g.: SAINT-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, nio-somes, plasmids, viruses, calcium phosphate nucleotides, ap-tamers, peptides, peptide conjugates, vectorial tags, poly-lactic-co-glycolic acid (PLGA) polymers; preferably small-molecule targeted conjugates, or viral capsid proteins, preferably cationic polymers and liposomes, especially cationic polymers.
Embodiment 83. The pharmaceutical formulation according to any one of the preceding Embodiments, comprising cationic polymers including linear and branched PEI and viromers, lipid nanoparticles and liposomes, transfersomes, and nanoparticulates, preferably calcium phosphate nanoparticulates and cationic polymers, especially cationic polymers.
Embodiment 84. The pharmaceutical formulation according to any one of the preceding Embodiments, wherein the mRNA is non-complexed mRNA, and wherein preferably the non-complexed mRNA is contained in a suitable aqueous buffer solution, especially a physiological glucose buffered aqueous solution.
Embodiment 85. The pharmaceutical formulation according to any one of the preceding Embodiments, wherein the formulation comprises a 1xHEPES buffered solution; a 1xPhosphate buffered solution, a Na-Citrate buffered solution; a Na-Acetate buffered solution; Ringer's lactate solution; preferably additionally comprising glucose, especially 5% Glucose.
Embodiment 86. The pharmaceutical formulation according to any one of the preceding Embodiments, wherein the formulation comprises a further mRNA, wherein the further mRNA has a further 5' CAP region, a further 5'-UTR, a further coding region encoding a further polypeptide, a further 3'-UTR and a further poly-A tail.
Embodiment 87. The pharmaceutical formulation according to any one of the preceding Embodiments, wherein the further polypeptide is Bactericidal/Permeability Increasing Protein Family B, member 4 (BPIFB4) protein or an isoform or variant thereof, preferably Longevity-Associated Variant of BPIFB4 (LAV-BPIFB4).
Embodiment 88. The pharmaceutical formulation according to any one of the preceding Embodiments, wherein the further polypeptide is a BPIFB4 protein or an isoform or variant thereof as set forth in WO 2014/102343 A1 or WO 2019/034723 A1.
Embodiment 89. The pharmaceutical formulation according to any one of the preceding Embodiments, wherein the further polypeptide is a protein selected from Apolipoprotein E, Tumor protein p53, Sirtuin 1 protein, FOXO1 transcription factor, Cholinergic receptor nicotinic alpha 3 subunit, SH2B adaptor protein 3, Cyclin dependent kinase inhibitor 2A, Elongation of very-long-chain fatty acids-like 2, Werner protein, Paraoxonase 1, Superoxide dismutase 2, Lamin A protein, Cholesteryl ester transfer protein, Apolipoprotein C3, Microsomal triglyceride transfer protein, Phosphatidylinositol 3-kinase (PI3K), Insulin-like growth factor 1 (IGF-1) receptor, Protein-L-isoaspartyl methyltransferase, Growth hormone, cAMP-response element binding protein, Mitogen-activated protein kinase, epidermal growth factor receptor, Nuclear factor kappa B, Phospholipase C beta, Methionine sulfoxide reductase A, Mediator of cell motility 1, Nei like DNA Glycosylase 1, Peroxisome proliferator-activated receptor gamma 2, Eukaryotic translation initiation factor 3 subunit K, ATM serine/threonine kinase, B-cell lymphoma 2, Cell division cycle 42, Diacylglycerol O -acyltransferase 1, Early growth response 1, Fibroblast growth factor 23, Fibroblast growth factor 21, Fructosamine 3 kinase related protein, Phosphoglycolate phosphatase, Insulin receptor substrate 1, Polycomb complex protein BMI-1, Neuregulin 1, Signal transducer and activator of transcription, E2F Transcription Factor 1, Vascular endothelial growth factor A, Xenobiotic metabolizing enzymes, Myc proto-oncogene protein, C-X-C chemokine receptor type 4, Silent information regulator 2, Extracellular signal-regulated kinase, and SLC31.
Embodiment 90. Kit for administering the Klotho mRNA according to any one of the preceding Embodiments to an individual comprising
   - the Klotho mRNA according to any one of the preceding Embodiments, and
   - a device for administering the Klotho mRNA.
Embodiment 91. The kit according to any one of the preceding Embodiments, wherein the individual is a mammal, preferably a human.
Embodiment 92. The kit according to any one of the preceding Embodiments, wherein the device is for intravenous, intramuscular, subcutaneous, intradermal, transdermal, epidermal, or topical administration.
Embodiment 93. The kit according to any one of the preceding Embodiments, wherein the device for administering the Klotho mRNA is a syringe, a needle, an autoinjector, and/or a needle-free injection system; preferably wherein the device is for intravenous and/or intramuscular injection.
Embodiment 94. The kit according to any one of the preceding Embodiments, wherein the device for administering the Klotho mRNA is a skin delivery device.
Embodiment 95. The kit according to any one of the preceding Embodiments, wherein the skin delivery device is an intradermal delivery device, preferably selected from the group consisting of needle based injection systems.
Embodiment 96. The kit according to any one of the preceding Embodiments, wherein the skin delivery device is a transdermal delivery device, preferably selected from the group consisting of transdermal patches, hollow and solid microneedle systems, micro-structured transdermal systems, electrophoresis systems, and ion-tophoresis systems.
Embodiment 97. The kit according to any one of the preceding Embodiments, wherein the skin delivery device is an epidermal delivery device, preferably selected from the group consisting of needle free injection systems, laser based systems, especially Erbium YAG laser systems, and gene gun systems.
Embodiment 98. The Klotho mRNA or the pharmaceutical composition according to any one of the preceding Embodiments for use as a medicament.
Embodiment 99. The Klotho mRNA or the pharmaceutical composition according to any one of the preceding Embodiments for use in the prevention or treatment of a disease selected from the group consisting of kidney diseases, cardiovascular diseases, brain diseases, lung diseases, bone diseases, and metabolic diseases.
Embodiment 100. The Klotho mRNA or the pharmaceutical composition according to any one of the preceding Embodiments for use in the prevention or treatment of a kidney disease, preferably in the prevention or treatment of a disease selected from the group consisting of chronic kidney disease, fibrosis, hyperphosphatemia, ischemic injury, nephrectomy, toxic injury, diabetic nephropathy, and calciprotein deposition.
Embodiment 101. The Klotho mRNA or the pharmaceutical composition according to any one of the preceding Embodiments for use in the prevention or treatment of a cardiovascular disease, preferably in the prevention or treatment of a disease selected from the group consisting of arterial/aortic calcification, atherosclerosis, cardiomyopathy, cardiac hypertrophy, hypertension, and myocardial ischemic injury/infarct.
Embodiment 102. The Klotho mRNA or the pharmaceutical composition according to any one of the preceding Embodiments for use in the prevention or treatment of a brain disease, preferably in the prevention or treatment of a disease selected from the group consisting of Alzheimer's disease, hippocampal neuronal loss, cognitive deficits, and frailty.
Embodiment 103. The Klotho mRNA or the pharmaceutical composition according to any one of the preceding Embodiments for use in the prevention or treatment of cancer, preferably in the prevention or treatment of a cancer selected from the group consisting of colorectal cancer, esophageal cancer, gastric cancer, pancreatic cancer, breast cancer, lung cancer, ovarian cancer, thyroid cancer, melanoma, renal cancer, and cervical cancer.
Embodiment 104. The Klotho mRNA or the pharmaceutical composition according to any one of the preceding Embodiments for use in the prevention or treatment of a lung disease, preferably in the prevention or treatment of pulmonary fibrosis or chronic obstructive pulmonary disease.
Embodiment 105. The Klotho mRNA or the pharmaceutical composition according to any one of the preceding Embodiments for use in the prevention or treatment of a bone disease, preferably in the prevention or treatment of osteoporosis or osteomalacia, especially osteomalacia from chronic kidney disease.
Embodiment 106. The Klotho mRNA or the pharmaceutical composition according to any one of the preceding Embodiments for use in the prevention or treatment of a metabolic disease, preferably in the prevention or treatment of a disease selected from the group consisting of diabetes, in particular type 1 and/or type 2 diabetes, pancreatic β-cell apoptosis, autoimmune disorders, and inflammation, especially insulitis.
Embodiment 107. The Klotho mRNA for use or the pharmaceutical composition for use according to any one of the preceding Embodiments, wherein the Klotho mRNA is administered intravenously, intramuscularly, subcutaneously, intradermally, transdermally, epidermally, or topically, especially epidermally.
Embodiment 108. The Klotho mRNA for use or the pharmaceutical composition for use according to any one of the preceding Embodiments, wherein the Klotho mRNA is administered at least twice within one month, preferably weekly.
Embodiment 109. The Klotho mRNA for use or the pharmaceutical composition for use according to any one of the preceding Embodiments, wherein the Klotho mRNA is administered in an amount of 0.01µg to 100 mg per dose, preferably of 0.1µg to 10mg per dose, especially of 1µg to 1mg per dose.
Embodiment 110. The Klotho mRNA or the pharmaceutical composition according to any one of the preceding Embodiments for use in the prevention or treatment of an aging-related disorder and/or a symptom of aging.
Embodiment 111. The Klotho mRNA for use or the pharmaceutical composition for use according to any one of the preceding Embodiments, wherein the aging-related disorder or symptom of aging is selected from one or more of actinic keratosis, age-related macular degeneration (AMD), Alzheimer's disease, arthritis, atherosclerosis and cardiovascular disease, benign prostatic hyperplasia (BPH), bone atrophy, cachexia, cancer, cardiomyopathy, cataracts, chronic obstructive pulmonary disease (CORD), constipation, decrease in overall energy, decrease in visual acuity, delirium, dementia, depression, dermal atrophy (thinning of the skin), diminished peripheral vision, greater risk of heat stroke or hypothermia, hearing loss, hypertension, increased susceptibility to infection (including influenza and pneumonia), lentigines (aging spots), liver conditions (e.g ., nonalcoholic fatty liver disease, nonalcoholic steatohepatitis, and cirrhosis), memory loss, metabolic syndrome, muscle atrophy (e.g., Sarcopenia and myopenia), frailty, muscle repair or rejuvenation deficiency, muscular dystrophy, osteoarthritis, osteoporosis, periodontitis, photoaging, reduced metabolism (including increased risk for obesity), reduced reflexes and coordination including difficulty with balance, respiratory disease (including acute respiratory distress syndrome (ARDS) with or without acute lung injury (ALI) and pulmonary fibrosis (e.g, idiopathic pulmonary fibrosis)), rheumatoid arthritis, sarcopenic obesity, sexual dysfunction, shingles, type 2 diabetes, urologic changes (including incontinence), vaginal atrophy, whitening or graying of hair, prolonged/inefficient wound healing, wrinkling/sagging skin (including loss of skin elasticity), and xerosis cutis (skin dryness); or the disease includes asthma, deafness, or a viral infections and/or a symptom of the disease comprises sepsis.
Embodiment 112. Use of the Klotho mRNA or the pharmaceutical composition according to any one of the preceding Embodiments for preventing and/or reducing a symptom of aging.
Embodiment 113. Use of the Klotho mRNA or the pharmaceutical composition according to any one of the preceding Embodiments for increasing the lifespan in a mammal, preferably a human.
Embodiment 114. Cosmetic formulation comprising the Klotho mRNA according to any one of the preceding Embodiments.
Embodiment 115. The cosmetic formulation according to any one of the preceding Embodiments, comprising a cosmetic carrier.
Embodiment 116. The cosmetic formulation according to any one of the preceding Embodiments, wherein the cosmetic carrier is an ointment, a gel, especially a hydrogel, a liposome, nano/microparticles, or an emulsion.
Embodiment 117. Cosmetic skin care method, comprising contacting a skin of an individual with the Klotho mRNA according to any one of the preceding Embodiments.
Embodiment 118. The cosmetic skin care method according to any one of the preceding Embodiments, wherein the individual is a human individual.
Embodiment 119. The cosmetic skin care method according to any one of the preceding Embodiments, wherein the skin is an ageing skin.

The present invention is further illustrated by the following figures and examples, without being limited thereto.
**Figure 1****.** Quantification of protein levels obtained after transfection of mammalian cell lines with different Klotho mRNA variants. Three different cell lines were used: (A) Hs27; (B) HEK293; (C) 3T3.
**Figure 2****.** Results of a biologically independent repetition of the experiments shown in Figure 1. (A) Hs27; (B) HEK293; (C) 3T3.

### Example 1. Klotho expression levels obtained from different mRNA sequence variants.

The Klotho protein expression levels obtained with different sequence variants were tested in order to quantify the effect of the different mRNA sequences on the expression levels in different cell lines.

### Sequence variants

The mRNA sequence of native human Klotho isoform 1 according to NCBI Reference Sequence: NM_004795.4 was used as a starting point. From this, the sequence coding for soluble Klotho, i.e., amino acids M1 to S981 was extracted (SEQ ID NO: 17). This sequence is 2946 nt long and can be divided into the signal peptide (amino acids 1-33; nt 1-99) and KL1 and KL2 domains including adjacent linking regions (amino acids E34 to S981; nt 100-2946). The part coding for the signal peptide was kept constant for the following comparative tests. The latter part of the sequence corresponding to nt 100-2946 was varied. As explained in the description herein above, four different publicly available codon optimization algorithms were used. In addition, custom variants with increasing GC-contents were created. To all these variants, the sequence for the signal peptide was then added. This resulted in the following variants:
- Variant "GC62" (SEQ ID NO: 18); GC content: 61.6 %
- Variant "GC60" (SEQ ID NO: 19); GC content: 60.1 %
- Variant "GC57" (SEQ ID NO: 22); GC content: 57.4 %
- Variant "GC55" (SEQ ID NO: 23); GC content: 54.9 %
- Variant "GC53" (SEQ ID NO: 24); GC content: 52.8 %
- "GENEius algorithm" (SEQ ID NO: 25); GC content: 52.6 %
- "ExpOptimizer" (SEQ ID NO: 27); GC content: 51.1 %
- "IDT Codon Optimization Tool" (SEQ ID NO: 26); GC content: 49.9 %
- "Twist Codon Optimization Tool" (SEQ ID NO: 28); GC content: 48.7 %

In order to finalize the constructs for synthesis, the sequences for the 5'-UTR (SEQ ID NO: 61) and 3'-UTR (SEQ ID NO: 62) and a T7 promotor sequence were added. The full mRNA sequences of the "GC62" and "GC60" variants are given in SEQ ID NOs: 29 and 30, respectively.

### Cloning and in vitro transcription (IVT)

Standard gene synthesis services and cloning techniques were used to obtain vectors containing the coding sequences of the different Klotho variants described above. The poly A tail needed for the in vitro transcription was added by polymerase chain reaction (PCR). The vectors containing the different coding sequences were used as templates. For the amplification the Phusion^{™} Plus DNA Polymerase Kit (Thermo Fisher) was used in accordance with the manufacturer's instructions.

For the PCR, 10ng plasmid DNA, 0.7µM of the forward primer, 5'-TGCTGCCGTAATACGACTCACTATAAGG-3' (SEQ ID NO: 77) and 0.7µM of the reverse primer, 5'-T120-TGCCGCCCACTCAGACTTTATTC-3' (SEQ ID NO: 78) (IDT technologies), were used. PCR was performed using the following cycling protocol: initial activation step at 98°C for 30s, followed by 35 cycles of denaturation at 98°C for 5s, annealing at 60°C for 10s, extension at 72°C for 1:45min, and final extension at 72°C for 5min. After the DNA amplification, PCR products were purified using QIAquick PCR purification kit (Qiagen) and eluted in 2×20 µl nuclease-free water (Qiagen). The quality and purity of the DNA was assessed by 1% agarose gel electrophoresis.

The IVT of the DNA into mRNA was performed using MEGAscript^{®} T7 Kit (Life Technologies, Darmstadt, Germany) according to the manufacturer's instructions. In brief, 20µl IVT reaction mixture containing 7.5mM ATP, GTP, CTP and UDP and 6mM CleanCAP (TriLink), 40U RiboLock RNase inhibitor (Thermo Fisher Scientific, Waltham, USA), 100ng PCR product, 1x reaction buffer and 1x T7 RNA polymerase enzyme mix was prepared. The mixture was incubated for 4h at 37°C, then 1µL TURBO DNase (from MEGAscript^{®} T7 Kit) was added to the IVT reaction mixture and incubated for 15min at 37°C to remove the template DNA. After the incubation, mRNA was purified using RNeasy Mini Kit (Qiagen) according to the manufacturer's instructions and eluted in 2 × 35µL nuclease-free water. Subsequently, dephosphorylation was performed with 10U Antarctic phosphatase (New England Biolabs) at 37 °C for 30min. The mRNA was purified and eluted in 2 × 35µL nuclease-free water using RNeasy Mini Kit. The quality and purity of the synthesized and modified mRNA were confirmed in a 1% agarose gel. The synthesized mRNA was stored at -80°C and used for transfections.

### In vitro transfection

In preparation for mRNA transfections cells were plated at ~80% density (human: HEK, Hs27; mouse: 3T3). After 4 hours the mRNA was complexed with JetMessenger (Polyplus) at a ratio of 1:2 (pg mRNA : µL JetMessenger) according to the manufacturers manual. After 15min of incubation the mRNA complex was added dropwise to the cells.

0.75pg mRNA was added to the cells contained in a 24 well plate. The cell numbers per well were as follows: 70,000 (for Hs27), 200,000 (for HEK), 100,000 (for 3T3).

Depending on the type of the experiment, the cell pellets and/or the supernatant were harvested and frozen at -80°C until further analysis.

### ELISA

Klotho levels in cell culture supernatants were measured by ELISA (R&D systems, DY5334-05) according to the manufacturer's instructions.

### Cell lines

All cell lines were cultured at 37 °C and 5% CO2 atmosphere. Medium and supplements were used as recommend by the ATCC.

NIH/3T3 fibroblasts (ATCC, Manassas, Virginia, USA) were cultivated in DMEM with high glucose containing 10% fetal calf serum (FCS), and 1% penicillin/streptomycin. Cell culture medium and supplements were obtained from Thermo Fisher Scientific (Waltham, USA). Cells were kept at 37°C with 5% CO2 and medium was changed every 3 days. Cells were passaged using TrypLE^{™} Express Enzyme (Thermo Fisher Scientific, 1260401).

Hs27 human fibroblasts (ATCC, Manassas, Virginia, USA) were cultivated in DMEM with high glucose containing 20% fetal calf serum (FCS), and 1% penicillin/streptomycin. Cell culture medium and supplements were obtained from Thermo Fisher Scientific (Waltham, USA). Cells were kept at 37°C with 5% CO2 and medium was changed every 3 days. Cells were passaged using TrypLE^{™} Express Enzyme (Thermo Fisher Scientific, 1260401).

HEK293 cells (ATCC, Manassas, Virginia, USA) were cultivated in DMEM with high glucose containing 10% fetal calf serum (FCS), and 1% penicillin/streptomycin. Cell culture medium and supplements were obtained from Thermo Fisher Scientific (Waltham, USA). Cells were kept at 37°C with 5% CO2 and medium was changed every 3 days. Cells were passaged using TrypLE^{™} Express Enzyme (Thermo Fisher Scientific, 1260401).

### Results

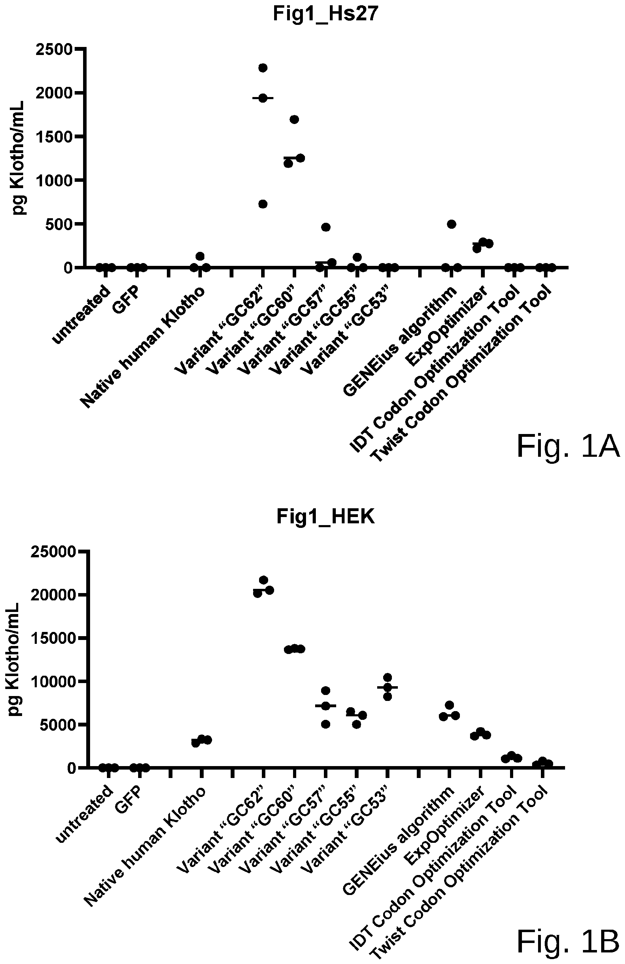

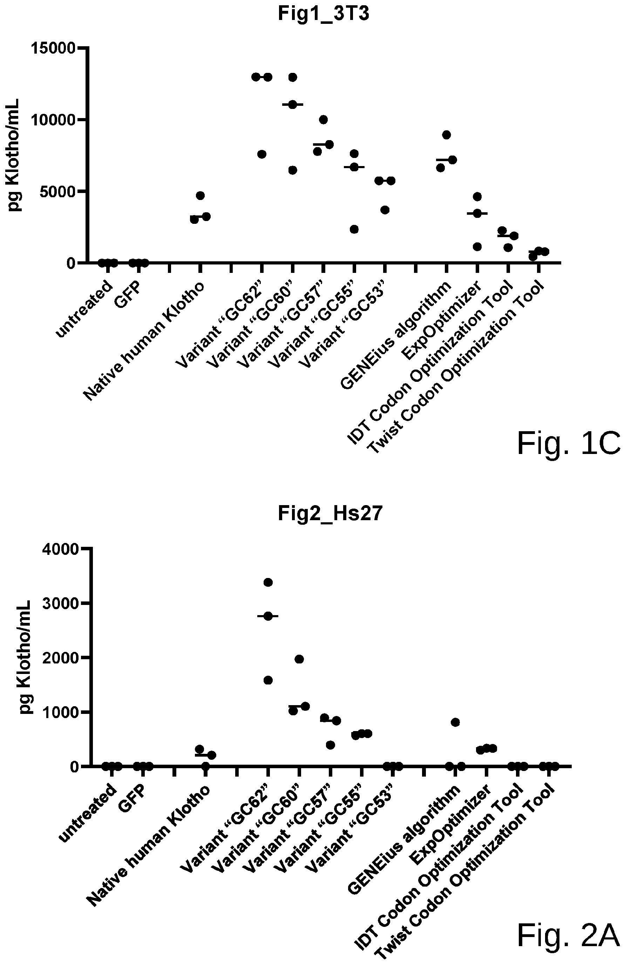

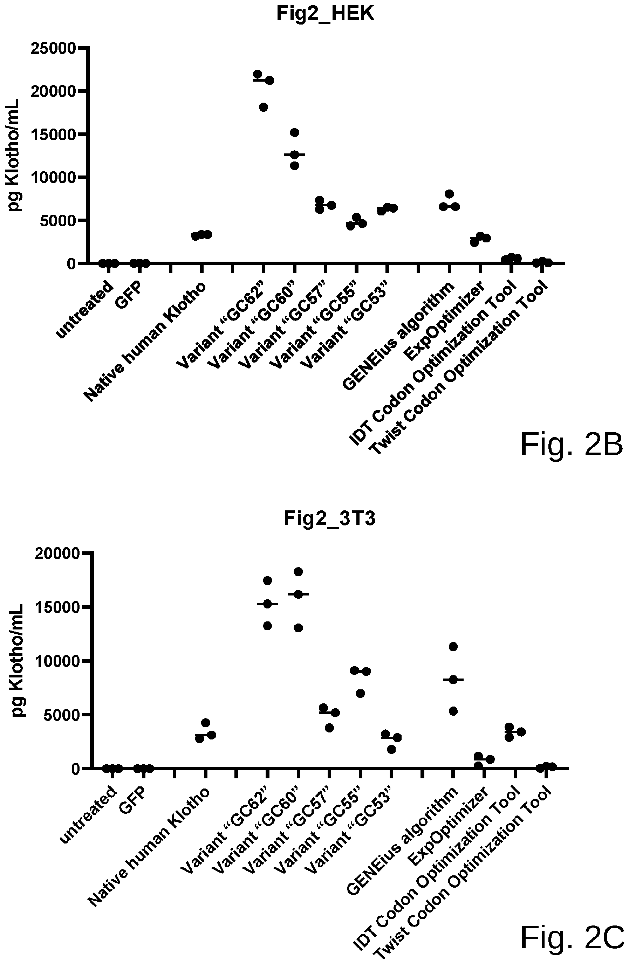

Figure 1 and Figure 2 show two biologically independent in vitro experiments in 3 different cell lines (human: Hs27 and HEK, murine: 3T3). The cell culture supernatant was harvested 24h after the mRNA transfection and analyzed by ELISA.

Over all the experiments and cell lines, the "GC62" variant outperformed all other variants as well as the native Klotho sequence. The increases in expression levels compared to the native sequence were between 3-fold and 6-fold in HEK and 3T3 cells and more than 10-fold in Hs27 cells. In addition, surprisingly, a direct relationship between the GC content and the expression level was found.

### Sequences

The following table provides an overview of the RNA sequences disclosed herein.

**Table 3: Overview of disclosed RNA sequences.**

| **Klotho Isoform** | **Domains** | **Amino acids** | **SEQ ID NO:** | **GC content (%)** | **Length** |
|---|---|---|---|---|---|
| Isoforms 1/2 | KL1 | L56 to F506 | 1 | 61,9 | 1353 |
| | | | 2 | 59, 9 | 1353 |
| | | | 3 | 61,9 | 1353 |
| | | | 4 | 60,1 | 1353 |
| Isoforms 1/2 | SP-lr-KL1 | M1 to F506 | 5 | 63,1 | 1521 |
| | | | 6 | 60,8 | 1521 |
| | | | 7 | 63,1 | 1521 |
| | | | 8 | 60,9 | 1521 |
| Isoform 1 | KL1-lr-KL2 | L56 to G952 | 9 | 60,9 | 2691 |
| | | | 10 | 59,5 | 2691 |
| | | | 11 | 60,9 | 2691 |
| | | | 12 | 59, 6 | 2691 |
| Isoform 1 | lr-KL1-lr-KL2-lr | E34 to S981 | 13 | 61,3 | 2844 |
| | | | 14 | 59,7 | 2844 |
| | | | 15 | 61,3 | 2844 |
| | | | 16 | 59, 8 | 2844 |
| Isoform 1 | SP-lr-KL1-lr-KL2-lr | M1 to S981 | 17 | 54,5 | 2946 |
| | | | 18 | 61,6 | 2946 |
| | | | 19 | 60,1 | 2946 |
| | | | 20 | 61,6 | 2946 |
| | | | 21 | 60,1 | 2946 |
| | | | 22 | 57,4 | 2946 |
| | | | 23 | 54,9 | 2946 |
| | | | 24 | 52,8 | 2946 |
| | | | 25 | 52,6 | 2946 |
| | | | 26 | 49,9 | 2946 |
| | | | 27 | 51,1 | 2946 |
| | | | 28 | 48,1 | 2946 |
| Isoform 1 | full_mRNA | M1 to S981 | 29 | 59,5 | 3249 |
| | | | 30 | 58,2 | 3249 |
| | | | 31 | 59,6 | 3249 |
| | | | 32 | 58,2 | 3249 |
| Isoform 2 | KL1-end | L56 to H549 | 33 | 61,7 | 1482 |
| | | | 34 | 59,5 | 1482 |
| | | | 35 | 61,7 | 1482 |
| | | | 36 | 59,6 | 1482 |
| Isoform 2 | lr-KL1-end | E34 to H549 | 37 | 62,3 | 1548 |
| | | | 38 | 59,8 | 1548 |
| | | | 39 | 62,4 | 1548 |
| | | | 40 | 59,9 | 1548 |
| Isoform 2 | SP-lr-KL1-end | M1 to H549 | 41 | 62,8 | 1650 |
| | | | 42 | 60,4 | 1650 |
| | | | 43 | 62,8 | 1650 |
| | | | 44 | 60,5 | 1650 |
| Isoform 2 | full_mRNA | M1 to H549 | 45 | 59,2 | 1953 |
| | | | 46 | 57,1 | 1953 |
| | | | 47 | 59,2 | 1953 |
| | | | 48 | 57,2 | 1953 |
| Isoform 1 | lr-KL1-lr-KL2-lr-TM-CT | E34 to K1012 | 64 | 60,5 | 2937 |
| | | | 65 | 59,0 | 2937 |
| | | | 66 | 60,6 | 2937 |
| | | | 67 | 59,1 | 2937 |
| Isoform 1 | SP-lr-KL1-lr-KL2-lr-TM-CT | M1 to K1012 | 68 | 60,8 | 3039 |
| | | | 69 | 59,4 | 3039 |
| | | | 70 | 60,9 | 3039 |
| | | | 71 | 59,5 | 3039 |
| Isoform 1 | full_mRNA | M1 to K1012 | 72 | 58,9 | 3342 |
| | | | 73 | 57,6 | 3342 |
| | | | 74 | 58,9 | 3342 |
| | | | 75 | 57,7 | 3342 |

The following table provides an overview of the protein sequences disclosed herein.

**Table 4: Overview of disclosed protein sequences.**

| **Klotho Isoform** | **Domains** | **Amino acids** | **SEQ ID NO:** | **Length** |
|---|---|---|---|---|
| Isoform 1 | native sequence | M1 to K1012 | 49 | 1012 |
| Isoform 2 | native sequence | M1 to H549 | 50 | 549 |
| Isoforms 1/2 | SP | M1 to A33 | 51 | 33 |
| Isoforms 1/2 | KL1 | L56 to F506 | 52 | 451 |
| | | | 53 | 451 |
| Isoforms 1 | KL2 | L515 to G952 | 54 | 897 |
| Isoforms 1/2 | SP-lr-KL1 | M1 to F506 | 55 | 506 |
| | | | 56 | 506 |
| Isoform 1 | SP-lr-KL1-lr-KL2-lr | M1 to S981 | 57 | 981 |
| | | | 58 | 981 |
| Isoform 2 | SP-lr-KL1-end | M1 to H549 | 59 | 549 |
| | | | 60 | 549 |
| Isoform 1 | native sequence | M1 to K1012 | 76 | 1012 |

SEQ ID NO: 1: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUC
SEQ ID NO: 2: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
 AAGAUGUUGCUGCCAAAGAGUAGCGCCCUUUUCUACCAGAAGUUGAUUGAGAAGAACGGCUUC
SEQ ID NO: 3: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUC
SEQ ID NO: 4: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUC
SEQ ID NO: 5: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCUGA
SEQ ID NO: 6: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
 
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCUGA
SEQ ID NO: 7: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCUGA
SEQ ID NO: 8: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCUGA
SEQ ID NO: 9: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGGACACCACCCUGAGUCAGUUCACUG
ACCUGAACGUGUACCUUUGGGACGUGCACCACAGCAAGCGCCUGAUAAAGGUCGACGGCGUGGUGACCAAGAAGCGCAAAAGCUAC
UGCGUGGACUUUGCCGCCAUCCAGCCCCAGAUCGCCCUGCUGCAGGAGAUGCACGUGACUCACUUCCGCUUCAGCCUCGACUGGGC
CCUGAUUCUGCCCCUGGGCAACCAGUCACAAGUGAACCACACCAUUCUGCAGUACUACCGCUGUAUGGCCAGCGAGCUGGUACGCG
UGAACAUCACCCCCGUUGUGGCCCUUUGGCAGCCCAUGGCCCCCAACCAGGGCCUGCCUCGCCUGCUGGCCCGCCAGGGAGCCUGG
GAGAAUCCCUACACCGCACUGGCCUUCGCUGAAUACGCCCGCCUGUGCUUCCAGGAGCUGGGGCACCACGUGAAACUCUGGAUCAC
CAUGAACGAGCCCUACACCAGAAACAUGACCUACAGCGCCGGACACAAUCUGCUGAAGGCCCACGCUCUGGCCUGGCACGUGUACA
ACGAGAAGUUUCGCCACGCCCAGAACGGCAAGAUCAGCAUCGCCCUGCAGGCCGAUUGGAUCGAGCCCGCCUGCCCCUUCAGCCAG
AAGGACAAGGAGGUGGCUGAGCGGGUGCUGGAGUUCGACAUCGGGUGGCUGGCUGAGCCUAUCUUCGGCAGCGGAGACUACCCCUG
GGUGAUGCGCGAUUGGCUUAACCAGAGGAACAAUUUCCUGCUGCCCUACUUCACCGAGGACGAGAAGAAGCUGAUCCAGGGCACCU
 
UUGACUUCCUGGCUUUGAGCCACUAUACCACUAUCCUGGUGGACAGCGAGAAGGAGGACCCCAUCAAGUACAACGACUACUUGGAG
GUGCAGGAGAUGACCGACAUCACCUGGCUGAACAGCCCUAGCCAGGUGGCUGUGGUGCCUUGGGGCCUGCGCAAGGUGCUGAACUG
GCUGAAGUUCAAGUACGGCGACCUGCCCAUGUAUAUCAUCAGCAACGGCAUCGACGACGGCCUGCACGCCGAGGACGACCAGCUGA
GAGUGUACUACAUGCAGAACUACAUCAACGAGGCCCUCAAGGCCCACAUCCUGGACGGCAUCAACCUGUGUGGCUACUUUGCCUAC
AGCUUCAACGACAGAACCGCCCCCCGCUUCGGCCUGUACAGGUACGCCGCCGACCAGUUCGAACCCAAGGCCAGCAUGAAGCACUA
CCGCAAAAUCAUCGACAGCAACGGA
SEQ ID NO: 10: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGGACACCACCCUGUCACAGUUCACUG
ACCUGAACGUGUACCUGUGGGACGUGCACCAUUCCAAGCGCCUGAUCAAGGUGGACGGCGUGGUGACCAAGAAGCGCAAAUCCUAC
UGCGUUGACUUUGCCGCCAUUCAGCCACAGAUCGCCCUGCUGCAGGAGAUGCACGUCACCCACUUCCGCUUCAGCCUGGACUGGGC
CCUCAUCCUGCCACUGGGCAACCAGUCCCAGGUGAACCACACCAUUCUGCAGUACUACCGCUGUAUGGCCAGCGAGCUGGUACGCG
UGAACAUCACCCCCGUGGUGGCCCUGUGGCAGCCCAUGGCCCCCAACCAGGGCCUGCCACGCCUGCUGGCCAGGCAGGGGGCCUGG
GAGAACCCCUACACCGCACUCGCUUUCGCCGAAUAUGCCAGGCUGUGCUUCCAGGAGCUGGGGCACCACGUGAAGCUCUGGAUCAC
CAUGAACGAACCCUACACCAGAAACAUGACCUACUCUGCCGGACACAAUCUGCUGAAGGCCCACGCCCUGGCCUGGCACGUCUACA
ACGAGAAGUUCCGCCACGCCCAAAAUGGAAAGAUCUCUAUAGCCCUGCAGGCCGAUUGGAUCGAGCCCGCCUGCCCCUUCUCACAG
AAGGACAAGGAGGUGGCCGAGCGCGUGCUGGAGUUCGACAUCGGCUGGCUGGCCGAGCCCAUCUUCGGCAGCGGAGACUACCCCUG
GGUGAUGCGCGAUUGGCUUAACCAGCGCAACAACUUUCUGCUGCCCUACUUCACCGAGGACGAGAAGAAGCUGAUCCAGGGCACCU
UCGACUUCCUGGCCCUGAGCCACUAUACUACCAUCCUGGUCGACAGCGAGAAGGAGGACCCAAUCAAGUACAACGACUACCUGGAG
GUGCAGGAGAUGACCGACAUCACCUGGCUGAACAGCCCUAGCCAGGUCGCCGUGGUGCCUUGGGGCCUGCGCAAGGUGCUGAACUG
GCUGAAGUUCAAGUAUGGCGACCUGCCAAUGUACAUCAUCAGCAACGGCAUUGAUGACGGCCUGCACGCCGAGGACGAUCAGCUGA
GAGUGUACUAUAUGCAGAACUACAUCAACGAGGCACUGAAGGCCCACAUCCUGGACGGCAUCAACCUGUGUGGCUACUUUGCAUAC
UCCUUCAACGACAGAACCGCCCCCCGCUUCGGCCUGUACAGGUACGCUGCCGAUCAGUUCGAGCCCAAGGCCAGCAUGAAGCACUA
CAGAAAAAUCAUUGAUAGCAACGGG
SEQ ID NO: 11: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGGACACCACCCUGAGUCAGUUCACUG
ACCUGAACGUGUACCUUUGGGACGUGCACCACAGCAAGCGCCUGAUAAAGGUCGACGGCGUGGUGACCAAGAAGCGCAAAAGCUAC
UGCGUGGACUUUGCCGCCAUCCAGCCCCAGAUCGCCCUGCUGCAGGAGAUGCACGUGACUCACUUCCGCUUCAGCCUCGACUGGGC
CCUGAUUCUGCCCCUGGGCAACCAGUCACAAGUGAACCACACCAUUCUGCAGUACUACCGCUGUAUGGCCAGCGAGCUGGUACGCG
UGAACAUCACCCCCGUUGUGGCCCUUUGGCAGCCCAUGGCCCCCAACCAGGGCCUGCCUCGCCUGCUGGCCCGCCAGGGAGCCUGG
GAGAAUCCCUACACCGCACUGGCCUUCGCUGAAUACGCCCGCCUGUGCUUCCAGGAGCUGGGGCACCACGUGAAACUCUGGAUCAC
CAUGAACGAGCCCUACACCAGAAACAUGACCUACAGCGCCGGACACAAUCUGCUGAAGGCCCACGCUCUGGCCUGGCACGUGUACA
ACGAGAAGUUUCGCCACGCCCAGAACGGCAAGAUCAGCAUCGCCCUGCAGGCCGAUUGGAUCGAGCCCGCCUGCCCCUUCAGCCAG
AAGGACAAGGAGGUGGCUGAGCGGGUGCUGGAGUUCGACAUCGGGUGGCUGGCUGAGCCUAUCUUCGGCAGCGGAGACUACCCCUG
GGUGAUGCGCGAUUGGCUUAACCAGAGGAACAAUUUCCUGCUGCCCUACUUCACCGAGGACGAGAAGAAGCUGAUCCAGGGCACCU
UUGACUUCCUGGCUUUGAGCCACUAUACCACUAUCCUGGUGGACAGCGAGAAGGAGGACCCCAUCAAGUACAACGACUACUUGGAG
GUGCAGGAGAUGACCGACAUCACCUGGCUGAACAGCCCUAGCCAGGUGGCUGUGGUGCCUUGGGGCCUGCGCAAGGUGCUGAACUG
GCUGAAGUUCAAGUACGGCGACCUGCCCAUGUAUAUCAUCAGCAACGGCAUCGACGACGGCCUGCACGCCGAGGACGACCAGCUGA
 
GAGUGUACUACAUGCAGAACUACAUCAACGAGGCCCUCAAGGCCCACAUCCUGGACGGCAUCAACCUGUGUGGCUACUUUGCCUAC
AGCUUCAACGACAGAACCGCCCCCCGCUUCGGCCUGUACAGGUACGCCGCCGACCAGUUCGAACCCAAGGCCAGCAUGAAGCACUA
CCGCAAAAUCAUCGACAGCAACGGA
SEQ ID NO: 12: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGGACACCACCCUGUCACAGUUCACUG
ACCUGAACGUGUACCUGUGGGACGUGCACCAUUCCAAGCGCCUGAUCAAGGUGGACGGCGUGGUGACCAAGAAGCGCAAAUCCUAC
UGCGUUGACUUUGCCGCCAUUCAGCCACAGAUCGCCCUGCUGCAGGAGAUGCACGUCACCCACUUCCGCUUCAGCCUGGACUGGGC
CCUCAUCCUGCCACUGGGCAACCAGUCCCAGGUGAACCACACCAUUCUGCAGUACUACCGCUGUAUGGCCAGCGAGCUGGUACGCG
UGAACAUCACCCCCGUGGUGGCCCUGUGGCAGCCCAUGGCCCCCAACCAGGGCCUGCCACGCCUGCUGGCCAGGCAGGGGGCCUGG
GAGAACCCCUACACCGCACUCGCUUUCGCCGAAUAUGCCAGGCUGUGCUUCCAGGAGCUGGGGCACCACGUGAAGCUCUGGAUCAC
CAUGAACGAACCCUACACCAGAAACAUGACCUACUCUGCCGGACACAAUCUGCUGAAGGCCCACGCCCUGGCCUGGCACGUCUACA
ACGAGAAGUUCCGCCACGCCCAAAAUGGAAAGAUCUCUAUAGCCCUGCAGGCCGAUUGGAUCGAGCCCGCCUGCCCCUUCUCACAG
AAGGACAAGGAGGUGGCCGAGCGCGUGCUGGAGUUCGACAUCGGCUGGCUGGCCGAGCCCAUCUUCGGCAGCGGAGACUACCCCUG
GGUGAUGCGCGAUUGGCUUAACCAGCGCAACAACUUUCUGCUGCCCUACUUCACCGAGGACGAGAAGAAGCUGAUCCAGGGCACCU
UCGACUUCCUGGCCCUGAGCCACUAUACUACCAUCCUGGUCGACAGCGAGAAGGAGGACCCAAUCAAGUACAACGACUACCUGGAG
GUGCAGGAGAUGACCGACAUCACCUGGCUGAACAGCCCUAGCCAGGUCGCCGUGGUGCCUUGGGGCCUGCGCAAGGUGCUGAACUG
GCUGAAGUUCAAGUAUGGCGACCUGCCAAUGUACAUCAUCAGCAACGGCAUUGAUGACGGCCUGCACGCCGAGGACGAUCAGCUGA
GAGUGUACUAUAUGCAGAACUACAUCAACGAGGCACUGAAGGCCCACAUCCUGGACGGCAUCAACCUGUGUGGCUACUUUGCAUAC
UCCUUCAACGACAGAACCGCCCCCCGCUUCGGCCUGUACAGGUACGCUGCCGAUCAGUUCGAGCCCAAGGCCAGCAUGAAGCACUA
CAGAAAAAUCAUUGAUAGCAACGGG
SEQ ID NO: 13: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
 
ACGGAUUCCCUGGACCCGAGACACUGGAGCGCUUCUGCCCAGAGGAGUUCACCGUGUGCACCGAGUGCAGUUUCUUCCACACCCGC
AAGAGC
SEQ ID NO: 14: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGC
SEQ ID NO: 15: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
 
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGC
SEQ ID NO: 16: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGC
SEQ ID NO: 17: 
AUGCCCGCCAGCGCCCCGCCGCGCCGCCCGCGGCCGCCGCCGUCGCUGUCGCUGCUGCUGGUGCUGCUGGGCCUGGGCGGCCG
CCGCCUGCGUGCGGAGCCGGGCGACGGCGCGCAGACCUGGGCCCGUUUCUCGCGGCCUCCUGCCCCCGAGGCCGCGGGCCUCUUCC
AGGGCACCUUCCCCGACGGCUUCCUCUGGGCCGUGGGCAGCGCCGCCUACCAGACCGAGGGCGGCUGGCAGCAGCACGGCAAGGGU
GCGUCCAUCUGGGAUACGUUCACCCACCACCCCCUGGCACCCCCGGGAGACUCCCGGAACGCCAGUCUGCCGUUGGGCGCCCCGUC
GCCGCUGCAGCCCGCCACCGGGGACGUAGCCAGCGACAGCUACAACAACGUCUUCCGCGACACGGAGGCGCUGCGCGAGCUCGGGG
UCACUCACUACCGCUUCUCCAUCUCGUGGGCGCGAGUGCUCCCCAAUGGCAGCGCGGGCGUCCCCAACCGCGAGGGGCUGCGCUAC
UACCGGCGCCUGCUGGAGCGGCUGCGGGAGCUGGGCGUGCAGCCCGUGGUCACCCUGUACCACUGGGACCUGCCCCAGCGCCUGCA
GGACGCCUACGGCGGCUGGGCCAACCGCGCCCUGGCCGACCACUUCAGGGAUUACGCGGAGCUCUGCUUCCGCCACUUCGGCGGUC
AGGUCAAGUACUGGAUCACCAUCGACAACCCCUACGUGGUGGCCUGGCACGGCUACGCCACCGGGCGCCUGGCCCCCGGCAUCCGG
GGCAGCCCGCGGCUCGGGUACCUGGUGGCGCACAACCUCCUCCUGGCUCAUGCCAAAGUCUGGCAUCUCUACAAUACUUCUUUCCG
UCCCACUCAGGGAGGUCAGGUGUCCAUUGCCCUAAGCUCUCACUGGAUCAAUCCUCGAAGAAUGACCGACCACAGCAUCAAAGAAU
GUCAAAAAUCUCUGGACUUUGUACUAGGUUGGUUUGCCAAACCCGUAUUUAUUGAUGGUGACUAUCCCGAGAGCAUGAAGAAUAAC
CUUUCAUCUAUUCUGCCUGAUUUUACUGAAUCUGAGAAAAAGUUCAUCAAAGGAACUGCUGACUUUUUUGCUCUUUGCUUUGGACC
CACCUUGAGUUUUCAACUUUUGGACCCUCACAUGAAGUUCCGCCAAUUGGAAUCUCCCAACCUGAGGCAACUGCUUUCCUGGAUUG
ACCUUGAAUUUAACCAUCCUCAAAUAUUUAUUGUGGAAAAUGGCUGGUUUGUCUCAGGGACCACCAAGAGAGAUGAUGCCAAAUAU
AUGUAUUACCUCAAAAAGUUCAUCAUGGAAACCUUAAAAGCCAUCAAGCUGGAUGGGGUGGAUGUCAUCGGGUAUACCGCAUGGUC
CCUCAUGGAUGGUUUCGAGUGGCACAGAGGUUACAGCAUCAGGCGUGGACUCUUCUAUGUUGACUUUCUAAGCCAGGACAAGAUGU
UGUUGCCAAAGUCUUCAGCCUUGUUCUACCAAAAGCUGAUAGAGAAAAAUGGCUUCCCUCCUUUACCUGAAAAUCAGCCCCUAGAA
GGGACAUUUCCCUGUGACUUUGCUUGGGGAGUUGUUGACAACUACAUUCAAGUAGAUACCACUCUGUCUCAGUUUACCGACCUGAA
UGUUUACCUGUGGGAUGUCCACCACAGUAAAAGGCUUAUUAAAGUGGAUGGGGUUGUGACCAAGAAGAGGAAAUCCUACUGUGUUG
ACUUUGCUGCCAUCCAGCCCCAGAUCGCUUUACUCCAGGAAAUGCACGUUACACAUUUUCGCUUCUCCCUGGACUGGGCCCUGAUU
CUCCCUCUGGGUAACCAGUCCCAGGUGAACCACACCAUCCUGCAGUACUAUCGCUGCAUGGCCAGCGAGCUUGUCCGUGUCAACAU
CACCCCAGUGGUGGCCCUGUGGCAGCCUAUGGCCCCGAACCAAGGACUGCCGCGCCUCCUGGCCAGGCAGGGCGCCUGGGAGAACC
CCUACACUGCCCUGGCCUUUGCAGAGUAUGCCCGACUGUGCUUUCAAGAGCUCGGCCAUCACGUCAAGCUUUGGAUAACGAUGAAU
GAGCCGUAUACAAGGAAUAUGACAUACAGUGCUGGCCACAACCUUCUGAAGGCCCAUGCCCUGGCUUGGCAUGUGUACAAUGAAAA
GUUUAGGCAUGCUCAGAAUGGGAAAAUAUCCAUAGCCUUGCAGGCUGAUUGGAUAGAACCUGCCUGCCCUUUCUCCCAAAAGGACA
AAGAGGUGGCUGAGAGAGUUUUGGAAUUUGACAUUGGCUGGCUGGCUGAGCCCAUUUUCGGCUCUGGAGAUUAUCCAUGGGUGAUG
AGGGACUGGCUGAACCAAAGAAACAAUUUUCUUCUUCCUUAUUUCACUGAAGAUGAAAAAAAGCUAAUCCAGGGUACCUUUGACUU
UUUGGCUUUAAGCCAUUAUACCACCAUCCUUGUAGACUCAGAAAAAGAAGAUCCAAUAAAAUACAAUGAUUACCUAGAAGUGCAAG
AAAUGACCGACAUCACGUGGCUCAACUCCCCCAGUCAGGUGGCGGUAGUGCCCUGGGGGUUGCGCAAAGUGCUGAACUGGCUGAAG
 
UUCAAGUACGGAGACCUCCCCAUGUACAUAAUAUCCAAUGGAAUCGAUGACGGGCUGCAUGCUGAGGACGACCAGCUGAGGGUGUA
UUAUAUGCAGAAUUACAUAAACGAAGCUCUCAAAGCCCACAUACUGGAUGGUAUCAAUCUUUGCGGAUACUUUGCUUAUUCGUUUA
ACGACCGCACAGCUCCGAGGUUUGGCCUCUAUCGUUAUGCUGCAGAUCAGUUUGAGCCCAAGGCAUCCAUGAAACAUUACAGGAAA
AUUAUUGACAGCAAUGGUUUCCCGGGCCCAGAAACUCUGGAAAGAUUUUGUCCAGAAGAAUUCACCGUGUGUACUGAGUGCAGUUU
UUUUCACACCCGAAAGUCUUGA
SEQ ID NO: 18: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCUGA
SEQ ID NO: 19: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
 
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCUGA
SEQ ID NO: 20: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCCUGA
SEQ ID NO: 21: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
 
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCUGA
SEQ ID NO: 22: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCAGGCGAUGGCGCCCAGACUUGGGCCCGCUUCAGCAGACCUCCCGCCCCCGAGGCCGCCGGAUUGUUCC
AGGGAACCUUCCCUGACGGUUUCCUGUGGGCCGUGGGUAGCGCCGCCUACCAGACCGAGGGCGGCUGGCAGCAGCACGGCAAGGGC
GCAAGCAUCUGGGACACCUUUACCCACCACCCCCUGGCCCCCCCCGGCGACAGUAGAAAUGCUAGUCUCCCCCUGGGCGCCCCCAG
CCCCCUUCAGCCCGCCACCGGAGACGUGGCCUCAGACAGCUACAACAACGUGUUCAGAGACACCGAAGCUCUGAGGGAGCUCGGCG
UGACCCACUAUCGGUUUAGCAUUAGCUGGGCCAGAGUGCUGCCCAACGGCAGCGCCGGCGUGCCCAACCGCGAAGGCCUCAGAUAC
UAUCGCAGACUGCUGGAGAGGCUGCGGGAGCUGGGCGUGCAGCCCGUGGUAACUCUGUACCACUGGGAUCUGCCCCAGAGAUUGCA
AGACGCUUACGGCGGAUGGGCCAACCGCGCUCUCGCCGACCACUUCCGGGACUACGCAGAGCUGUGCUUCCGCCACUUCGGCGGUC
AGGUGAAAUAUUGGAUUACAAUCGACAACCCCUACGUUGUGGCCUGGCACGGGUACGCUACUGGGCGCCUGGCACCCGGCAUCCGC
GGCAGCCCCAGACUGGGCUACCUUGUUGCUCACAACCUCCUGCUGGCUCAUGCAAAGGUGUGGCACUUGUACAACACAAGUUUCCG
CCCCACCCAGGGUGGACAGGUGUCAAUCGCACUGUCUUCACACUGGAUUAACCCCCGGCGCAUGACUGACCACUCAAUUAAGGAAU
GCCAGAAGAGCCUGGACUUCGUUCUGGGCUGGUUCGCCAAGCCCGUGUUUAUCGACGGGGACUACCCCGAGAGCAUGAAGAAUAAU
CUGAGCAGCAUCUUGCCCGACUUUACCGAGAGCGAAAAGAAAUUCAUCAAGGGCACCGCCGACUUUUUCGCACUGUGUUUCGGCCC
CACUCUGAGCUUUCAACUGCUCGAUCCCCACAUGAAGUUUAGACAACUGGAGUCCCCUAAUCUGCGCCAGCUUCUGAGCUGGAUUG
AUCUGGAAUUUAACCAUCCCCAGAUUUUUAUAGUUGAGAAUGGGUGGUUUGUUAGCGGCACCACUAAGCGCGAUGACGCAAAGUAC
AUGUAUUAUCUGAAGAAAUUUAUCAUGGAAACUCUGAAGGCCAUAAAACUCGACGGCGUUGACGUGAUCGGUUAUACCGCAUGGAG
CCUUAUGGACGGGUUCGAGUGGCACCGCGGGUACUCCAUCAGGAGGGGCUUGUUCUACGUCGACUUCCUCAGUCAGGACAAGAUGU
UGCUGCCUAAGAGCAGCGCCUUGUUCUACCAGAAGUUGAUCGAAAAGAACGGCUUUCCACCACUCCCCGAGAACCAACCCCUGGAG
GGCACCUUUCCCUGCGAUUUCGCUUGGGGCGUGGUGGACAACUACAUCCAGGUGGAUACCACUCUGUCUCAGUUCACUGAUCUCAA
CGUGUAUCUGUGGGACGUCCACCACUCCAAGAGGCUGAUCAAGGUGGACGGCGUCGUGACCAAGAAGAGAAAGUCCUACUGCGUGG
ACUUUGCCGCCAUUCAGCCCCAGAUCGCCCUGCUGCAGGAGAUGCAUGUCACACACUUCCGCUUUUCUCUGGACUGGGCCCUGAUU
CUGCCCCUGGGGAACCAGUCCCAGGUCAACCACACCAUUCUCCAGUACUACCGCUGUAUGGCCAGUGAGCUGGUACGCGUGAACAU
CACCCCCGUCGUGGCCCUCUGGCAGCCCAUGGCCCCCAAUCAGGGCCUGCCACGCCUGCUCGCCCGGCAGGGAGCCUGGGAAAAUC
CAUACACUGCACUCGCCUUCGCUGAGUACGCCAGGCUGUGCUUCCAGGAGCUGGGACAUCACGUGAAGCUGUGGAUUACCAUGAAC
GAACCAUACACUAGAAAUAUGACCUACUCCGCCGGACACAAUCUCCUGAAGGCCCACGCACUGGCUUGGCACGUGUACAACGAGAA
GUUCAGGCACGCCCAGAAUGGAAAGAUUAGCAUCGCCCUGCAGGCCGACUGGAUCGAGCCCGCCUGUCCCUUCAGCCAGAAAGACA
AAGAGGUGGCUGAGCGCGUGCUGGAGUUUGAUAUUGGCUGGCUUGCUGAGCCAAUAUUCGGCAGUGGAGACUACCCCUGGGUGAUG
AGAGAUUGGCUUAACCAGAGAAACAACUUUCUCCUGCCCUACUUCACCGAGGACGAAAAGAAACUGAUCCAGGGUACCUUCGAUUU
CCUUGCUCUGAGCCACUAUACUACCAUCCUGGUCGACAGCGAGAAGGAGGACCCCAUCAAAUAUAACGACUACCUGGAGGUGCAAG
AGAUGACUGACAUCACAUGGCUGAAUAGCCCUAGCCAGGUCGCCGUGGUGCCUUGGGGCCUGCGCAAGGUGCUCAACUGGCUGAAG
UUCAAGUAUGGCGACCUGCCCAUGUACAUCAUCAGCAACGGCAUUGAUGACGGACUGCACGCCGAGGAUGAUCAACUCAGAGUAUA
CUAUAUGCAGAACUAUAUCAACGAGGCAUUGAAGGCCCACAUCCUGGACGGCAUCAACCUGUGUGGGUACUUUGCAUACUCCUUCA
ACGACAGAACCGCUCCCCGCUUUGGUCUGUAUAGGUACGCUGCCGACCAGUUCGAGCCCAAGGCCAGCAUGAAGCACUACCGCAAG
AUUAUCGAUAGCAAUGGCUUCCCUGGCCCCGAGACCCUUGAGCGCUUUUGCCCCGAGGAGUUCACCGUGUGUACCGAAUGUUCCUU
CUUCCACACACGCAAGUCAUGA
SEQ ID NO: 23: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGAUGGCGCUCAGACUUGGGCUCGCUUCAGCAGACCCCCAGCCCCCGAAGCAGCCGGAUUGUUCC
AGGGAACCUUCCCCGAUGGUUUCCUGUGGGCCGUAGGUAGCGCCGCCUACCAGACUGAGGGCGGAUGGCAGCAGCACGGCAAGGGC
GCAAGCAUCUGGGACACCUUCACCCACCACCCCCUGGCCCCCCCUGGCGACUCCCGCAAUGCUAGUCUCCCCCUGGGCGCCCCUAG
CCCCCUUCAGCCCGCCACUGGCGACGUGGCCUCAGACAGCUACAACAAUGUGUUCCGGGACACAGAAGCUCUGAGGGAGCUUGGCG
UGACCCAUUAUAGGUUUAGCAUCUCAUGGGCCCGCGUGCUGCCAAAUGGCAGCGCCGGCGUGCCUAACCGCGAAGGACUUCGCUAC
UAUCGCAGACUGCUGGAGAGGCUGCGGGAACUGGGGGUGCAACCUGUGGUGACAUUGUACCACUGGGAUCUGCCUCAGCGCUUGCA
AGACGCUUACGGUGGAUGGGCCAACCGCGCUCUGGCCGACCAUUUCCGGGACUACGCAGAGCUUUGCUUCCGCCACUUCGGGGGUC
AAGUGAAAUAUUGGAUUACAAUCGAUAACCCCUACGUGGUUGCAUGGCAUGGCUACGCCACAGGGCGCCUUGCUCCCGGCAUCAGA
GGAAGCCCCAGGCUGGGUUAUCUUGUGGCACACAACCUCCUGCUGGCACACGCAAAAGUGUGGCACUUGUAUAACACAAGUUUCCG
CCCCACCCAGGGUGGACAGGUGUCAAUAGCACUGUCUUCACACUGGAUUAAUCCUCGGAGGAUGACAGAUCACUCCAUCAAGGAAU
GCCAGAAGAGCCUGGACUUCGUCCUGGGCUGGUUUGCUAAGCCCGUGUUUAUCGACGGGGACUACCCCGAGAGCAUGAAGAAUAAU
CUGAGCAGCAUAUUGCCAGAUUUUACCGAGUCCGAGAAGAAAUUUAUCAAAGGGACCGCUGACUUUUUCGCUCUGUGUUUCGGCCC
CACUCUGAGCUUUCAAUUGCUGGAUCCACACAUGAAGUUUAGACAACUGGAAAGUCCCAAUCUGCGGCAGUUGCUGAGCUGGAUUG
AUCUGGAAUUCAACCAUCCCCAGAUUUUUAUUGUUGAGAAUGGGUGGUUUGUUAGUGGCACUACAAAGAGGGAUGACGCCAAGUAC
AUGUACUAUCUGAAGAAAUUCAUCAUGGAGACCCUGAAGGCCAUAAAGCUCGACGGAGUUGACGUGAUCGGUUAUACCGCAUGGAG
CCUGAUGGACGGGUUCGAGUGGCACCGCGGGUACAGCAUCAGGAGAGGCUUGUUCUACGUCGACUUCUUGUCACAGGACAAAAUGU
UGUUGCCUAAGUCAAGUGCCUUGUUUUACCAGAAACUGAUUGAAAAAAACGGCUUUCCUCCUUUGCCCGAGAACCAACCUCUGGAG
GGAACCUUUCCCUGCGAUUUUGCUUGGGGCGUGGUGGACAACUACAUCCAGGUGGACACAACCCUGUCUCAGUUCACCGAUUUGAA
UGUGUAUCUCUGGGAUGUCCACCACUCCAAGCGCCUGAUCAAGGUGGAUGGCGUCGUGACCAAGAAAAGAAAGAGCUACUGCGUGG
ACUUUGCCGCCAUUCAGCCCCAGAUUGCCCUGCUCCAGGAGAUGCACGUCACACACUUUAGGUUCUCCCUGGACUGGGCCCUGAUA
 
CUGCCCCUGGGUAACCAGUCACAAGUGAACCAUACCAUUUUGCAGUACUACCGCUGUAUGGCUUCAGAGCUGGUACGCGUGAACAU
CACCCCCGUAGUUGCCCUCUGGCAGCCCAUGGCCCCCAAUCAGGGCCUGCCUCGCCUGCUCGCCCGGCAGGGAGCCUGGGAAAACC
CAUACACCGCACUUGCCUUCGCUGAGUACGCCAGGUUGUGCUUCCAGGAGCUGGGACAUCACGUGAAGCUUUGGAUUACCAUGAAC
GAGCCAUACACCAGGAACAUGACAUACUCCGCCGGACAUAAUCUGCUGAAGGCUCACGCACUGGCCUGGCACGUGUACAACGAGAA
GUUUCGGCAUGCCCAGAAUGGAAAGAUCAGCAUCGCUCUGCAGGCAGACUGGAUCGAGCCUGCCUGUCCUUUCAGUCAGAAAGAUA
AAGAGGUGGCCGAGCGGGUGCUGGAAUUCGAUAUCGGAUGGCUGGCUGAACCAAUCUUCGGCAGUGGGGACUACCCCUGGGUGAUG
AGAGAUUGGCUGAACCAGCGCAACAAUUUUCUGCUGCCCUACUUUACCGAGGACGAAAAGAAGCUUAUCCAGGGUACCUUCGAUUU
CCUUGCCCUGUCCCACUAUACUACCAUACUUGUCGACUCUGAGAAAGAGGACCCCAUCAAAUACAAUGACUACCUGGAGGUGCAAG
AAAUGACAGACAUAACCUGGCUGAAUAGCCCUAGUCAGGUCGCUGUGGUGCCUUGGGGCCUCCGCAAAGUGUUGAACUGGCUGAAG
UUUAAGUAUGGCGACCUGCCCAUGUACAUCAUUAGCAACGGGAUUGAUGACGGACUGCACGCUGAGGAUGAUCAACUGAGAGUAUA
CUAUAUGCAGAAUUACAUCAAUGAGGCUUUGAAGGCCCACAUCCUGGAUGGAAUUAACCUGUGCGGGUACUUUGCAUACUCCUUCA
ACGAUAGAACCGCUCCCCGCUUUGGUCUGUAUAGGUACGCUGCCGAUCAGUUCGAGCCCAAAGCCAGCAUGAAGCACUACCGCAAG
AUCAUCGAUAGCAACGGCUUCCCUGGGCCCGAGACUCUCGAGAGGUUUUGCCCCGAGGAGUUCACCGUGUGUACCGAAUGCAGUUU
UUUCCACACAAGGAAAUCAUGA
SEQ ID NO: 24: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGACGGCGCUCAGACAUGGGCCCGGUUCAGCCGGCCACCAGCCCCCGAAGCUGCUGGAUUGUUCC
AGGGAACCUUUCCUGACGGGUUCCUGUGGGCAGUAGGCUCUGCUGCCUAUCAGACUGAGGGUGGAUGGCAGCAGCACGGUAAAGGC
GCAAGCAUUUGGGAUACAUUCACCCACCACCCACUGGCACCCCCAGGCGACAGCCGCAACGCUUCUCUUCCACUGGGAGCCCCCAG
UCCUCUUCAGCCCGCCACAGGCGACGUUGCAUCAGAUAGCUAUAACAAUGUGUUCCGGGACACCGAAGCUCUGAGGGAGCUCGGCG
UGACACACUAUCGGUUCAGCAUCAGUUGGGCCAGGGUGCUGCCAAACGGCAGUGCUGGUGUGCCCAACCGCGAAGGUCUCAGAUAC
UAUCGCCGCCUUCUGGAGAGGCUGAGAGAACUGGGCGUGCAGCCAGUGGUGACUCUGUAUCACUGGGAUCUGCCACAGAGACUGCA
AGAUGCUUAUGGUGGUUGGGCCAACCGCGCCCUGGCAGAUCAUUUCCGGGACUAUGCAGAACUGUGCUUCCGGCACUUUGGAGGAC
AGGUUAAAUAUUGGAUUACUAUCGACAACCCCUAUGUUGUGGCCUGGCACGGGUACGCCACUGGGCGCCUCGCUCCCGGCAUAAGG
GGCUCCCCCAGGCUGGGUUACCUUGUCGCCCACAACCUCUUGCUCGCUCACGCCAAGGUCUGGCAUCUGUACAAUACUUCUUUCCG
CCCCACCCAGGGUGGUCAGGUGUCAAUCGCACUGAGCAGCCACUGGAUUAACCCCAGGCGCAUGACCGAUCAUUCUAUUAAGGAGU
GCCAGAAGAGCCUUGAUUUCGUUCUGGGCUGGUUUGCUAAACCAGUCUUUAUUGACGGUGACUACCCUGAGAGCAUGAAAAAUAAU
CUGUCCAGCAUCUUGCCAGAUUUUACCGAGAGCGAAAAGAAAUUUAUCAAGGGUACCGCUGAUUUUUUCGCUUUGUGUUUCGGCCC
CACAUUGAGCUUCCAGCUGCUGGACCCACACAUGAAGUUUCGCCAGCUGGAGUCACCCAACUUGCGGCAGCUGCUGUCAUGGAUCG
ACCUGGAAUUUAAUCACCCCCAGAUCUUUAUUGUUGAGAAUGGGUGGUUUGUUUCUGGCACUACUAAGAGGGAUGACGCAAAGUAU
AUGUAUUAUCUGAAGAAGUUUAUUAUGGAGACCCUGAAAGCAAUCAAGCUCGAUGGAGUGGAUGUGAUCGGUUAUACCGCUUGGAG
CCUUAUGGAUGGAUUUGAGUGGCACAGGGGGUAUUCAAUAAGGAGAGGCUUGUUCUACGUGGACUUUCUGUCACAGGAUAAAAUGU
UGCUGCCCAAAUCAAGCGCCCUUUUUUACCAGAAAUUGAUAGAAAAGAACGGCUUCCCUCCUCUCCCAGAGAACCAGCCACUGGAG
GGAACAUUUCCUUGCGAUUUCGCAUGGGGAGUGGUGGAUAACUACAUACAGGUGGACACCACUCUGUCUCAGUUUACCGAUCUCAA
UGUGUAUCUGUGGGACGUUCACCAUUCCAAGCGCCUCAUUAAAGUGGAUGGCGUUGUCACUAAGAAAAGAAAGAGCUACUGUGUGG
ACUUUGCUGCCAUUCAGCCACAGAUCGCUCUGCUCCAGGAGAUGCACGUGACCCACUUCCGGUUCUCCCUGGACUGGGCCUUGAUA
CUGCCCUUGGGCAACCAGAGUCAGGUCAAUCAUACCAUCCUGCAGUACUAUCGCUGUAUGGCCUCAGAGCUGGUUCGCGUGAACAU
AACCCCCGUGGUGGCACUGUGGCAGCCCAUGGCACCAAAUCAGGGCCUGCCCAGACUGCUGGCUAGACAGGGCGCCUGGGAAAAUC
CCUACACUGCACUGGCAUUUGCUGAGUACGCCAGGUUGUGUUUUCAGGAGCUGGGACAUCACGUUAAGCUUUGGAUUACCAUGAAC
GAACCAUAUACUAGAAACAUGACCUAUUCCGCCGGACACAAUCUGCUGAAAGCCCACGCCCUGGCAUGGCAUGUGUAUAACGAGAA
AUUCAGGCACGCCCAGAACGGAAAGAUCAGCAUUGCCCUGCAAGCAGACUGGAUUGAGCCAGCCUGUCCUUUCUCACAAAAAGACA
AAGAGGUGGCCGAACGGGUCCUGGAGUUCGACAUCGGCUGGCUCGCUGAACCAAUCUUUGGCAGUGGCGACUACCCUUGGGUUAUG
AGGGACUGGCUCAACCAGAGAAACAACUUUCUUCUUCCCUAUUUUACAGAGGAUGAGAAGAAACUGAUUCAGGGCACCUUCGAUUU
UCUUGCUCUCAGCCAUUAUACUACUAUCCUUGUGGAUAGUGAAAAAGAGGACCCAAUUAAGUACAAUGACUAUCUCGAAGUGCAAG
AGAUGACCGACAUCACCUGGCUGAAUAGCCCAUCCCAGGUCGCAGUGGUGCCUUGGGGCCUGCGGAAGGUGCUGAACUGGCUGAAG
UUUAAGUAUGGCGACCUGCCCAUGUACAUUAUUAGCAACGGGAUAGACGAUGGACUGCACGCAGAAGAUGAUCAGCUGCGCGUGUA
UUAUAUGCAAAAUUAUAUCAACGAAGCCUUGAAGGCCCACAUCCUGGAUGGAAUCAACCUGUGUGGCUAUUUUGCAUACUCAUUCA
ACGACCGCACCGCACCACGGUUUGGUCUGUAUAGGUACGCUGCCGACCAGUUUGAGCCAAAAGCCAGUAUGAAGCACUACCGGAAA
AUCAUCGAUUCUAAUGGCUUCCCUGGCCCUGAGACCCUGGAGAGAUUUUGCCCCGAGGAAUUCACCGUGUGUACAGAAUGUUCAUU
UUUUCACACCCGGAAAAGUUGA
SEQ ID NO: 25: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAACCCGGCGAUGGAGCCCAAACAUGGGCCAGAUUCAGCAGACCUCCCGCUCCAGAAGCUGCUGGUCUGUUUC
AGGGUACGUUUCCUGACGGUUUUCUCUGGGCAGUCGGCUCUGCUGCCUAUCAGACUGAGGGAGGUUGGCAACAACAUGGCAAAGGU
GCUAGCAUAUGGGACACUUUCACGCAUCACCCACUGGCCCCGCCUGGGGAUAGUCGCAACGCAAGUCUGCCACUGGGAGCACCCUC
UCCUCUGCAACCGGCUACCGGGGAUGUGGCUUCCGACUCUUACAACAACGUGUUCAGGGAUACUGAAGCGCUUAGGGAAUUGGGCG
UGACGCAUUACCGCUUUAGCAUUAGCUGGGCCAGAGUCUUGCCUAAUGGAUCAGCCGGUGUGCCAAAUCGCGAAGGGCUGCGAUAC
UAUAGGCGGCUGUUGGAGAGGCUCCGGGAACUGGGUGUUCAGCCUGUGGUGACACUCUAUCACUGGGACCUCCCACAGAGACUUCA
AGACGCGUACGGAGGCUGGGCAAAUCGGGCCUUGGCUGAUCAUUUCCGGGACUAUGCAGAGCUGUGUUUCCGUCACUUUGGCGGCC
AGGUAAAGUAUUGGAUUACCAUAGAUAACCCUUAUGUCGUAGCUUGGCAUGGCUAUGCGACAGGGAGACUUGCUCCUGGGAUUCGC
GGAUCUCCCAGGCUGGGAUAUCUGGUCGCUCACAAUCUGUUGCUGGCCCAUGCCAAAGUAUGGCACCUGUACAACACCUCAUUCCG
GCCGACCCAAGGCGGCCAGGUGUCAAUUGCCCUUAGCAGCCACUGGAUUAAUCCCCGCCGAAUGACCGAUCACAGCAUUAAGGAGU
GCCAGAAAUCCCUGGACUUUGUUUUGGGGUGGUUUGCGAAACCGGUGUUCAUCGAUGGGGACUACCCCGAGUCCAUGAAGAACAAU
CUGAGUUCUAUCCUGCCAGACUUCACAGAAUCCGAGAAGAAGUUCAUCAAGGGGACUGCCGACUUCUUCGCACUGUGCUUUGGCCC
CACACUCUCCUUUCAGCUGCUCGAUCCUCACAUGAAAUUUCGCCAGCUUGAGAGCCCAAAUCUCCGUCAGCUGCUUUCAUGGAUAG
ACCUGGAGUUCAACCAUCCCCAGAUCUUUAUCGUCGAAAACGGUUGGUUCGUGAGUGGAACCACAAAGAGGGACGAUGCCAAAUAC
AUGUAUUACCUCAAGAAGUUCAUUAUGGAGACUCUGAAGGCCAUCAAACUCGACGGAGUUGACGUGAUCGGCUAUACUGCAUGGUC
CUUGAUGGAUGGAUUCGAAUGGCAUAGAGGGUACAGCAUCCGAAGAGGCCUGUUUUACGUGGACUUUCUGAGCCAGGACAAGAUGC
UGCUCCCCAAAAGCUCAGCCCUCUUCUACCAGAAACUGAUCGAGAAGAACGGCUUUCCACCCUUGCCCGAGAAUCAGCCUCUGGAG
 
GGCACAUUCCCCUGUGACUUUGCCUGGGGCGUUGUGGACAAUUACAUUCAGGUGGAUACCACCCUGUCUCAGUUCACCGAUCUGAA
CGUGUACCUCUGGGAUGUUCACCACAGUAAACGUCUGAUCAAGGUCGAUGGGGUGGUUACAAAGAAACGCAAGAGUUAUUGCGUCG
AUUUUGCAGCCAUCCAGCCGCAGAUUGCCCUGCUCCAAGAGAUGCACGUGACACACUUCAGAUUUUCCCUGGAUUGGGCCCUGAUU
CUCCCUCUGGGAAACCAGAGCCAGGUAAACCACACCAUUCUGCAGUACUACCGUUGCAUGGCAAGUGAACUGGUGAGAGUGAACAU
UACUCCAGUGGUAGCUUUGUGGCAGCCUAUGGCCCCUAAUCAAGGACUGCCAAGACUGCUCGCCCGUCAAGGCGCAUGGGAAAAUC
CCUAUACGGCUCUUGCCUUUGCUGAGUAUGCAAGGCUGUGCUUUCAGGAACUGGGACAUCACGUGAAGUUGUGGAUUACCAUGAAU
GAGCCUUAUACUCGAAACAUGACCUAUUCAGCUGGCCAUAAUCUCCUCAAAGCCCAUGCAUUGGCUUGGCACGUCUACAACGAGAA
GUUUCGCCAUGCCCAAAAUGGCAAGAUCAGCAUUGCCCUCCAAGCAGACUGGAUCGAGCCAGCAUGUCCCUUUUCUCAGAAAGACA
AGGAAGUCGCCGAACGGGUGCUGGAGUUUGACAUCGGAUGGCUGGCUGAGCCCAUAUUUGGGAGUGGCGAUUACCCCUGGGUGAUG
AGGGACUGGUUGAACCAGAGGAACAACUUUCUUCUCCCGUAUUUCACAGAGGACGAGAAGAAGCUGAUCCAGGGUACAUUUGACUU
CCUGGCUCUGUCCCAUUACACCACCAUUCUGGUUGAUUCUGAGAAGGAGGAUCCCAUCAAGUACAAUGACUACCUGGAAGUACAGG
AGAUGACGGACAUUACCUGGCUGAAUUCCCCAUCACAGGUUGCUGUGGUUCCAUGGGGUUUGCGCAAAGUGCUGAACUGGCUCAAG
UUCAAAUAUGGGGAUCUCCCGAUGUAUAUUAUCUCUAAUGGCAUAGACGAUGGGCUUCAUGCAGAAGAUGAUCAGCUGAGAGUCUA
CUAUAUGCAGAACUACAUCAACGAAGCGCUUAAAGCGCACAUAUUGGACGGAAUUAAUCUGUGUGGGUACUUCGCCUAUAGCUUCA
AUGAUCGGACUGCUCCCCGGUUUGGCCUGUAUCGCUAUGCCGCGGACCAGUUCGAGCCUAAAGCGAGCAUGAAGCACUACAGGAAA
AUCAUCGACUCAAACGGCUUCCCAGGUCCUGAAACUCUUGAACGGUUUUGUCCCGAGGAAUUCACCGUCUGCACUGAGUGUAGCUU
CUUCCACACACGAAAAUCCUGA
SEQ ID NO: 26: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCCGGCGAUGGGGCCCAAACCUGGGCUCGGUUCAGUAGACCGCCUGCACCUGAAGCUGCAGGGUUGUUCC
AGGGCACUUUUCCGGAUGGGUUUCUCUGGGCGGUAGGGUCCGCGGCAUAUCAGACCGAGGGGGGGUGGCAACAACACGGGAAGGGU
GCGUCAAUAUGGGACACUUUUACUCAUCACCCACUUGCGCCUCCCGGAGAUUCAAGGAAUGCCUCUCUGCCUCUGGGAGCACCCAG
UCCUCUCCAACCCGCAACUGGCGAUGUCGCAUCAGACAGUUACAACAACGUUUUUAGGGACACAGAAGCGCUCCGAGAACUGGGGG
UUACUCAUUAUCGGUUUUCCAUUUCAUGGGCCCGAGUACUCCCAAAUGGGUCCGCUGGAGUACCGAACCGCGAGGGCCUCCGCUAU
UAUAGAAGACUCCUCGAGCGCCUGCGCGAACUCGGUGUUCAACCGGUUGUCACCCUGUACCAUUGGGAUCUCCCGCAACGCCUGCA
AGAUGCUUACGGGGGCUGGGCAAACCGCGCACUGGCGGAUCAUUUUCGAGAUUACGCCGAGCUGUGCUUCCGGCAUUUUGGUGGGC
AAGUCAAGUAUUGGAUCACCAUUGACAACCCAUAUGUCGUUGCAUGGCAUGGAUAUGCAACCGGGAGGCUUGCACCGGGGAUUAGG
GGGAGCCCUAGACUUGGAUACCUGGUCGCCCAUAAUCUGUUGCUCGCUCAUGCGAAGGUCUGGCAUCUGUAUAACACUAGCUUCCG
GCCGACGCAAGGGGGCCAAGUAUCCAUUGCACUGUCUUCCCACUGGAUUAAUCCCAGACGCAUGACCGAUCACAGCAUAAAGGAGU
GUCAAAAGUCCCUGGAUUUUGUCCUGGGCUGGUUUGCAAAGCCCGUCUUCAUAGACGGCGACUACCCUGAGAGCAUGAAGAAUAAC
CUGUCUUCUAUUCUUCCGGAUUUCACGGAGUCAGAAAAAAAGUUUAUCAAGGGCACCGCCGACUUCUUCGCACUGUGUUUCGGACC
GACAUUGUCCUUUCAGCUUUUGGACCCUCAUAUGAAAUUCAGGCAGCUCGAGUCCCCCAAUCUGCGACAGCUGUUGUCAUGGAUUG
AUUUGGAAUUUAACCACCCACAAAUUUUUAUUGUCGAAAAUGGUUGGUUUGUUAGUGGGACUACCAAACGCGACGAUGCGAAGUAC
AUGUACUAUUUGAAGAAGUUUAUAAUGGAGACCCUUAAAGCCAUCAAAUUGGAUGGCGUGGAUGUGAUAGGUUAUACUGCAUGGAG
CUUGAUGGACGGCUUCGAGUGGCAUCGAGGGUACAGCAUAAGGAGGGGUCUUUUCUACGUUGAUUUCUUGUCUCAGGAUAAGAUGC
UUCUGCCAAAAAGUUCAGCGCUUUUUUAUCAGAAAUUGAUAGAAAAGAAUGGUUUUCCGCCGCUUCCAGAAAAUCAGCCCCUCGAA
GGUACGUUUCCGUGCGACUUUGCCUGGGGCGUGGUCGACAACUACAUACAGGUAGACACUACUUUGUCCCAAUUCACUGAUCUCAA
UGUGUAUCUCUGGGAUGUUCAUCACAGCAAGCGACUGAUAAAGGUAGAUGGGGUCGUUACUAAAAAGAGGAAGUCAUACUGCGUCG
AUUUUGCAGCCAUCCAGCCUCAGAUAGCGUUGCUCCAGGAGAUGCACGUGACACAUUUUCGGUUUUCACUGGACUGGGCCCUUAUU
CUGCCGCUCGGGAACCAAUCUCAAGUUAAUCAUACUAUUCUGCAAUAUUACAGGUGCAUGGCGAGCGAGCUUGUUCGAGUAAAUAU
UACGCCUGUGGUUGCACUCUGGCAGCCGAUGGCGCCUAAUCAGGGGCUUCCGAGACUUCUUGCUCGACAAGGUGCUUGGGAGAACC
CAUAUACAGCAUUGGCCUUUGCAGAAUACGCGCGCUUGUGUUUCCAAGAGCUGGGCCAUCAUGUGAAACUUUGGAUAACCAUGAAU
GAGCCUUAUACACGGAACAUGACAUACUCAGCAGGGCACAACUUGCUGAAGGCUCAUGCUCUUGCAUGGCAUGUUUAUAAUGAAAA
AUUUCGACACGCACAAAACGGGAAAAUCAGCAUUGCUCUGCAGGCUGAUUGGAUAGAACCCGCAUGUCCAUUUUCACAAAAAGACA
AGGAAGUAGCGGAGCGCGUACUUGAGUUCGACAUAGGCUGGCUUGCCGAGCCCAUAUUUGGUUCCGGUGAUUAUCCGUGGGUAAUG
CGGGAUUGGCUUAAUCAACGCAACAACUUCCUCCUGCCUUACUUUACAGAGGACGAGAAAAAACUCAUUCAGGGCACCUUUGAUUU
CCUCGCUCUUAGCCAUUAUACAACUAUACUCGUUGACAGUGAAAAGGAAGACCCUAUCAAGUACAACGAUUAUCUUGAAGUCCAGG
AAAUGACAGAUAUAACCUGGCUUAACAGCCCGUCCCAGGUAGCUGUUGUUCCUUGGGGGUUGCGGAAAGUUUUGAAUUGGCUGAAA
UUCAAGUAUGGGGAUCUUCCCAUGUAUAUUAUUUCAAACGGCAUCGAUGAUGGUUUGCAUGCCGAAGAUGACCAACUUAGGGUAUA
UUACAUGCAAAACUACAUCAACGAGGCGCUCAAGGCCCAUAUCCUUGACGGUAUCAACUUGUGCGGUUAUUUUGCCUAUUCCUUUA
AUGAUAGAACAGCCCCUAGGUUCGGUUUGUACCGCUACGCAGCGGACCAAUUUGAGCCGAAAGCGUCAAUGAAACACUAUAGGAAG
AUAAUAGAUAGCAAUGGAUUUCCUGGGCCCGAGACUCUCGAGAGGUUCUGUCCAGAGGAAUUCACAGUCUGCACAGAAUGUAGCUU
UUUUCAUACGAGAAAGUCCUGA
SEQ ID NO: 27: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAACCGGGGGAUGGAGCUCAGACUUGGGCUAGGUUCUCUAGACCUCCAGCUCCCGAAGCUGCAGGACUGUUUC
AGGGAACUUUCCCCGAUGGGUUUCUGUGGGCCGUGGGAUCUGCUGCCUACCAAACCGAGGGCGGAUGGCAACAGCAUGGAAAAGGU
GCAUCCAUUUGGGAUACCUUCACCCACCAUCCUCUGGCACCCCCCGGAGAUUCAAGAAAUGCUUCUCUCCCUCUCGGUGCUCCAUC
ACCACUGCAGCCUGCUACAGGAGAUGUUGCCAGUGAUUCAUACAACAAUGUGUUUAGGGACACCGAAGCUUUGCGAGAGCUCGGGG
UCACUCAUUACCGCUUUUCUAUUUCAUGGGCCCGCGUACUUCCAAAUGGAUCUGCGGGAGUCCCAAAUAGAGAGGGCUUGAGAUAU
UACCGAAGGUUGCUGGAACGCCUGAGGGAACUUGGAGUGCAACCCGUAGUGACAUUGUACCACUGGGAUCUCCCCCAGAGGCUUCA
GGAUGCUUACGGUGGUUGGGCUAACAGAGCUCUCGCAGACCACUUCAGAGAUUAUGCAGAACUGUGCUUUCGACAUUUCGGCGGCC
AGGUAAAAUACUGGAUCACCAUAGACAAUCCCUAUGUGGUUGCGUGGCAUGGUUAUGCCACAGGAAGACUGGCUCCCGGUAUCCGG
GGGAGUCCUAGACUUGGCUACCUCGUGGCCCACAAUCUGCUGUUGGCGCACGCUAAGGUGUGGCAUCUGUAUAAUACUAGCUUUAG
ACCGACCCAGGGAGGUCAAGUGAGCAUCGCCCUGUCCUCUCACUGGAUAAAUCCCCGCAGAAUGACCGACCACUCCAUUAAGGAGU
GUCAAAAGUCUCUCGACUUCGUGCUCGGAUGGUUUGCUAAGCCGGUUUUCAUUGAUGGAGACUACCCCGAGAGCAUGAAAAACAAC
UUGAGCUCCAUUCUGCCUGACUUUACAGAAAGCGAAAAAAAAUUUAUCAAGGGUACUGCCGACUUUUUCGCACUCUGCUUUGGCCC
CACUCUUAGCUUUCAGUUGCUCGAUCCACACAUGAAGUUCCGCCAACUGGAGAGUCCUAAUCUGCGCCAACUCCUUUCUUGGAUUG
ACUUGGAGUUCAAUCACCCGCAGAUCUUCAUCGUGGAAAACGGCUGGUUCGUGUCAGGAACCACGAAGCGCGACGAUGCCAAGUAC
 
AUGUACUACCUCAAAAAGUUCAUCAUGGAAACUCUCAAAGCAAUUAAACUGGACGGCGUCGACGUGAUCGGCUAUACCGCGUGGUC
UCUGAUGGACGGUUUCGAGUGGCAUAGGGGCUAUUCCAUAAGGCGAGGGUUGUUUUACGUUGAUUUUCUCAGCCAAGAUAAAAUGC
UGCUGCCCAAGUCUUCAGCCUUGUUUUAUCAGAAAUUGAUUGAGAAGAAUGGCUUCCCGCCACUCCCCGAGAAUCAACCCCUCGAGG
GGACAUUUCCAUGUGACUUCGCCUGGGGGGUAGUCGAUAAUUACAUCCAAGUGGAUACAACCCUCAGCCAGUUUACCGACCUGAA
CGUUUACUUGUGGGACGUGCACCACAGUAAGAGACUGAUUAAAGUGGAUGGAGUGGUUACUAAAAAGCGGAAGAGUUAUUGUGUAG
AUUUUGCCGCAAUCCAGCCACAAAUCGCCCUGCUGCAGGAGAUGCACGUGACCCAUUUCAGAUUUAGUCUCGACUGGGCUCUGAUU
UUGCCCUUGGGCAAUCAGAGCCAGGUGAAUCAUACAAUCCUCCAGUACUAUCGGUGUAUGGCCAGUGAAUUGGUCCGAGUCAACAU
CACCCCAGUGGUGGCACUCUGGCAGCCAAUGGCUCCUAACCAAGGGCUGCCAAGACUGCUGGCAAGGCAGGGUGCCUGGGAGAAUC
CUUACACUGCUCUGGCCUUUGCCGAGUACGCUCGCCUUUGUUUUCAGGAGCUCGGGCAUCAUGUGAAACUGUGGAUUACAAUGAAU
GAACCAUAUACUCGCAACAUGACCUAUUCCGCAGGUCACAAUCUCCUCAAGGCUCAUGCACUCGCAUGGCACGUCUACAAUGAAAA
AUUCCGACACGCCCAGAAUGGGAAGAUUAGUAUCGCCCUGCAGGCUGAUUGGAUCGAGCCCGCAUGUCCAUUUUCACAAAAGGACA
AAGAGGUAGCCGAGAGAGUGCUGGAGUUCGAUAUCGGCUGGCUUGCCGAACCUAUUUUCGGCUCUGGCGAUUACCCCUGGGUGAUG
AGGGAUUGGUUGAACCAGAGGAACAAUUUCCUCCUGCCGUAUUUUACCGAGGACGAAAAGAAGUUGAUCCAAGGCACAUUCGAUUU
CCUCGCCCUUUCUCACUACACAACCAUACUUGUGGAUAGCGAGAAGGAGGACCCAAUUAAAUAUAAUGACUAUCUGGAAGUUCAGG
AGAUGACUGAUAUCACAUGGCUGAAUUCCCCCAGCCAAGUAGCAGUCGUGCCCUGGGGUCUGAGAAAGGUGCUUAAUUGGUUGAAG
UUUAAGUACGGUGAUCUGCCUAUGUAUAUAAUUUCUAACGGCAUAGAUGAUGGGCUGCACGCUGAGGACGAUCAACUGCGAGUGUA
CUACAUGCAGAAUUAUAUCAACGAAGCCUUGAAAGCCCACAUUCUGGACGGCAUCAAUCUUUGUGGCUACUUCGCUUAUUCAUUCA
AUGAUAGAACAGCCCCCCGCUUUGGAUUGUAUCGGUAUGCCGCCGACCAAUUCGAGCCAAAGGCUUCUAUGAAGCACUACCGGAAG
AUUAUCGACUCCAACGGGUUUCCAGGGCCAGAGACACUGGAAAGGUUUUGCCCAGAGGAGUUCACCGUGUGUACAGAGUGCAGCUU
UUUCCACACCCGGAAAUCAUGA
SEQ ID NO: 28: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAACCAGGAGAUGGUGCCCAAACUUGGGCUAGGUUCUCUCGUCCACCCGCACCUGAAGCUGCUGGGCUGUUUC
AAGGUACUUUUCCUGAUGGAUUUCUUUGGGCUGUCGGGUCCGCUGCUUAUCAAACUGAAGGUGGAUGGCAACAACAUGGGAAAGGC
GCUUCAAUUUGGGACACCUUUACACAUCAUCCUCUCGCCCCACCUGGUGAUAGUCGCAAUGCGAGCUUACCUCUCGGAGCACCAUC
UCCCUUGCAACCAGCAACAGGCGAUGUGGCUUCAGAUUCCUAUAAUAAUGUGUUUAGAGAUACUGAAGCUCUCAGAGAAUUGGGAG
UGACCCAUUAUAGAUUUUCUAUUUCUUGGGCCCGGGUCCUGCCUAACGGGUCCGCCGGGGUUCCUAAUCGAGAAGGUUUGCGGUAU
UAUAGAAGGCUUCUCGAAAGGUUGCGAGAACUUGGAGUACAACCUGUCGUGACUUUGUAUCAUUGGGAUCUUCCGCAAAGACUUCA
AGAUGCAUAUGGUGGAUGGGCAAAUCGUGCACUCGCUGAUCAUUUUCGGGACUAUGCCGAACUUUGUUUUAGGCAUUUUGGCGGCC
AAGUUAAAUAUUGGAUUACAAUAGAUAAUCCUUAUGUAGUUGCAUGGCAUGGUUAUGCAACAGGCCGGUUGGCACCAGGGAUUCGA
GGUUCACCACGACUUGGAUAUUUAGUCGCCCAUAAUCUUCUUUUGGCCCACGCGAAGGUAUGGCACUUAUAUAACACCUCUUUUAG
GCCGACGCAAGGUGGACAAGUCUCUAUCGCAUUGUCCUCACAUUGGAUUAACCCCAGGCGUAUGACAGAUCAUUCUAUUAAGGAAU
GCCAGAAGAGUCUCGAUUUCGUGUUAGGCUGGUUCGCAAAGCCAGUCUUCAUCGACGGAGAUUACCCGGAAUCAAUGAAGAACAAU
CUGAGCAGUAUCCUUCCCGACUUCACCGAGAGUGAAAAGAAAUUUAUUAAGGGCACCGCAGAUUUCUUCGCCCUGUGUUUCGGCCC
GACUCUCUCCUUCCAGCUGCUCGAUCCACAUAUGAAAUUUCGGCAGCUCGAGUCACCUAAUUUAAGACAGCUCCUGAGUUGGAUCG
AUCUGGAGUUCAAUCACCCACAGAUCUUCAUCGUUGAGAACGGGUGGUUCGUGUCUGGCACGACUAAACGCGACGACGCUAAGUAC
AUGUACUAUCUGAAGAAAUUUAUAAUGGAGACACUUAAGGCUAUAAAAUUGGACGGCGUAGACGUGAUUGGUUACACUGCUUGGAG
CCUGAUGGACGGCUUUGAAUGGCAUCGUGGGUAUUCAAUACGGCGGGGCUUAUUUUACGUGGAUUUCUUGUCUCAAGAUAAAAUGC
UGCUGCCCAAAAGCAGUGCACUUUUCUAUCAGAAACUCAUCGAAAAGAACGGAUUUCCACCCUUGCCAGAGAACCAACCUCUGGAA
GGAACCUUCCCAUGCGAUUUCGCGUGGGGUGUGGUGGAUAAUUAUAUCCAGGUGGACACUACCUUAAGUCAAUUCACAGAUCUCAA
CGUGUAUUUAUGGGACGUGCAUCAUAGCAAGAGACUGAUCAAGGUCGACGGUGUGGUUACUAAGAAGCGCAAGAGCUAUUGCGUCG
AUUUCGCAGCAAUUCAACCACAAAUUGCCCUGCUGCAAGAGAUGCAUGUGACCCACUUCAGAUUUAGUCUCGAUUGGGCAUUGAUC
CUGCCCCUCGGAAAUCAAUCACAAGUAAAUCAUACUAUACUCCAAUAUUACAGGUGUAUGGCUUCCGAACUGGUUCGAGUAAAUAU
UACACCUGUCGUUGCUUUGUGGCAACCGAUGGCACCUAAUCAGGGGCUUCCAAGGCUGCUCGCAAGACAAGGAGCGUGGGAAAAUC
CAUAUACAGCGUUAGCUUUCGCUGAAUACGCUAGGCUCUGUUUCCAGGAAUUGGGUCACCAUGUGAAAUUGUGGAUCACUAUGAAC
GAACCUUACACUCGGAACAUGACCUAUUCUGCAGGACAUAAUCUCCUCAAAGCUCACGCAUUAGCCUGGCACGUUUAUAACGAGAA
AUUCCGACACGCCCAAAACGGUAAGAUUUCUAUUGCGCUUCAAGCCGACUGGAUUGAGCCCGCUUGUCCCUUUAGCCAGAAAGAUA
AGGAAGUAGCAGAACGAGUACUGGAGUUCGAUAUAGGAUGGCUCGCGGAACCGAUAUUUGGAUCCGGCGACUACCCGUGGGUCAUG
CGAGAUUGGCUCAAUCAGCGCAAUAACUUCCUGCUCCCAUACUUUACAGAGGACGAGAAGAAACUCAUUCAAGGCACGUUCGAUUU
CCUUGCCCUGUCCCACUACACGACGAUUCUCGUGGAUUCCGAGAAAGAGGACCCUAUCAAGUAUAACGACUAUUUGGAAGUCCAGG
AGAUGACUGAUAUAACUUGGCUGAAUUCACCUUCCCAAGUCGCCGUUGUCCCGUGGGGUCUGAGAAAGGUACUUAAUUGGUUGAAA
UUUAAAUAUGGCGAUCUUCCUAUGUAUAUCAUUAGCAACGGGAUUGACGAUGGCUUGCACGCAGAAGAUGAUCAAUUGCGUGUCUA
CUACAUGCAAAACUAUAUCAAUGAGGCCCUGAAGGCUCAUAUCCUCGACGGCAUUAACUUGUGUGGGUAUUUCGCAUACUCAUUCA
AUGAUAGAACCGCACCUCGCUUCGGGCUUUACCGCUACGCCGCUGACCAAUUCGAACCUAAAGCCUCAAUGAAGCACUAUAGAAAG
AUCAUCGAUUCCAACGGGUUUCCCGGGCCUGAGACACUUGAGAGGUUCUGCCCCGAGGAGUUUACGGUCUGCACAGAAUGUAGCUU
CUUCCAUACACGGAAAUCCUGA
SEQ ID NO: 29: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
 
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAA
SEQ ID NO: 30: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAA
SEQ ID NO: 31: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
 
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAAAA
SEQ ID NO: 32: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
 
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCUGAGCUGGAGCCUCGGUGGCCAUGCUUCUUGCCCCUUGGGCCUCCCCCCAGCCCCUCCUCCCCUUCCUGCACCCGUACCCC
CGUGGUCUUUGAAUAAAGUCUGAGUGGGCGGCAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAA
SEQ ID NO: 33: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACUUCACCGAGAGCGAGAAGAAGUUCAUCAAGGGCACCGCCGACUUCUUCGCCCUGUGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGAGCCAGCUGACAAAACCUAUCAGCA
GCCUGACAAAGCCUUACCAU
SEQ ID NO: 34: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACUUCACCGAGAGCGAAAAAAAAUUCAUCAAGGGCACCGCCGAUUUCUUCGCCCUGUGUU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGAGCCAGCUGACAAAACCUAUCAGCA
GCCUGACAAAGCCUUACCAU
SEQ ID NO: 35: 
CUGUUCCAGGGCACCUUCCCCGACGGAUUUCUGUGGGCCGUGGGAAGCGCUGCCUACCAGACCGAGGGCGGCUGGCAACAGCACGG
CAAGGGCGCCAGCAUCUGGGACACCUUCACCCACCAUCCUCUGGCUCCUCCCGGAGACAGCAGAAACGCCAGCCUGCCUCUGGGAG
CCCCCAGCCCCCUGCAGCCCGCCACUGGAGACGUGGCCAGCGACUCCUACAACAACGUGUUCCGCGACACCGAGGCCCUGCGCGAG
CUGGGCGUGACCCACUACCGCUUUAGCAUCAGCUGGGCCAGAGUGCUGCCCAACGGCAGCGCCGGCGUGCCCAACCGGGAAGGCCU
GAGAUACUAUCGCCGCCUGCUGGAGAGGCUGAGAGAGUUGGGCGUGCAGCCCGUGGUGACCCUGUACCACUGGGACCUGCCCCAGC
GCCUGCAGGACGCCUACGGCGGCUGGGCCAACAGGGCACUCGCCGACCACUUCCGCGACUACGCCGAGCUGUGCUUCCGCCACUUC
GGCGGGCAGGUGAAGUACUGGAUCACCAUCGACAACCCCUACGUGGUGGCCUGGCACGGCUACGCUACAGGAAGGCUGGCCCCCGG
GAUCCGCGGCAGCCCCAGGCUGGGCUACCUGGUGGCCCACAACCUGCUGCUGGCACACGCCAAAGUGUGGCACCUGUAUAACACCA
GCUUUCGCCCCACCCAGGGUGGACAGGUGAGCAUUGCCCUGAGUUCUCAUUGGAUCAACCCCAGGCGCAUGACCGAUCACAGCAUC
AAGGAAUGCCAGAAGAGCCUGGACUUCGUGCUGGGCUGGUUCGCCAAGCCCGUGUUUAUCGACGGCGACUACCCCGAGUCUAUGAA
GAACAACCUCAGCAGCAUCCUGCCUGACGUCACCGAGAGCGAGAAGAAGUUCAUCAAGGGCACCGCCGACUUCUUCGCCCUGAGCU
UCGGCCCCACCCUGAGCUUCCAGCUGCUGGAUCCCCAUAUGAAGUUCAGACAACUCGAGAGUCCUAACCUGCGCCAGCUGCUGAGC
UGGAUCGACCUGGAAUUCAACCACCCACAGAUCUUCAUCGUGGAGAACGGCUGGUUUGUGAGCGGCACCACUAAGCGCGACGACGC
AAAGUAUAUGUAUUAUCUGAAGAAAUUCAUCAUGGAAACCCUGAAGGCCAUCAAGCUGGACGGCGUGGACGUGAUCGGAUACACCG
CAUGGAGCCUGAUGGACGGCUUCGAGUGGCACCGCGGUUACAGCAUCCGCCGGGGCCUGUUCUACGUUGACUUCCUGAGCCAGGAC
AAGAUGUUGCUGCCCAAGAGCUCCGCCCUGUUCUACCAGAAGUUGAUCGAGAAGAACGGCUUCCCACCACUCCCCGAGAACCAGCC
CCUGGAGGGCACCUUCCCCUGCGAUUUCGCCUGGGGCGUGGUGGACAACUACAUCCAGGUGAGCCAGCUGACAAAACCUAUCAGCA
GCCUGACAAAGCCUUACCAU
SEQ ID NO: 36: 
UUGUUUCAGGGAACCUUUCCUGACGGUUUCCUGUGGGCUGUGGGCAGCGCCGCCUAUCAGACCGAGGGCGGCUGGCAACAGCACGG
AAAAGGAGCCAGCAUCUGGGAUACCUUCACCCACCACCCCCUGGCCCCCCCCGGCGAUUCCCGCAAUGCCAGCCUCCCUCUGGGAG
CCCCUAGCCCCCUGCAACCUGCCACAGGAGACGUGGCCUCAGACAGCUAUAACAACGUGUUCCGCGACACCGAGGCACUGCGCGAG
CUGGGCGUGACCCAUUACCGCUUUAGCAUCAGCUGGGCCAGAGUGCUGCCCAAUGGCUCUGCCGGCGUGCCCAACCGGGAGGGCCU
GAGGUACUACCGCCGCCUGCUGGAGAGGCUGCGGGAGCUGGGCGUGCAGCCCGUGGUAACCCUGUACCACUGGGACCUGCCCCAGC
GCCUGCAGGACGCUUACGGCGGCUGGGCCAACCGCGCUCUCGCAGACCACUUCCGCGACUACGCCGAGCUGUGCUUUCGCCACUUU
 
GGCGGCCAGGUGAAGUAUUGGAUCACCAUCGAUAACCCUUACGUGGUGGCCUGGCACGGAUACGCUACAGGCCGCCUGGCCCCCGG
CAUCCGCGGCAGCCCCAGACUGGGCUACCUGGUGGCCCACAACCUGCUGCUGGCCCACGCCAAGGUGUGGCACCUGUACAACACCA
GCUUCCGCCCCACCCAGGGUGGACAGGUCUCUAUUGCCCUGAGCUCUCACUGGAUUAACCCCAGGAGGAUGACCGAUCACAGCAUC
AAGGAAUGCCAGAAGAGCCUGGACUUCGUUCUGGGCUGGUUCGCCAAGCCCGUGUUUAUCGACGGCGACUACCCCGAGAGCAUGAA
GAACAACCUGUCUAGCAUCCUGCCCGACGUCACCGAGAGCGAAAAAAAAUUCAUCAAGGGCACCGCCGAUUUCUUCGCCCUGAGCU
UCGGCCCAACCCUGAGCUUCCAACUGCUGGAUCCCCACAUGAAGUUCAGACAACUCGAGAGUCCCAACCUGCGCCAGCUGCUGAGC
UGGAUCGAUCUGGAAUUCAACCACCCCCAGAUAUUCAUAGUGGAGAACGGCUGGUUUGUAAGCGGCACUACUAAGCGCGAUGAUGC
AAAGUACAUGUACUAUCUGAAGAAGUUCAUCAUGGAAACCCUGAAGGCCAUCAAGCUGGAUGGCGUGGACGUGAUCGGAUACACCG
CAUGGAGCCUGAUGGACGGCUUCGAGUGGCACCGCGGUUACUCAAUCCGCAGGGGGUUGUUCUACGUCGACUUCCUGAGUCAGGAC
AAGAUGUUGCUGCCAAAGAGUAGCGCCCUUUUCUACCAGAAGUUGAUUGAGAAGAACGGCUUCCCACCACUCCCCGAGAACCAGCC
CCUGGAGGGCACCUUUCCCUGCGAUUUCGCUUGGGGCGUGGUGGAUAAUUAUAUUCAGGUGAGCCAGCUGACAAAACCUAUCAGCA
GCCUGACAAAGCCUUACCAU
SEQ ID NO: 37: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGAGCCAGCUGACAAAACCUAUCAGCAGCCUGACAAAGCCUUACCAU
SEQ ID NO: 38: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGAGCCAGCUGACAAAACCUAUCAGCAGCCUGACAAAGCCUUACCAU
SEQ ID NO: 39: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGAGCCAGCUGACAAAACCUAUCAGCAGCCUGACAAAGCCUUACCAU
SEQ ID NO: 40: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGAGCCAGCUGACAAAACCUAUCAGCAGCCUGACAAAGCCUUACCAU
SEQ ID NO: 41: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGAGCCAGCUGACAAAACCUAUCAGCAGCCUGAC
AAAGCCUUACCAUUGA
SEQ ID NO: 42: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGAGCCAGCUGACAAAACCUAUCAGCAGCCUGAC
AAAGCCUUACCAUUGA
SEQ ID NO: 43: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
 
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGAGCCAGCUGACAAAACCUAUCAGCAGCCUGAC
AAAGCCUUACCAUUGA
SEQ ID NO: 44: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGAGCCAGCUGACAAAACCUAUCAGCAGCCUGAC
AAAGCCUUACCAUUGA
SEQ ID NO: 45: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAA
SEQ ID NO: 46: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
 
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAAAA
SEQ ID NO: 47: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAA
SEQ ID NO: 48: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGAGCCAGCUGACAAAACCUAUCAGCAGCCUGACAAAGCCUUACCAUU
GAGCUGGAGCCUCGGUGGCCAUGCUUCUUGCCCCUUGGGCCUCCCCCCAGCCCCUCCUCCCCUUCCUGCACCCGUACCCCCGUGGU
CUUUGAAUAAAGUCUGAGUGGGCGGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAA
SEQ ID NO: 64: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
 
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACUUCACCGAGAGCGAGAAGAAGUUCAUCAAGGGCACCGCCGACUUCUUCGCCCUGUGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 65: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACUUCACCGAGAGCGAAAAAAAAUUCAUCAAGGGCACCGCCGAUUUCUUCGCCCUGUGUUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 66: 
GAGCCUGGCGACGGGGCCCAGACCUGGGCCAGAUUCAGCAGACCCCCCGCACCCGAGGCCGCCGGCCUGUUCCAGGGCACCUUCCC
CGACGGAUUUCUGUGGGCCGUGGGAAGCGCUGCCUACCAGACCGAGGGCGGCUGGCAACAGCACGGCAAGGGCGCCAGCAUCUGGG
ACACCUUCACCCACCAUCCUCUGGCUCCUCCCGGAGACAGCAGAAACGCCAGCCUGCCUCUGGGAGCCCCCAGCCCCCUGCAGCCC
GCCACUGGAGACGUGGCCAGCGACUCCUACAACAACGUGUUCCGCGACACCGAGGCCCUGCGCGAGCUGGGCGUGACCCACUACCG
 
CUUUAGCAUCAGCUGGGCCAGAGUGCUGCCCAACGGCAGCGCCGGCGUGCCCAACCGGGAAGGCCUGAGAUACUAUCGCCGCCUGC
UGGAGAGGCUGAGAGAGUUGGGCGUGCAGCCCGUGGUGACCCUGUACCACUGGGACCUGCCCCAGCGCCUGCAGGACGCCUACGGC
GGCUGGGCCAACAGGGCACUCGCCGACCACUUCCGCGACUACGCCGAGCUGUGCUUCCGCCACUUCGGCGGGCAGGUGAAGUACUG
GAUCACCAUCGACAACCCCUACGUGGUGGCCUGGCACGGCUACGCUACAGGAAGGCUGGCCCCCGGGAUCCGCGGCAGCCCCAGGC
UGGGCUACCUGGUGGCCCACAACCUGCUGCUGGCACACGCCAAAGUGUGGCACCUGUAUAACACCAGCUUUCGCCCCACCCAGGGU
GGACAGGUGAGCAUUGCCCUGAGUUCUCAUUGGAUCAACCCCAGGCGCAUGACCGAUCACAGCAUCAAGGAAUGCCAGAAGAGCCU
GGACUUCGUGCUGGGCUGGUUCGCCAAGCCCGUGUUUAUCGACGGCGACUACCCCGAGUCUAUGAAGAACAACCUCAGCAGCAUCC
UGCCUGACGUCACCGAGAGCGAGAAGAAGUUCAUCAAGGGCACCGCCGACUUCUUCGCCCUGAGCUUCGGCCCCACCCUGAGCUUC
CAGCUGCUGGAUCCCCAUAUGAAGUUCAGACAACUCGAGAGUCCUAACCUGCGCCAGCUGCUGAGCUGGAUCGACCUGGAAUUCAA
CCACCCACAGAUCUUCAUCGUGGAGAACGGCUGGUUUGUGAGCGGCACCACUAAGCGCGACGACGCAAAGUAUAUGUAUUAUCUGA
AGAAAUUCAUCAUGGAAACCCUGAAGGCCAUCAAGCUGGACGGCGUGGACGUGAUCGGAUACACCGCAUGGAGCCUGAUGGACGGC
UUCGAGUGGCACCGCGGUUACAGCAUCCGCCGGGGCCUGUUCUACGUUGACUUCCUGAGCCAGGACAAGAUGUUGCUGCCCAAGAG
CUCCGCCCUGUUCUACCAGAAGUUGAUCGAGAAGAACGGCUUCCCACCACUCCCCGAGAACCAGCCCCUGGAGGGCACCUUCCCCU
GCGAUUUCGCCUGGGGCGUGGUGGACAACUACAUCCAGGUGGACACCACCCUGAGUCAGUUCACUGACCUGAACGUGUACCUUUGG
GACGUGCACCACAGCAAGCGCCUGAUAAAGGUCGACGGCGUGGUGACCAAGAAGCGCAAAAGCUACUGCGUGGACUUUGCCGCCAU
CCAGCCCCAGAUCGCCCUGCUGCAGGAGAUGCACGUGACUCACUUCCGCUUCAGCCUCGACUGGGCCCUGAUUCUGCCCCUGGGCA
ACCAGUCACAAGUGAACCACACCAUUCUGCAGUACUACCGCUGUAUGGCCAGCGAGCUGGUACGCGUGAACAUCACCCCCGUUGUG
GCCCUUUGGCAGCCCAUGGCCCCCAACCAGGGCCUGCCUCGCCUGCUGGCCCGCCAGGGAGCCUGGGAGAAUCCCUACACCGCACU
GGCCUUCGCUGAAUACGCCCGCCUGUGCUUCCAGGAGCUGGGGCACCACGUGAAACUCUGGAUCACCAUGAACGAGCCCUACACCA
GAAACAUGACCUACAGCGCCGGACACAAUCUGCUGAAGGCCCACGCUCUGGCCUGGCACGUGUACAACGAGAAGUUUCGCCACGCC
CAGAACGGCAAGAUCAGCAUCGCCCUGCAGGCCGAUUGGAUCGAGCCCGCCUGCCCCUUCAGCCAGAAGGACAAGGAGGUGGCUGA
GCGGGUGCUGGAGUUCGACAUCGGGUGGCUGGCUGAGCCUAUCUUCGGCAGCGGAGACUACCCCUGGGUGAUGCGCGAUUGGCUUA
ACCAGAGGAACAAUUUCCUGCUGCCCUACUUCACCGAGGACGAGAAGAAGCUGAUCCAGGGCACCUUUGACUUCCUGGCUUUGAGC
CACUAUACCACUAUCCUGGUGGACAGCGAGAAGGAGGACCCCAUCAAGUACAACGACUACUUGGAGGUGCAGGAGAUGACCGACAU
CACCUGGCUGAACAGCCCUAGCCAGGUGGCUGUGGUGCCUUGGGGCCUGCGCAAGGUGCUGAACUGGCUGAAGUUCAAGUACGGCG
ACCUGCCCAUGUAUAUCAUCAGCAACGGCAUCGACGACGGCCUGCACGCCGAGGACGACCAGCUGAGAGUGUACUACAUGCAGAAC
UACAUCAACGAGGCCCUCAAGGCCCACAUCCUGGACGGCAUCAACCUGUGUGGCUACUUUGCCUACAGCUUCAACGACAGAACCGC
CCCCCGCUUCGGCCUGUACAGGUACGCCGCCGACCAGUUCGAACCCAAGGCCAGCAUGAAGCACUACCGCAAAAUCAUCGACAGCA
ACGGAUUCCCUGGACCCGAGACACUGGAGCGCUUCUGCCCAGAGGAGUUCACCGUGUGCACCGAGUGCAGUUUCUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 67: 
GAGCCUGGAGAUGGAGCCCAAACAUGGGCCAGAUUCAGCAGACCUCCCGCUCCUGAAGCCGCCGGCUUGUUUCAGGGAACCUUUCC
UGACGGUUUCCUGUGGGCUGUGGGCAGCGCCGCCUAUCAGACCGAGGGCGGCUGGCAACAGCACGGAAAAGGAGCCAGCAUCUGGG
AUACCUUCACCCACCACCCCCUGGCCCCCCCCGGCGAUUCCCGCAAUGCCAGCCUCCCUCUGGGAGCCCCUAGCCCCCUGCAACCU
GCCACAGGAGACGUGGCCUCAGACAGCUAUAACAACGUGUUCCGCGACACCGAGGCACUGCGCGAGCUGGGCGUGACCCAUUACCG
CUUUAGCAUCAGCUGGGCCAGAGUGCUGCCCAAUGGCUCUGCCGGCGUGCCCAACCGGGAGGGCCUGAGGUACUACCGCCGCCUGC
UGGAGAGGCUGCGGGAGCUGGGCGUGCAGCCCGUGGUAACCCUGUACCACUGGGACCUGCCCCAGCGCCUGCAGGACGCUUACGGC
GGCUGGGCCAACCGCGCUCUCGCAGACCACUUCCGCGACUACGCCGAGCUGUGCUUUCGCCACUUUGGCGGCCAGGUGAAGUAUUG
GAUCACCAUCGAUAACCCUUACGUGGUGGCCUGGCACGGAUACGCUACAGGCCGCCUGGCCCCCGGCAUCCGCGGCAGCCCCAGAC
UGGGCUACCUGGUGGCCCACAACCUGCUGCUGGCCCACGCCAAGGUGUGGCACCUGUACAACACCAGCUUCCGCCCCACCCAGGGU
GGACAGGUCUCUAUUGCCCUGAGCUCUCACUGGAUUAACCCCAGGAGGAUGACCGAUCACAGCAUCAAGGAAUGCCAGAAGAGCCU
GGACUUCGUUCUGGGCUGGUUCGCCAAGCCCGUGUUUAUCGACGGCGACUACCCCGAGAGCAUGAAGAACAACCUGUCUAGCAUCC
UGCCCGACGUCACCGAGAGCGAAAAAAAAUUCAUCAAGGGCACCGCCGAUUUCUUCGCCCUGAGCUUCGGCCCAACCCUGAGCUUC
CAACUGCUGGAUCCCCACAUGAAGUUCAGACAACUCGAGAGUCCCAACCUGCGCCAGCUGCUGAGCUGGAUCGAUCUGGAAUUCAA
CCACCCCCAGAUAUUCAUAGUGGAGAACGGCUGGUUUGUAAGCGGCACUACUAAGCGCGAUGAUGCAAAGUACAUGUACUAUCUGA
AGAAGUUCAUCAUGGAAACCCUGAAGGCCAUCAAGCUGGAUGGCGUGGACGUGAUCGGAUACACCGCAUGGAGCCUGAUGGACGGC
UUCGAGUGGCACCGCGGUUACUCAAUCCGCAGGGGGUUGUUCUACGUCGACUUCCUGAGUCAGGACAAGAUGUUGCUGCCAAAGAG
UAGCGCCCUUUUCUACCAGAAGUUGAUUGAGAAGAACGGCUUCCCACCACUCCCCGAGAACCAGCCCCUGGAGGGCACCUUUCCCU
GCGAUUUCGCUUGGGGCGUGGUGGAUAAUUAUAUUCAGGUGGACACCACCCUGUCACAGUUCACUGACCUGAACGUGUACCUGUGG
GACGUGCACCAUUCCAAGCGCCUGAUCAAGGUGGACGGCGUGGUGACCAAGAAGCGCAAAUCCUACUGCGUUGACUUUGCCGCCAU
UCAGCCACAGAUCGCCCUGCUGCAGGAGAUGCACGUCACCCACUUCCGCUUCAGCCUGGACUGGGCCCUCAUCCUGCCACUGGGCA
ACCAGUCCCAGGUGAACCACACCAUUCUGCAGUACUACCGCUGUAUGGCCAGCGAGCUGGUACGCGUGAACAUCACCCCCGUGGUG
GCCCUGUGGCAGCCCAUGGCCCCCAACCAGGGCCUGCCACGCCUGCUGGCCAGGCAGGGGGCCUGGGAGAACCCCUACACCGCACU
CGCUUUCGCCGAAUAUGCCAGGCUGUGCUUCCAGGAGCUGGGGCACCACGUGAAGCUCUGGAUCACCAUGAACGAACCCUACACCA
GAAACAUGACCUACUCUGCCGGACACAAUCUGCUGAAGGCCCACGCCCUGGCCUGGCACGUCUACAACGAGAAGUUCCGCCACGCC
CAAAAUGGAAAGAUCUCUAUAGCCCUGCAGGCCGAUUGGAUCGAGCCCGCCUGCCCCUUCUCACAGAAGGACAAGGAGGUGGCCGA
GCGCGUGCUGGAGUUCGACAUCGGCUGGCUGGCCGAGCCCAUCUUCGGCAGCGGAGACUACCCCUGGGUGAUGCGCGAUUGGCUUA
ACCAGCGCAACAACUUUCUGCUGCCCUACUUCACCGAGGACGAGAAGAAGCUGAUCCAGGGCACCUUCGACUUCCUGGCCCUGAGC
CACUAUACUACCAUCCUGGUCGACAGCGAGAAGGAGGACCCAAUCAAGUACAACGACUACCUGGAGGUGCAGGAGAUGACCGACAU
CACCUGGCUGAACAGCCCUAGCCAGGUCGCCGUGGUGCCUUGGGGCCUGCGCAAGGUGCUGAACUGGCUGAAGUUCAAGUAUGGCG
ACCUGCCAAUGUACAUCAUCAGCAACGGCAUUGAUGACGGCCUGCACGCCGAGGACGAUCAGCUGAGAGUGUACUAUAUGCAGAAC
UACAUCAACGAGGCACUGAAGGCCCACAUCCUGGACGGCAUCAACCUGUGUGGCUACUUUGCAUACUCCUUCAACGACAGAACCGC
CCCCCGCUUCGGCCUGUACAGGUACGCUGCCGAUCAGUUCGAGCCCAAGGCCAGCAUGAAGCACUACAGAAAAAUCAUUGAUAGCA
ACGGGUUCCCAGGCCCCGAGACCCUGGAGCGCUUCUGCCCCGAGGAGUUCACCGUGUGCACCGAGUGCAGUUUUUUCCACACCCGC
AAGAGCCUGCUGGCUUUCAUUGCUUUUCUGUUUUUUGCUUCCAUUAUUUCUCUCUCCCUCAUCUUUUACUACAGCAAGAAAGGAAG
AAGAAGCUACAAA
SEQ ID NO: 68: AUGCCCGCUAGCGCCCCACCGAGAAGACCGCGGCCGCCACCGCCAUCGCUGUCGCUGCUGCUGGUGCUGCUGGGACUGGGCGGAAG 
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACUUCACCGAGAGCGAGAAGAAGUUCAUCAAGGGCACCGCCGACUUCUUCGCCCUGUGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 69: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACUUCACCGAGAGCGAAAAAAAAUUCAUCAAGGGCACCGCCGAUUUCUUCGCCCUGUGUUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
 
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 70: 
AUGCCCGCUAGCGCCCCACCGAGAAGACCGCGGCCGCCACCGCCAUCGCUGUCGCUGCUGGUGCUGCUGGGACUGGGCGGAAG
AAGACUGCGUGCGGAGCCUGGCGACGGGGCCCAGACCUGGGCCAGAUUCAGCAGACCCCCCGCACCCGAGGCCGCCGGCCUGUUCC
AGGGCACCUUCCCCGACGGAUUUCUGUGGGCCGUGGGAAGCGCUGCCUACCAGACCGAGGGCGGCUGGCAACAGCACGGCAAGGGC
GCCAGCAUCUGGGACACCUUCACCCACCAUCCUCUGGCUCCUCCCGGAGACAGCAGAAACGCCAGCCUGCCUCUGGGAGCCCCCAG
CCCCCUGCAGCCCGCCACUGGAGACGUGGCCAGCGACUCCUACAACAACGUGUUCCGCGACACCGAGGCCCUGCGCGAGCUGGGCG
UGACCCACUACCGCUUUAGCAUCAGCUGGGCCAGAGUGCUGCCCAACGGCAGCGCCGGCGUGCCCAACCGGGAAGGCCUGAGAUAC
UAUCGCCGCCUGCUGGAGAGGCUGAGAGAGUUGGGCGUGCAGCCCGUGGUGACCCUGUACCACUGGGACCUGCCCCAGCGCCUGCA
GGACGCCUACGGCGGCUGGGCCAACAGGGCACUCGCCGACCACUUCCGCGACUACGCCGAGCUGUGCUUCCGCCACUUCGGCGGGC
AGGUGAAGUACUGGAUCACCAUCGACAACCCCUACGUGGUGGCCUGGCACGGCUACGCUACAGGAAGGCUGGCCCCCGGGAUCCGC
GGCAGCCCCAGGCUGGGCUACCUGGUGGCCCACAACCUGCUGCUGGCACACGCCAAAGUGUGGCACCUGUAUAACACCAGCUUUCG
CCCCACCCAGGGUGGACAGGUGAGCAUUGCCCUGAGUUCUCAUUGGAUCAACCCCAGGCGCAUGACCGAUCACAGCAUCAAGGAAU
GCCAGAAGAGCCUGGACUUCGUGCUGGGCUGGUUCGCCAAGCCCGUGUUUAUCGACGGCGACUACCCCGAGUCUAUGAAGAACAAC
CUCAGCAGCAUCCUGCCUGACGUCACCGAGAGCGAGAAGAAGUUCAUCAAGGGCACCGCCGACUUCUUCGCCCUGAGCUUCGGCCC
CACCCUGAGCUUCCAGCUGCUGGAUCCCCAUAUGAAGUUCAGACAACUCGAGAGUCCUAACCUGCGCCAGCUGCUGAGCUGGAUCG
ACCUGGAAUUCAACCACCCACAGAUCUUCAUCGUGGAGAACGGCUGGUUUGUGAGCGGCACCACUAAGCGCGACGACGCAAAGUAU
AUGUAUUAUCUGAAGAAAUUCAUCAUGGAAACCCUGAAGGCCAUCAAGCUGGACGGCGUGGACGUGAUCGGAUACACCGCAUGGAG
CCUGAUGGACGGCUUCGAGUGGCACCGCGGUUACAGCAUCCGCCGGGGCCUGUUCUACGUUGACUUCCUGAGCCAGGACAAGAUGU
UGCUGCCCAAGAGCUCCGCCCUGUUCUACCAGAAGUUGAUCGAGAAGAACGGCUUCCCACCACUCCCCGAGAACCAGCCCCUGGAG
GGCACCUUCCCCUGCGAUUUCGCCUGGGGCGUGGUGGACAACUACAUCCAGGUGGACACCACCCUGAGUCAGUUCACUGACCUGAA
CGUGUACCUUUGGGACGUGCACCACAGCAAGCGCCUGAUAAAGGUCGACGGCGUGGUGACCAAGAAGCGCAAAAGCUACUGCGUGG
ACUUUGCCGCCAUCCAGCCCCAGAUCGCCCUGCUGCAGGAGAUGCACGUGACUCACUUCCGCUUCAGCCUCGACUGGGCCCUGAUU
CUGCCCCUGGGCAACCAGUCACAAGUGAACCACACCAUUCUGCAGUACUACCGCUGUAUGGCCAGCGAGCUGGUACGCGUGAACAU
CACCCCCGUUGUGGCCCUUUGGCAGCCCAUGGCCCCCAACCAGGGCCUGCCUCGCCUGCUGGCCCGCCAGGGAGCCUGGGAGAAUC
CCUACACCGCACUGGCCUUCGCUGAAUACGCCCGCCUGUGCUUCCAGGAGCUGGGGCACCACGUGAAACUCUGGAUCACCAUGAAC
GAGCCCUACACCAGAAACAUGACCUACAGCGCCGGACACAAUCUGCUGAAGGCCCACGCUCUGGCCUGGCACGUGUACAACGAGAA
GUUUCGCCACGCCCAGAACGGCAAGAUCAGCAUCGCCCUGCAGGCCGAUUGGAUCGAGCCCGCCUGCCCCUUCAGCCAGAAGGACA
AGGAGGUGGCUGAGCGGGUGCUGGAGUUCGACAUCGGGUGGCUGGCUGAGCCUAUCUUCGGCAGCGGAGACUACCCCUGGGUGAUG
CGCGAUUGGCUUAACCAGAGGAACAAUUUCCUGCUGCCCUACUUCACCGAGGACGAGAAGAAGCUGAUCCAGGGCACCUUUGACUU
CCUGGCUUUGAGCCACUAUACCACUAUCCUGGUGGACAGCGAGAAGGAGGACCCCAUCAAGUACAACGACUACUUGGAGGUGCAGG
AGAUGACCGACAUCACCUGGCUGAACAGCCCUAGCCAGGUGGCUGUGGUGCCUUGGGGCCUGCGCAAGGUGCUGAACUGGCUGAAG
UUCAAGUACGGCGACCUGCCCAUGUAUAUCAUCAGCAACGGCAUCGACGACGGCCUGCACGCCGAGGACGACCAGCUGAGAGUGUA
CUACAUGCAGAACUACAUCAACGAGGCCCUCAAGGCCCACAUCCUGGACGGCAUCAACCUGUGUGGCUACUUUGCCUACAGCUUCA
ACGACAGAACCGCCCCCCGCUUCGGCCUGUACAGGUACGCCGCCGACCAGUUCGAACCCAAGGCCAGCAUGAAGCACUACCGCAAA
AUCAUCGACAGCAACGGAUUCCCUGGACCCGAGACACUGGAGCGCUUCUGCCCAGAGGAGUUCACCGUGUGCACCGAGUGCAGUUU
CUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 71: 
AUGCCCGCUAGCGCCCCACCGAGAAGACCGCGGCCGCCACCGCCAUCGCUGUCGCUGCUGCUGGUGCUGCUGGGACUGGGCGGAAG
AAGACUGCGUGCGGAGCCUGGAGAUGGAGCCCAAACAUGGGCCAGAUUCAGCAGACCUCCCGCUCCUGAAGCCGCCGGCUUGUUUC
AGGGAACCUUUCCUGACGGUUUCCUGUGGGCUGUGGGCAGCGCCGCCUAUCAGACCGAGGGCGGCUGGCAACAGCACGGAAAAGGA
GCCAGCAUCUGGGAUACCUUCACCCACCACCCCCUGGCCCCCCCCGGCGAUUCCCGCAAUGCCAGCCUCCCUCUGGGAGCCCCUAG
CCCCCUGCAACCUGCCACAGGAGACGUGGCCUCAGACAGCUAUAACAACGUGUUCCGCGACACCGAGGCACUGCGCGAGCUGGGCG
UGACCCAUUACCGCUUUAGCAUCAGCUGGGCCAGAGUGCUGCCCAAUGGCUCUGCCGGCGUGCCCAACCGGGAGGGCCUGAGGUAC
UACCGCCGCCUGCUGGAGAGGCUGCGGGAGCUGGGCGUGCAGCCCGUGGUAACCCUGUACCACUGGGACCUGCCCCAGCGCCUGCA
GGACGCUUACGGCGGCUGGGCCAACCGCGCUCUCGCAGACCACUUCCGCGACUACGCCGAGCUGUGCUUUCGCCACUUUGGCGGCC
AGGUGAAGUAUUGGAUCACCAUCGAUAACCCUUACGUGGUGGCCUGGCACGGAUACGCUACAGGCCGCCUGGCCCCCGGCAUCCGC
GGCAGCCCCAGACUGGGCUACCUGGUGGCCCACAACCUGCUGCUGGCCCACGCCAAGGUGUGGCACCUGUACAACACCAGCUUCCG
CCCCACCCAGGGUGGACAGGUCUCUAUUGCCCUGAGCUCUCACUGGAUUAACCCCAGGAGGAUGACCGAUCACAGCAUCAAGGAAU
GCCAGAAGAGCCUGGACUUCGUUCUGGGCUGGUUCGCCAAGCCCGUGUUUAUCGACGGCGACUACCCCGAGAGCAUGAAGAACAAC
CUGUCUAGCAUCCUGCCCGACGUCACCGAGAGCGAAAAAAAAUUCAUCAAGGGCACCGCCGAUUUCUUCGCCCUGAGCUUCGGCCC
AACCCUGAGCUUCCAACUGCUGGAUCCCCACAUGAAGUUCAGACAACUCGAGAGUCCCAACCUGCGCCAGCUGCUGAGCUGGAUCG
AUCUGGAAUUCAACCACCCCCAGAUAUUCAUAGUGGAGAACGGCUGGUUUGUAAGCGGCACUACUAAGCGCGAUGAUGCAAAGUAC
AUGUACUAUCUGAAGAAGUUCAUCAUGGAAACCCUGAAGGCCAUCAAGCUGGAUGGCGUGGACGUGAUCGGAUACACCGCAUGGAG
CCUGAUGGACGGCUUCGAGUGGCACCGCGGUUACUCAAUCCGCAGGGGGUUGUUCUACGUCGACUUCCUGAGUCAGGACAAGAUGU
UGCUGCCAAAGAGUAGCGCCCUUUUCUACCAGAAGUUGAUUGAGAAGAACGGCUUCCCACCACUCCCCGAGAACCAGCCCCUGGAG
GGCACCUUUCCCUGCGAUUUCGCUUGGGGCGUGGUGGAUAAUUAUAUUCAGGUGGACACCACCCUGUCACAGUUCACUGACCUGAA
CGUGUACCUGUGGGACGUGCACCAUUCCAAGCGCCUGAUCAAGGUGGACGGCGUGGUGACCAAGAAGCGCAAAUCCUACUGCGUUG
ACUUUGCCGCCAUUCAGCCACAGAUCGCCCUGCUGCAGGAGAUGCACGUCACCCACUUCCGCUUCAGCCUGGACUGGGCCCUCAUC
CUGCCACUGGGCAACCAGUCCCAGGUGAACCACACCAUUCUGCAGUACUACCGCUGUAUGGCCAGCGAGCUGGUACGCGUGAACAU
CACCCCCGUGGUGGCCCUGUGGCAGCCCAUGGCCCCCAACCAGGGCCUGCCACGCCUGCUGGCCAGGCAGGGGGCCUGGGAGAACC
CCUACACCGCACUCGCUUUCGCCGAAUAUGCCAGGCUGUGCUUCCAGGAGCUGGGGCACCACGUGAAGCUCUGGAUCACCAUGAAC
GAACCCUACACCAGAAACAUGACCUACUCUGCCGGACACAAUCUGCUGAAGGCCCACGCCCUGGCCUGGCACGUCUACAACGAGAA
GUUCCGCCACGCCCAAAAUGGAAAGAUCUCUAUAGCCCUGCAGGCCGAUUGGAUCGAGCCCGCCUGCCCCUUCUCACAGAAGGACA
AGGAGGUGGCCGAGCGCGUGCUGGAGUUCGACAUCGGCUGGCUGGCCGAGCCCAUCUUCGGCAGCGGAGACUACCCCUGGGUGAUG
CGCGAUUGGCUUAACCAGCGCAACAACUUUCUGCUGCCCUACUUCACCGAGGACGAGAAGAAGCUGAUCCAGGGCACCUUCGACUU
 
CCUGGCCCUGAGCCACUAUACUACCAUCCUGGUCGACAGCGAGAAGGAGGACCCAAUCAAGUACAACGACUACCUGGAGGUGCAGG
AGAUGACCGACAUCACCUGGCUGAACAGCCCUAGCCAGGUCGCCGUGGUGCCUUGGGGCCUGCGCAAGGUGCUGAACUGGCUGAAG
UUCAAGUAUGGCGACCUGCCAAUGUACAUCAUCAGCAACGGCAUUGAUGACGGCCUGCACGCCGAGGACGAUCAGCUGAGAGUGUA
CUAUAUGCAGAACUACAUCAACGAGGCACUGAAGGCCCACAUCCUGGACGGCAUCAACCUGUGUGGCUACUUUGCAUACUCCUUCA
ACGACAGAACCGCCCCCCGCUUCGGCCUGUACAGGUACGCUGCCGAUCAGUUCGAGCCCAAGGCCAGCAUGAAGCACUACAGAAAA
AUCAUUGAUAGCAACGGGUUCCCAGGCCCCGAGACCCUGGAGCGCUUCUGCCCCGAGGAGUUCACCGUGUGCACCGAGUGCAGUUU
UUUCCACACCCGCAAGAGCCUGCUGGCUUUCAUUGCUUUUCUGUUUUUUGCUUCCAUUAUUUCUCUCUCCCUCAUCUUUUACUACA
GCAAGAAAGGAAGAAGAAGCUACAAAUGA
SEQ ID NO: 72: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACUUCACCGAGAGCGAGAAGAAGUUCAUCAAGGGCACCGCCGACUUCUUCGCCCUGUGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAAAAAAAAAAA
SEQ ID NO: 73: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACUUCACCGAGAGCGAAAAAAAAUUCAUCAAGGGCACCGCCGAUUUCUUCGCCCUGUGUUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
 
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAA
SEQ ID NO: 74: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGCGACGGGGCCCAGACCUGGGCCAGAUUCAGCAGACCCCCCGCACCCGAGGCCGCCGGCCUGUUCCAGGGCACCUUCCCC
GACGGAUUUCUGUGGGCCGUGGGAAGCGCUGCCUACCAGACCGAGGGCGGCUGGCAACAGCACGGCAAGGGCGCCAGCAUCUGGGA
CACCUUCACCCACCAUCCUCUGGCUCCUCCCGGAGACAGCAGAAACGCCAGCCUGCCUCUGGGAGCCCCCAGCCCCCUGCAGCCCG
CCACUGGAGACGUGGCCAGCGACUCCUACAACAACGUGUUCCGCGACACCGAGGCCCUGCGCGAGCUGGGCGUGACCCACUACCGC
UUUAGCAUCAGCUGGGCCAGAGUGCUGCCCAACGGCAGCGCCGGCGUGCCCAACCGGGAAGGCCUGAGAUACUAUCGCCGCCUGCU
GGAGAGGCUGAGAGAGUUGGGCGUGCAGCCCGUGGUGACCCUGUACCACUGGGACCUGCCCCAGCGCCUGCAGGACGCCUACGGCG
GCUGGGCCAACAGGGCACUCGCCGACCACUUCCGCGACUACGCCGAGCUGUGCUUCCGCCACUUCGGCGGGCAGGUGAAGUACUGG
AUCACCAUCGACAACCCCUACGUGGUGGCCUGGCACGGCUACGCUACAGGAAGGCUGGCCCCCGGGAUCCGCGGCAGCCCCAGGCU
GGGCUACCUGGUGGCCCACAACCUGCUGCUGGCACACGCCAAAGUGUGGCACCUGUAUAACACCAGCUUUCGCCCCACCCAGGGUG
GACAGGUGAGCAUUGCCCUGAGUUCUCAUUGGAUCAACCCCAGGCGCAUGACCGAUCACAGCAUCAAGGAAUGCCAGAAGAGCCUG
GACUUCGUGCUGGGCUGGUUCGCCAAGCCCGUGUUUAUCGACGGCGACUACCCCGAGUCUAUGAAGAACAACCUCAGCAGCAUCCU
GCCUGACGUCACCGAGAGCGAGAAGAAGUUCAUCAAGGGCACCGCCGACUUCUUCGCCCUGAGCUUCGGCCCCACCCUGAGCUUCC
AGCUGCUGGAUCCCCAUAUGAAGUUCAGACAACUCGAGAGUCCUAACCUGCGCCAGCUGCUGAGCUGGAUCGACCUGGAAUUCAAC
CACCCACAGAUCUUCAUCGUGGAGAACGGCUGGUUUGUGAGCGGCACCACUAAGCGCGACGACGCAAAGUAUAUGUAUUAUCUGAA
GAAAUUCAUCAUGGAAACCCUGAAGGCCAUCAAGCUGGACGGCGUGGACGUGAUCGGAUACACCGCAUGGAGCCUGAUGGACGGCU
UCGAGUGGCACCGCGGUUACAGCAUCCGCCGGGGCCUGUUCUACGUUGACUUCCUGAGCCAGGACAAGAUGUUGCUGCCCAAGAGC
UCCGCCCUGUUCUACCAGAAGUUGAUCGAGAAGAACGGCUUCCCACCACUCCCCGAGAACCAGCCCCUGGAGGGCACCUUCCCCUG
CGAUUUCGCCUGGGGCGUGGUGGACAACUACAUCCAGGUGGACACCACCCUGAGUCAGUUCACUGACCUGAACGUGUACCUUUGGG
ACGUGCACCACAGCAAGCGCCUGAUAAAGGUCGACGGCGUGGUGACCAAGAAGCGCAAAAGCUACUGCGUGGACUUUGCCGCCAUC
CAGCCCCAGAUCGCCCUGCUGCAGGAGAUGCACGUGACUCACUUCCGCUUCAGCCUCGACUGGGCCCUGAUUCUGCCCCUGGGCAA
CCAGUCACAAGUGAACCACACCAUUCUGCAGUACUACCGCUGUAUGGCCAGCGAGCUGGUACGCGUGAACAUCACCCCCGUUGUGG
CCCUUUGGCAGCCCAUGGCCCCCAACCAGGGCCUGCCUCGCCUGCUGGCCCGCCAGGGAGCCUGGGAGAAUCCCUACACCGCACUG
GCCUUCGCUGAAUACGCCCGCCUGUGCUUCCAGGAGCUGGGGCACCACGUGAAACUCUGGAUCACCAUGAACGAGCCCUACACCAG
AAACAUGACCUACAGCGCCGGACACAAUCUGCUGAAGGCCCACGCUCUGGCCUGGCACGUGUACAACGAGAAGUUUCGCCACGCCC
AGAACGGCAAGAUCAGCAUCGCCCUGCAGGCCGAUUGGAUCGAGCCCGCCUGCCCCUUCAGCCAGAAGGACAAGGAGGUGGCUGAG
CGGGUGCUGGAGUUCGACAUCGGGUGGCUGGCUGAGCCUAUCUUCGGCAGCGGAGACUACCCCUGGGUGAUGCGCGAUUGGCUUAA
CCAGAGGAACAAUUUCCUGCUGCCCUACUUCACCGAGGACGAGAAGAAGCUGAUCCAGGGCACCUUUGACUUCCUGGCUUUGAGCC
ACUAUACCACUAUCCUGGUGGACAGCGAGAAGGAGGACCCCAUCAAGUACAACGACUACUUGGAGGUGCAGGAGAUGACCGACAUC
ACCUGGCUGAACAGCCCUAGCCAGGUGGCUGUGGUGCCUUGGGGCCUGCGCAAGGUGCUGAACUGGCUGAAGUUCAAGUACGGCGA
CCUGCCCAUGUAUAUCAUCAGCAACGGCAUCGACGACGGCCUGCACGCCGAGGACGACCAGCUGAGAGUGUACUACAUGCAGAACU
ACAUCAACGAGGCCCUCAAGGCCCACAUCCUGGACGGCAUCAACCUGUGUGGCUACUUUGCCUACAGCUUCAACGACAGAACCGCC
CCCCGCUUCGGCCUGUACAGGUACGCCGCCGACCAGUUCGAACCCAAGGCCAGCAUGAAGCACUACCGCAAAAUCAUCGACAGCAA
CGGAUUCCCUGGACCCGAGACACUGGAGCGCUUCUGCCCAGAGGAGUUCACCGUGUGCACCGAGUGCAGUUUCUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAA
SEQ ID NO: 75: 
GGGAGACAUAAACCCUGGCGCGCUCGCGGCCCGGCACUCUUCUGGUCCCCACAGACUCAGAGAGAACCCACCAUGCCCGCUAGCGC
CCCACCGAGAAGACCGCGGCCGCCACCGCCAUCGCUGUCGCUGCUGCUGGUGCUGCUGGGACUGGGCGGAAGAAGACUGCGUGCGG
AGCCUGGAGAUGGAGCCCAAACAUGGGCCAGAUUCAGCAGACCUCCCGCUCCUGAAGCCGCCGGCUUGUUUCAGGGAACCUUUCCU
GACGGUUUCCUGUGGGCUGUGGGCAGCGCCGCCUAUCAGACCGAGGGCGGCUGGCAACAGCACGGAAAAGGAGCCAGCAUCUGGGA
UACCUUCACCCACCACCCCCUGGCCCCCCCCGGCGAUUCCCGCAAUGCCAGCCUCCCUCUGGGAGCCCCUAGCCCCCUGCAACCUG
CCACAGGAGACGUGGCCUCAGACAGCUAUAACAACGUGUUCCGCGACACCGAGGCACUGCGCGAGCUGGGCGUGACCCAUUACCGC
UUUAGCAUCAGCUGGGCCAGAGUGCUGCCCAAUGGCUCUGCCGGCGUGCCCAACCGGGAGGGCCUGAGGUACUACCGCCGCCUGCU
GGAGAGGCUGCGGGAGCUGGGCGUGCAGCCCGUGGUAACCCUGUACCACUGGGACCUGCCCCAGCGCCUGCAGGACGCUUACGGCG
GCUGGGCCAACCGCGCUCUCGCAGACCACUUCCGCGACUACGCCGAGCUGUGCUUUCGCCACUUUGGCGGCCAGGUGAAGUAUUGG
 
AUCACCAUCGAUAACCCUUACGUGGUGGCCUGGCACGGAUACGCUACAGGCCGCCUGGCCCCCGGCAUCCGCGGCAGCCCCAGACU
GGGCUACCUGGUGGCCCACAACCUGCUGCUGGCCCACGCCAAGGUGUGGCACCUGUACAACACCAGCUUCCGCCCCACCCAGGGUG
GACAGGUCUCUAUUGCCCUGAGCUCUCACUGGAUUAACCCCAGGAGGAUGACCGAUCACAGCAUCAAGGAAUGCCAGAAGAGCCUG
GACUUCGUUCUGGGCUGGUUCGCCAAGCCCGUGUUUAUCGACGGCGACUACCCCGAGAGCAUGAAGAACAACCUGUCUAGCAUCCU
GCCCGACGUCACCGAGAGCGAAAAAAAAUUCAUCAAGGGCACCGCCGAUUUCUUCGCCCUGAGCUUCGGCCCAACCCUGAGCUUCC
AACUGCUGGAUCCCCACAUGAAGUUCAGACAACUCGAGAGUCCCAACCUGCGCCAGCUGCUGAGCUGGAUCGAUCUGGAAUUCAAC
CACCCCCAGAUAUUCAUAGUGGAGAACGGCUGGUUUGUAAGCGGCACUACUAAGCGCGAUGAUGCAAAGUACAUGUACUAUCUGAA
GAAGUUCAUCAUGGAAACCCUGAAGGCCAUCAAGCUGGAUGGCGUGGACGUGAUCGGAUACACCGCAUGGAGCCUGAUGGACGGCU
UCGAGUGGCACCGCGGUUACUCAAUCCGCAGGGGGUUGUUCUACGUCGACUUCCUGAGUCAGGACAAGAUGUUGCUGCCAAAGAGU
AGCGCCCUUUUCUACCAGAAGUUGAUUGAGAAGAACGGCUUCCCACCACUCCCCGAGAACCAGCCCCUGGAGGGCACCUUUCCCUG
CGAUUUCGCUUGGGGCGUGGUGGAUAAUUAUAUUCAGGUGGACACCACCCUGUCACAGUUCACUGACCUGAACGUGUACCUGUGGG
ACGUGCACCAUUCCAAGCGCCUGAUCAAGGUGGACGGCGUGGUGACCAAGAAGCGCAAAUCCUACUGCGUUGACUUUGCCGCCAUU
CAGCCACAGAUCGCCCUGCUGCAGGAGAUGCACGUCACCCACUUCCGCUUCAGCCUGGACUGGGCCCUCAUCCUGCCACUGGGCAA
CCAGUCCCAGGUGAACCACACCAUUCUGCAGUACUACCGCUGUAUGGCCAGCGAGCUGGUACGCGUGAACAUCACCCCCGUGGUGG
CCCUGUGGCAGCCCAUGGCCCCCAACCAGGGCCUGCCACGCCUGCUGGCCAGGCAGGGGGCCUGGGAGAACCCCUACACCGCACUC
GCUUUCGCCGAAUAUGCCAGGCUGUGCUUCCAGGAGCUGGGGCACCACGUGAAGCUCUGGAUCACCAUGAACGAACCCUACACCAG
AAACAUGACCUACUCUGCCGGACACAAUCUGCUGAAGGCCCACGCCCUGGCCUGGCACGUCUACAACGAGAAGUUCCGCCACGCCC
AAAAUGGAAAGAUCUCUAUAGCCCUGCAGGCCGAUUGGAUCGAGCCCGCCUGCCCCUUCUCACAGAAGGACAAGGAGGUGGCCGAG
CGCGUGCUGGAGUUCGACAUCGGCUGGCUGGCCGAGCCCAUCUUCGGCAGCGGAGACUACCCCUGGGUGAUGCGCGAUUGGCUUAA
CCAGCGCAACAACUUUCUGCUGCCCUACUUCACCGAGGACGAGAAGAAGCUGAUCCAGGGCACCUUCGACUUCCUGGCCCUGAGCC
ACUAUACUACCAUCCUGGUCGACAGCGAGAAGGAGGACCCAAUCAAGUACAACGACUACCUGGAGGUGCAGGAGAUGACCGACAUC
ACCUGGCUGAACAGCCCUAGCCAGGUCGCCGUGGUGCCUUGGGGCCUGCGCAAGGUGCUGAACUGGCUGAAGUUCAAGUAUGGCGA
CCUGCCAAUGUACAUCAUCAGCAACGGCAUUGAUGACGGCCUGCACGCCGAGGACGAUCAGCUGAGAGUGUACUAUAUGCAGAACU
ACAUCAACGAGGCACUGAAGGCCCACAUCCUGGACGGCAUCAACCUGUGUGGCUACUUUGCAUACUCCUUCAACGACAGAACCGCC
CCCCGCUUCGGCCUGUACAGGUACGCUGCCGAUCAGUUCGAGCCCAAGGCCAGCAUGAAGCACUACAGAAAAAUCAUUGAUAGCAA
CGGGUUCCCAGGCCCCGAGACCCUGGAGCGCUUCUGCCCCGAGGAGUUCACCGUGUGCACCGAGUGCAGUUUUUUCCACACCCGCA
AGAGCCUGCUGGCUUUCAUUGCUUUUCUGUUUUUUGCUUCCAUUAUUUCUCUCUCCCUCAUCUUUUACUACAGCAAGAAAGGAAGA
AGAAGCUACAAAUGAGCUGGAGCCUCGGUGGCCAUGCUUCUUGCCCCUUGGGCCUCCCCCCAGCCCCUCCUCCCCUUCCUGCACCC
GUACCCCCGUGGUCUUUGAAUAAAGUCUGAGUGGGCGGCAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAAAAA
SEQ ID NO: 49:
SEQ ID NO: 50: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVSQLTKPISSLTKPYH
SEQ ID NO: 51:
   MPASAPPRRPRPPPPSLSLLLVLLGLGGRRLRA
SEQ ID NO: 52: 
LFQGTFPDGFLWAVGSAAYQTEGGWQQHGKGASIWDTFTHHPLAPPGDSRNASLPLGAPSPLQPATGDVASDSYNNVFRDTEALRE
LGVTHYRFSISWARVLPNGSAGVPNREGLRYYRRLLERLRELGVQPVVTLYHWDLPQRLQDAYGGWANRALADHFRDYAELCFRHF
GGQVKYWITIDNPYVVAWHGYATGRLAPGIRGSPRLGYLVAHNLLLAHAKVWHLYNTSFRPTQGGQVSIALSSHWINPRRMTDHSI
KECQKSLDFVLGWFAKPVFIDGDYPESMKNNLSSILPDFTESEKKFIKGTADFFALCFGPTLSFQLLDPHMKFRQLESPNLRQLLS
WIDLEFNHPQIFIVENGWFVSGTTKRDDAKYMYYLKKFIMETLKAIKLDGVDVIGYTAWSLMDGFEWHRGYSIRRGLFYVDFLSQD
KMLLPKSSALFYQKLIEKNGF
SEQ ID NO: 53: 
LFQGTFPDGFLWAVGSAAYQTEGGWQQHGKGASIWDTFTHHPLAPPGDSRNASLPLGAPSPLQPATGDVASDSYNNVFRDTEALRE
LGVTHYRFSISWARVLPNGSAGVPNREGLRYYRRLLERLRELGVQPVVTLYHWDLPQRLQDAYGGWANRALADHFRDYAELCFRHF
GGQVKYWITIDNPYVVAWHGYATGRLAPGIRGSPRLGYLVAHNLLLAHAKVWHLYNTSFRPTQGGQVSIALSSHWINPRRMTDHSI
KECQKSLDFVLGWFAKPVFIDGDYPESMKNNLSSILPDVTESEKKFIKGTADFFALSFGPTLSFQLLDPHMKFRQLESPNLRQLLS
WIDLEFNHPQIFIVENGWFVSGTTKRDDAKYMYYLKKFIMETLKAIKLDGVDVIGYTAWSLMDGFEWHRGYSIRRGLFYVDFLSQD
KMLLPKSSALFYQKLIEKNGF
SEQ ID NO: 54: LFQGTFPDGFLWAVGSAAYQTEGGWQQHGKGASIWDTFTHHPLAPPGDSRNASLPLGAPSPLQPATGDVASDSYNNVFRDTEALRE 
LGVTHYRFSISWARVLPNGSAGVPNREGLRYYRRLLERLRELGVQPVVTLYHWDLPQRLQDAYGGWANRALADHFRDYAELCFRHF
GGQVKYWITIDNPYVVAWHGYATGRLAPGIRGSPRLGYLVAHNLLLAHAKVWHLYNTSFRPTQGGQVSIALSSHWINPRRMTDHSI
KECQKSLDFVLGWFAKPVFIDGDYPESMKNNLSSILPDFTESEKKFIKGTADFFALCFGPTLSFQLLDPHMKFRQLESPNLRQLLS
WIDLEFNHPQIFIVENGWFVSGTTKRDDAKYMYYLKKFIMETLKAIKLDGVDVIGYTAWSLMDGFEWHRGYSIRRGLFYVDFLSQD
KMLLPKSSALFYQKLIEKNGFPPLPENQPLEGTFPCDFAWGVVDNYIQVDTTLSQFTDLNVYLWDVHHSKRLIKVDGVVTKKRKSY
CVDFAAIQPQIALLQEMHVTHFRFSLDWALILPLGNQSQVNHTILQYYRCMASELVRVNITPVVALWQPMAPNQGLPRLLARQGAW
ENPYTALAFAEYARLCFQELGHHVKLWITMNEPYTRNMTYSAGHNLLKAHALAWHVYNEKFRHAQNGKISIALQADWIEPACPFSQ
KDKEVAERVLEFDIGWLAEPIFGSGDYPWVMRDWLNQRNNFLLPYFTEDEKKLIQGTFDFLALSHYTTILVDSEKEDPIKYNDYLE
VQEMTDITWLNSPSQVAVVPWGLRKVLNWLKFKYGDLPMYIISNGIDDGLHAEDDQLRVYYMQNYINEALKAHILDGINLCGYFAY
SFNDRTAPRFGLYRYAADQFEPKASMKHYRKIIDSNG
SEQ ID NO: 55: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGF
SEQ ID NO: 56: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGF
SEQ ID NO: 57: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVDTTLSQFTDLNVYLWDVHHSKRLIKVDGVVTKKRKSYCVDFAAIQPQIALLQEMHVTHFRFSLDWALI
LPLGNQSQVNHTILQYYRCMASELVRVNITPVVALWQPMAPNQGLPRLLARQGAWENPYTALAFAEYARLCFQELGHHVKLWITMN
EPYTRNMTYSAGHNLLKAHALAWHVYNEKFRHAQNGKISIALQADWIEPACPFSQKDKEVAERVLEFDIGWLAEPIFGSGDYPWVM
RDWLNQRNNFLLPYFTEDEKKLIQGTFDFLALSHYTTILVDSEKEDPIKYNDYLEVQEMTDITWLNSPSQVAVVPWGLRKVLNWLK
FKYGDLPMYIISNGIDDGLHAEDDQLRVYYMQNYINEALKAHILDGINLCGYFAYSFNDRTAPRFGLYRYAADQFEPKASMKHYRK
IIDSNGFPGPETLERFCPEEFTVCTECSFFHTRKS
SEQ ID NO: 58: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVDTTLSQFTDLNVYLWDVHHSKRLIKVDGVVTKKRKSYCVDFAAIQPQIALLQEMHVTHFRFSLDWALI
LPLGNQSQVNHTILQYYRCMASELVRVNITPVVALWQPMAPNQGLPRLLARQGAWENPYTALAFAEYARLCFQELGHHVKLWITMN
EPYTRNMTYSAGHNLLKAHALAWHVYNEKFRHAQNGKISIALQADWIEPACPFSQKDKEVAERVLEFDIGWLAEPIFGSGDYPWVM
RDWLNQRNNFLLPYFTEDEKKLIQGTFDFLALSHYTTILVDSEKEDPIKYNDYLEVQEMTDITWLNSPSQVAVVPWGLRKVLNWLK
FKYGDLPMYIISNGIDDGLHAEDDQLRVYYMQNYINEALKAHILDGINLCGYFAYSFNDRTAPRFGLYRYAADQFEPKASMKHYRK
IIDSNGFPGPETLERFCPEEFTVCTECSFFHTRKS
SEQ ID NO: 59: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDFTESEKKFIKGTADFFALCFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVSQLTKPISSLTKPYH
SEQ ID NO: 60: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVSQLTKPISSLTKPYH
SEQ ID NO: 76: 
MPASAPPRRPRPPPPSLSLLLVLLGLGGRRLRAEPGDGAQTWARFSRPPAPEAAGLFQGTFPDGFLWAVGSAAYQTEGGWQQHGKG
ASIWDTFTHHPLAPPGDSRNASLPLGAPSPLQPATGDVASDSYNNVFRDTEALRELGVTHYRFSISWARVLPNGSAGVPNREGLRY
 
YRRLLERLRELGVQPVVTLYHWDLPQRLQDAYGGWANRALADHFRDYAELCFRHFGGQVKYWITIDNPYVVAWHGYATGRLAPGIR
GSPRLGYLVAHNLLLAHAKVWHLYNTSFRPTQGGQVSIALSSHWINPRRMTDHSIKECQKSLDFVLGWFAKPVFIDGDYPESMKNN
LSSILPDVTESEKKFIKGTADFFALSFGPTLSFQLLDPHMKFRQLESPNLRQLLSWIDLEFNHPQIFIVENGWFVSGTTKRDDAKY
MYYLKKFIMETLKAIKLDGVDVIGYTAWSLMDGFEWHRGYSIRRGLFYVDFLSQDKMLLPKSSALFYQKLIEKNGFPPLPENQPLE
GTFPCDFAWGVVDNYIQVDTTLSQFTDLNVYLWDVHHSKRLIKVDGVVTKKRKSYCVDFAAIQPQIALLQEMHVTHFRFSLDWALI
LPLGNQSQVNHTILQYYRCMASELVRVNITPVVALWQPMAPNQGLPRLLARQGAWENPYTALAFAEYARLCFQELGHHVKLWITMN
EPYTRNMTYSAGHNLLKAHALAWHVYNEKFRHAQNGKISIALQADWIEPACPFSQKDKEVAERVLEFDIGWLAEPIFGSGDYPWVM
RDWLNQRNNFLLPYFTEDEKKLIQGTFDFLALSHYTTILVDSEKEDPIKYNDYLEVQEMTDITWLNSPSQVAVVPWGLRKVLNWLK
FKYGDLPMYIISNGIDDGLHAEDDQLRVYYMQNYINEALKAHILDGINLCGYFAYSFNDRTAPRFGLYRYAADQFEPKASMKHYRK
IIDSNGFPGPETLERFCPEEFTVCTECSFFHTRKSLLAFIAFLFFASIISLSLIFYYSKKGRRSYK
SEQ ID NO: 61:
   GGGAGACAUAAACCCUGGCGCGCUCGCGGCCCGGCACUCUUCUGGUCCCCACAGACUCAGAGAGAACCCACC
SEQ ID NO: 62: 
GCUGGAGCCUCGGUGGCCAUGCUUCUUGCCCCUUGGGCCUCCCCCCAGCCCCUCCUCCCCUUCCUGCACCCGUACCCCCGUGGUCU
UUGAAUAAAGUCUGAGUGGGCGGCA
SEQ ID NO: 63:
   YCCANCCCWNUCYCC
SEQ ID NO: 77:
   TGCTGCCGTAATACGACTCACTATAAGG
SEQ ID NO: 78:

TTTTTTTTTTTTTTTTTTTTTTTTTTTTTTTTTTTTTTTTTTTTTTTTTTTTTTTTTTTTTTTTTTTTTTTTTTTTTTTTTTT
TTTTTTTTTTTTTTTTTTTTTTTTTTTTTTTTTTGCCGCCCACTCAGACTTTATTC

## Claims

1. Klotho messenger-RNA (mRNA), wherein the mRNA has a 5' CAP region, a 5' untranslated region (5'-UTR), a coding region encoding a Klotho polypeptide, a 3' untranslated region (3'-UTR) and a polyadenosine tail (poly-A tail),
wherein the Klotho polypeptide comprises the KL1 domain of human Klotho,
wherein the coding region encoding the Klotho polypeptide comprises an RNA sequence having at least 80 % sequence identity to SEQ ID NO: 1, and
wherein the coding region encoding the Klotho polypeptide has a GC content of at least 54 %.

2. The Klotho mRNA according to claim 1, wherein the Klotho polypeptide comprises the KL1 domain and the KL2 domain of human Klotho, and
wherein the coding region encoding the Klotho polypeptide comprises an RNA sequence having at least 80 % sequence identity to SEQ ID NO: 13.

3. The Klotho mRNA according to any one of the preceding claims, wherein the coding region encoding the Klotho polypeptide has at least 80 % sequence identity to SEQ ID NO: 18, 41, or 68.

4. The Klotho mRNA according to any one of the preceding claims, wherein the coding region encoding the Klotho polypeptide comprises a sequence selected from SEQ ID NO: 1, 2, 3, 4, 5, 6, 7, 8, 9, 10, 11, 12, 13, 14, 15, 16, 18, 19, 20, 21, 22, 23, 24, 33, 34, 35, 36, 37, 38, 39, 40, 41, 42, 43, 44, 64, 65, 66, 67, 68, 69, 70, 71.

5. The Klotho mRNA according to any one of the preceding claims, wherein the coding region encoding the Klotho polypeptide is selected from SEQ ID NO: 18, 19, 20, 21, 41, 42, 43, 44, 68, 69, 70, and 71.

6. The Klotho mRNA according to any one of the preceding claims, wherein the Klotho polypeptide has at least 90 % sequence identity to SEQ ID NO: 49, 55, 57, or 59, preferably wherein the Klotho polypeptide is selected from SEQ ID NO: 49, 55, 56, 57, 58, 59, 60, or 76, preferably 49, 57 or 59.

7. The Klotho mRNA according to any one of the preceding claims, wherein the Klotho mRNA is SEQ ID NO: 29, 30, 31, 32, 45, 46, 47, 48, 72, 73, 74, or 75; preferably SEQ ID NO: 29, 45, or 72, especially 29.

8. The Klotho mRNA according to any one of the preceding claims, wherein in the Klotho mRNA, at least 5%, preferably at least 10%, preferably at least 30%, especially at least 50% of all
- cytidine residues are replaced by 5-methyl-cytidine residues, and/or
- cytidine residues are replaced by 2-amino-2-deoxy-cytidine residues, and/or
- cytidine residues are replaced by 2-fluoro-2-deoxy-cytidine residues, and/or
- cytidine residues are replaced by 2-thio-cytidine residues, and/or
- cytidine residues are replaced by 5-iodo-cytidine residues, and/or
- uridine residues are replaced by pseudo-uridine residues, and/or
- uridine residues are replaced by 1-methyl-pseudo-uridine residues, and/or
- uridine residues are replaced by 2-thio-uridine residues, and/or
- uridine residues are replaced by 5-methyl-uridine residues, and/or
- adenosine residues are replaced by N6-methyl-adenosine residues.

9. Pharmaceutical formulation comprising the Klotho mRNA according to any one of the preceding claims and a pharmaceutically acceptable carrier.

10. The pharmaceutical formulation according to the preceding claim, wherein the formulation comprises a further mRNA, wherein the further mRNA has a further 5' CAP region, a further 5'-UTR, a further coding region encoding a further polypeptide, a further 3'-UTR and a further poly-A tail; preferably wherein the further polypeptide is Bactericidal/Permeability Increasing Protein Family B, member 4 (BPIFB4) protein or an isoform or variant thereof, preferably Longevity-Associated Variant of BPIFB4 (LAV-BPIFB4)

11. The pharmaceutical formulation according to any one of the preceding claims, wherein the further polypeptide is a protein selected from Apolipoprotein E, Tumor protein p53, Sirtuin 1 protein, FOXO1 transcription factor, Cholinergic receptor nicotinic alpha 3 subunit, SH2B adaptor protein 3, Cyclin dependent kinase inhibitor 2A, Elongation of very-long-chain fatty acids-like 2, Werner protein, Paraoxonase 1, Superoxide dismutase 2, Lamin A protein, Cholesteryl ester transfer protein, Apolipoprotein C3, Microsomal triglyceride transfer protein, Phosphatidylinositol 3-kinase (PI3K), Insulin-like growth factor 1 (IGF-1) receptor, Protein-L-isoaspartyl methyltransferase, Growth hormone, cAMP-response element binding protein, Mitogen-activated protein kinase, epidermal growth factor receptor, Nuclear factor kappa B, Phospholipase C beta, Methionine sulfoxide reductase A, Mediator of cell motility 1, Nei like DNA Glycosylase 1, Peroxisome proliferator-activated receptor gamma 2, Eukaryotic translation initiation factor 3 subunit K, ATM serine/threonine kinase, B-cell lymphoma 2, Cell division cycle 42, Diacylglycerol O -acyltransferase 1, Early growth response 1, Fibroblast growth factor 23, Fibroblast growth factor 21, Fructosamine 3 kinase related protein, Phosphoglycolate phosphatase, Insulin receptor substrate 1, Polycomb complex protein BMI-1, Neuregulin 1, Signal transducer and activator of transcription, E2F Transcription Factor 1, Vascular endothelial growth factor A, Xenobiotic metabolizing enzymes, Myc proto-oncogene protein, C-X-C chemokine receptor type 4, Silent information regulator 2, Extracellular signal-regulated kinase, and SLC31.

12. Kit for administering the Klotho mRNA according to any one of the preceding claims to an individual, preferably a human, comprising
- the Klotho mRNA according to any one of the preceding claims, and
- a device for administering the Klotho mRNA.

13. The Klotho mRNA or the pharmaceutical composition according to any one of the preceding claims for use as a medicament.

14. The Klotho mRNA or the pharmaceutical composition according to any one of the preceding claims for use in the prevention or treatment of an aging-related disorder and/or a symptom of aging.

15. Use of the Klotho mRNA according to any one of the preceding claims for increasing the lifespan in a mammal, preferably a human.
